(19) Europäisches Patentamt
European Patent Office
Office européen des brevets

(11) Publication number: **0 188 816**
**B1**

(12) **EUROPEAN PATENT SPECIFICATION**

(45) Date of publication of patent specification: **17.10.90**

(21) Application number: **85116610.8**

(22) Date of filing: **27.12.85**

(51) Int. Cl.[5]: **C 07 D 205/08,** C 07 F 7/18, C 07 D 487/04 // (C07D487/04, 209:00, 205:00)

(54) **Novel beta-lactams and their production.**

(30) Priority: **27.12.84 JP 279452/84**
**04.07.85 JP 147214/85**

(43) Date of publication of application:
**30.07.86 Bulletin 86/31**

(45) Publication of the grant of the patent:
**17.10.90 Bulletin 90/42**

(84) Designated Contracting States:
**AT BE CH DE FR GB IT LI LU NL SE**

(56) References cited:
**EP-A-0 026 816**
**EP-A-0 116 432**

**TETRAHEDRON LETTERS, vol. 25, no. 22, 1984, pages 2387-2390, Pergamon Press, Oxford, GB; M. HATANAKA et al.: "The synthesis of the l-carbapenem antibiotic (+/−)-PS-5 and its 6-Ipi analogue"**

**The file contains technical information submitted after the application was filed and not included in this specification**

(73) Proprietor: **SUMITOMO PHARMACEUTICALS COMPANY, LIMITED**
**2-8, Doshomachi 2-chome, Chuo-ku**
**Osaka-shi Osaka-fu (JP)**

(72) Inventor: **Sunagawa, Makoto**
**11-8-106, Sonehigahsi-machi 2-chome**
**Toyonaka Osaka (JP)**
Inventor: **Sasaki, Akira**
**2-40, Hirata 1-chome**
**Ibaraki Osaka (JP)**
Inventor: **Goda, Koshiro**
**1-5-502, Ohji-cho 4-chome Abeno-ku**
**Osaka (JP)**

(74) Representative: **Vossius & Partner**
**Siebertstrasse 4 P.O. Box 86 07 67**
**D-8000 München 86 (DE)**

(56) References cited:
**CHEMICAL ABSTRACTS, vol. 100, no. 7, 13 Feb 1984, Columbus, OH (US); p. 561, no. 51360h, Columbus, Ohio, US; & JP-A-58 144 386**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 188 816 B1**

Courier Press, Leamington Spa, England.

**Description**

Since the successful isolation of the antibiotic thienamycin [U.S. patent 3,950,357; J. Am. Chem. Soc., *100* (1978), 313], various carbapenem compounds have been reported. Among them, there are known some carbapenem compounds substituted with an alkyl group at the 1-position. 1-Methylcarbapenem compounds are particularly notable in exerting strong antimicrobial activity against various microorganisms [EP—0071908A; Heterocycles, *21* (1984), 29]. However, their synthetic methods as heretofore reported are troublesome in requiring a lengthy series of reaction steps. Further, those methods are defective in that the stereospecific formation of the 1-methyl group is not possible.

Hatanaka et al., Tetrahedron Letters, Vol. 25, No. 22 (1984), 2387—2390, describe the total synthesis of the 1-carbapenem antibiotic (±)—PS—5 (18) and its 6-epi analogue (19):

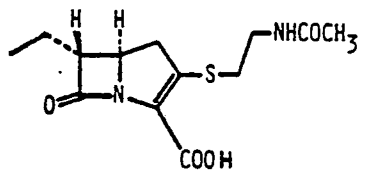

(18)

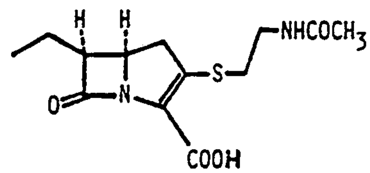

(19)

For the preparation of (18) the intermediate (12d) is regioselectively converted in a modified Dieckmann reaction to the 2-oxocarbapenem (14) followed by conversion into the 2-(2-acetamidoethylthio)-carbapenem (16) and reductive removal of the p-nitrobenzyl groups. Thus this synthesis involves three steps. (19) is prepared in an analogous manner.

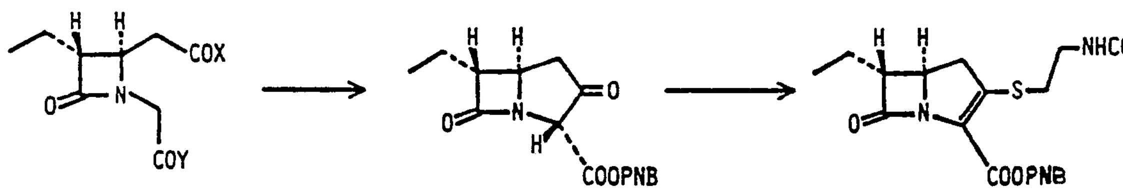

(12) (14) (16)

In EP—A—116 432 N-substituted azetidin-2-ones of the general formula

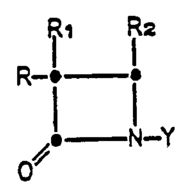

and a process for their preparation is described. The compounds are useful for the preparation of monocyclic and bicyclic β-lactams. No indications for the preparation of carbapenem compounds are given.

The technical problem underlying the present invention is to provide novel beta-lactam compounds which are valuable intermediates for the production of 1-alkylcarbapenem compounds having the following fundamental structure:

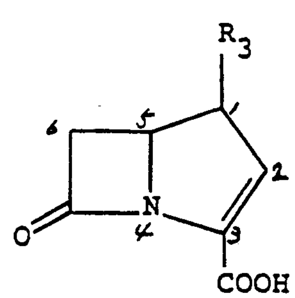

wherein $R_3$ is as defined below, particularly in making it possible to form an alkyl group at the 1-position

stereospecifically. The solution of this problem are the beta-lactam compounds of the following formula (I).

The present invention, therefore, relates to beta-lactams and their production. More particularly, it relates to novel beta-lactam compounds of the general formula:

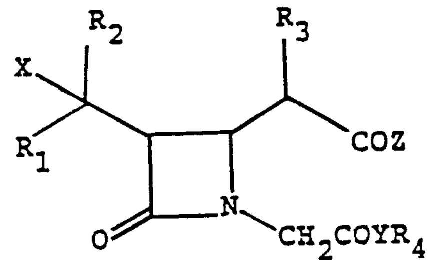

(I)

wherein $R_1$ and $R_2$ are, the same or different, each a hydrogen atom or a $C_{1-4}$ alkyl group, $R_3$ is a $C_{1-4}$ alkyl group, $R_4$ is a hydrogen atom, a conventional carboxyl-protecting group or a conventional thiolcarboxyl-protecting group, X is a hydrogen atom, a hydroxyl group or a conventionally protected hydroxyl group, Y is an oxygen atom or a sulfur atom and COZ is a carboxyl group, an activated or conventionally protected carboxyl group, a thiolcarboxyl group, or an activted or conventionally protected thiolcarboxyl group, and their production. The $C_{1-4}$ alkyl group represented by $R_1$, $R_2$ or $R_3$ may be e.g. a methyl, ethyl, n-propyl or isopropyl group.

A further technical problem underlying the present invention is to provide a novel and simple process for preparing 1-alkyl-carbapenem compounds.

The hydroxyl-protecting group (i.e. the group protecting a hydroxyl group) in the protected hydroxyl group may be a conventional group, e.g. a lower alkoxycarbonyl such as $C_1$—$C_4$ alkoxycarbonyl (e.g. t-butyloxycarbonyl), halogenated lower alkoxycarbonyl such as halogenated ($C_1$—$C_3$)alkoxycarbonyl (e.g. 2-iodethyloxycarbonyl, 2,2,2-trichloroethyloxycarbonyl), aryl(lower)alkoxycarbonyl such as phenyl ($C_1$—$C_4$)alkoxycarbonyl optionally bearing any substituent(s) on the benzene ring (e.g. benzyloxycarbonyl, o-nitrobenzyloxycarbonyl, p-nitrobenzyloxycarbonyl, p-methoxybenzyloxycarbonyl), tri(lower)alkylsilyl such as tri($C_1$—$C_4$)alkylsilyl (e.g. trimethylsilyl, t-butyldimethylsilyl), substituted methyl such as $C_1$—$C_4$ alkoxymethyl (e.g. methoxymethyl), $C_1$—$C_4$ alkoxy($C_1$—$C_4$)alkoxymethyl (e.g. 2-methoxyethoxymethyl), $C_1$—$C_4$ alkylthiomethyl (e.g. methylthiomethyl), tetrahydropyranyl, etc.

The carboxyl-protecting group (i.e. the group protecting a carboxyl group) and the thiolcarboxyl-protecting group (i.e. the group protecting a thiolcarboxyl group) may be conventional ones. Specific examples are lower alkyl such as $C_1$—$C_4$ alkyl (e.g. methyl, ethyl, isopropyl, t-butyl), halogenated lower alkyl such as halogenerated $C_1$—$C_3$ alkyl (e.g. 2-iodoethyl, 2,2,2-trichloroethyl), lower alkoxymethyl such as $C_1$—$C_4$ alkoxymethyl (e.g. methoxymethyl, ethoxymethyl, isobutoxymethyl), lower aliphatic acyloxymethyl such as $C_1$—$C_5$ alkanoyloxymethyl (e.g. acetoxymethyl, propionyloxymethyl, butyryloxymethyl, pivaloyloxymethyl), lower alkoxycarbonyloxyethyl such as 1-($C_1$—$C_4$ alkoxycarbonyloxy)ethyl (e.g. 1-methoxycarbonyloxyethyl, 1-ethoxycarbonyloxyethyl), optionally substituted lower alkenyl such as $C_3$—$C_{10}$ alkenyl optionally substituted with $C_1$—$C_4$ alkyl or phenyl (e.g. allyl, 2-methylallyl, 3-methylallyl, 3-phenylallyl), optionally substituted monoaryl(lower)alkyl such as phenyl($C_1$—$C_4$)alkyl optionally bearing any substituent(s) chosen from $C_1$—$C_4$ alkoxy, nitro, halogen and the like on the benzene ring (e.g. benzyl, p-methoxybenzyl, 2,4-dimethoxybenzyl, o-nitrobenzyl, p-nitrobenzyl, p-chlorobenzyl), optionally substituted diaryl(lower)alkyl such as diphenyl($C_1$—$C_4$)alkyl optionally bearing any substituent(s) chosen from $C_1$—$C_4$ alkoxy and the like on the benzene ring(s) (e.g. diphenylmethyl, di-p-anisylmethyl), aryl such as phenyl optionally substituted with halogen, nitro, $C_1$—$C_4$ alkoxy or the like (e.g. phenyl, p-chlorophenyl, 2,4,5-trichlorophenyl, p-nitrophenyl, o-nitrophenyl, p-methoxyphenyl), heteroaryl such as pyridyl or pyrimidyl optionally substituted with $C_1$—$C_4$ alkyl (e.g. 2-pyridyl, 3-pyridyl, 4-pyridyl, 2-pyrimidyl, 2-(4,6-dimethyl)pyrimidyl), phthalidyl, etc.

The carboxyl-activating group (i.e. the group activating a carboxyl group) and the thiolcarboxyl-activating group (i.e the group activating a thiolcarboxyl group may be the ones respectively derived from carboxyl and thiolcarboxyl so as to enhance their reactivity, and their examples include active ester, active acid anhydride, etc. Specific examples of the symbol Z are halogen (e.g. chlorine, bromine, iodine), lower alkoxycarbonyloxy such as $C_1$—$C_5$ alkoxycarbonyloxy (e.g. ethoxycarbonyloxy, isopropoxycarbonyloxy, sec-butoxycarbonyloxy), lower alkanesulfonyloxy such as $C_1$—$C_4$ alkanesulfonyloxy (e.g. methane-sulfonyloxy), arylsulfonyloxy such as phenylsulfonyloxy optionally bearing any substituent(s) on the benzene ring (e.g. p-toluenesulfonyloxy), di(lower)alkylphosphoryloxy such as di($C_1$—$C_4$)alkyl-phosphoryloxy (e.g. dimethylphosphoryloxy, diethylphosphoryloxy), diarylphosphoryloxy such as diphenylphosphoryloxy optionally beating any substituent(s) on the benzene ring (e.g. diphenylphosphoryloxy), cyclic imidoxy such as N-succinimidoxy or N-phthalimidoxy, heteroaryl such as imidazolyl or triazolyl, heterocycloalkyl such as 3-(2-thioxy)thiazolidinyl, etc.

Production of the beta-lactam compounds (I) according to the invention will be hereinafter explained in detail.

Process A:—

The beta-lactam compound of the general formula:

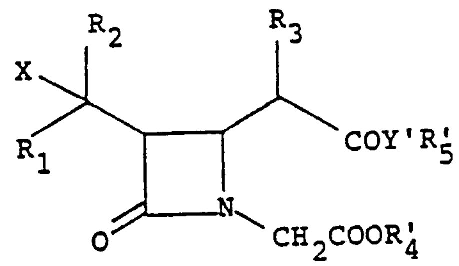

(I-1)

wherein $R_1$, $R_2$, $R_3$ and X are each as defined above, Y' is an oxygen atom or a sulfur atom, $R_4'$ is a protective group for carboxyl and $R_5'$ is a conventional protective group for carboxyl or thiolcarboxyl is obtainable by reacting a compound of the general formula:

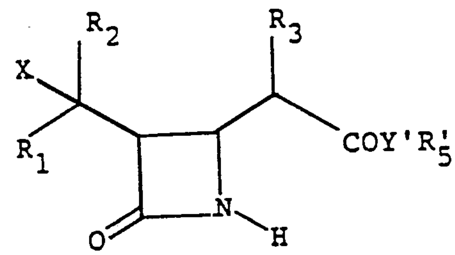

(II)

wherein $R_1$, $R_2$, $R_3$, $R_5'$, X and Y' are each as defined above with a compound of the general formula:

$$M—CH_2COOR_4' \quad (III)$$

wherein $R_4'$ is as defined above and M is an active ester of a hydroxyl group or a halogen atom, e.g. an active ester group in an inert solvent in the presence of a base. If necessary, a phase transfer catalyst may be used.

Examples of inert solvents are aromatic hydrocarbons (e.g. benzene, toluene), ethers (e.g. tetrahydrofuran, dioxane, diethyl ether), halogenated hydrocarbons (e.g. methylene chloride, dichloroethane, chloroform), ketones (e.g. acetone, methyl isobutyl, ketone), acetonitrile, dimethylformamide, dimethylsulfoxide, hexamethylphosphoric amide (HMPT), t-butanol, water, etc. These solvents may be used solely or in combination. As the base, there may be used organic bases (e.g. 1,8-diazabicyclo[5.4.0]undeca-7-ene (DBU), alkali metal hydrides (e.g. sodium hydride, potassium hydride), metal salts of amines (e.g. sodium amide, lithium diisopropylamide, lithium bis(trimethylsilyl)amide), alkali metal hydroxides (e.g. sodium hydroxide, potassium hydroxide), alkali metal carbonates (e.g. sodium carbonate, potassium carbonate), alkali metal alkoxides (e.g. potassium t-butoxide), etc. As the phase transfer catalyst, there may be employed benzyl triethyl ammonium chloride, tetra-n-butyl ammonium bromide, tetraethyl ammonium bromide, etc.

The base or the phase transfer catalyst may be used in such an amount that the reaction proceeds smoothly. Occasional heating or cooling is desirable to accelerate or control the reaction.

Still, preferred examples of active esters of a hydroxyl group represented by M are sulfonyl esters (e.g. the mesylate or tosylate) and halogen atoms (e.g. a chlorine, bromine or iodine atom).

Process B:—

(1) The beta-lactam compound of the general formula:

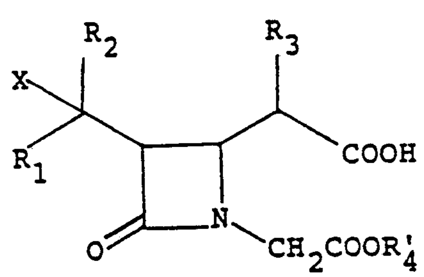

(I-2)

wherein $R_1$, $R_2$, $R_3$, $R_4'$ and X are each as defined above is obtainable by subjecting the compound (I—1) wherein Y' is a sulfur atom to selective hydrolysis. The selective hydrolysis may be carried out by a per se conventional procedure, for instance, under basic conditions.

(2) The beta-lactam compound of the general formula:

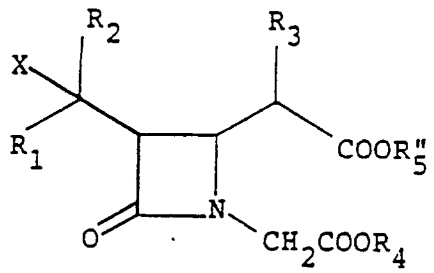

(I-3)

wherein $R_1$, $R_2$, $R_3$, $R_4$ and X are each as defined above and $R_5''$ is a hydrogen atom or a conventional protective group for carboxyl but at least either one of $R_5''$ and $R_4$ represents a hydrogen atom can be prepared by eliminating at least one of the protective groups $R_4'$ and $R_5'$ from the compound (I—1) wherein Y' is an oxygen atom.

(a) When both of $R_5''$ and $R_4$ in the compound (I—3) represent hydrogen atoms, a protective group $R_4'$ may be introduced.

(b) When $R_5''$ and $R_4$ in the compound (I—3) represent respectively a conventional protective group and a hydrogen atom, a conventional protective group $R_4'$ may be introduced, followed by selective elimination of the protective group $R_5''$. In both cases, the beta-lactam compound (I—2) can be obtained as the final product.

Elimination of the protective group(s) $R_4'$ and/or $R_5'$ may be accomplished by various per se conventional procedures depending upon their kinds, and those procedures can be chosen from hydrolysis, catalytic reduction, treatment with acids or bases, reduction, etc. For selective elimination of either one of the protective groups $R_4'$ and $R_5'$, it is convenient to select an appropriate combination of $R_4'$ and $R_5'$ so as to make such selective elimination possible. The introduction of the protective group $R_4'$ in the case (a) as well as the introduction of the protective group $R'_4$ and the subsequent elimination of the protective group $R_5''$ may be accomplished by per se conventional procedures.

Process C:—

The beta-lactam compound of the general formula:

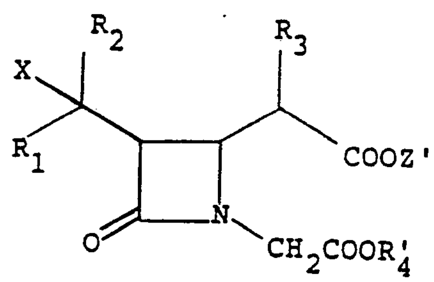

(I-4)

wherein $R_1$, $R_2$, $R_3$, $R_4'$ and X are each as defined above and COZ' is an activated or conventionally protected carboxyl group or an activated or conventionally protected thiolcarboxyl group can be produced by subjecting the compound (I—2) to any treatment for converting the carboxyl group into an activated or protected carboxyl or thiolcarboxyl group.

When the —COZ' group represents an activated carboxyl group such as active ester or active acid anhydride or a protected thiolcarboxyl group, the symbol Z' may be any one chosen from those as hereinabove exemplified for the symbol Z. In case of the —COZ' group being a substituted aryloxy group, for instance, it may be preferably p-nitrophenyloxy, o-nitrophenyloxy, 2,4,5-trichlorophenyloxy or the like. In case of the —COZ' group being a heteroaryloxy group, it is preferred to be o-pyridyloxy, p-pyridyloxy or the like.

The above conversion may be accomplished by various procedures, of which typical examples are shown below:

(a) The compound (I—2) is reacted with a halogenating agent (e.g. oxalyl chloride, thionyl chloride) in the presence or absence of a base to obtain the corresponding acid halide.

(b) The compound (I—2) is reacted with a chloroformic ester (e.g. ethyl chloroformate) in the presence

of a base to obtain the corresponding mixed acid anhydride.

(c) The compound (I—2) is reacted with 1,1'-carbonyldiimidazole to obtain the corresponding acylimidazole derivative.

(d) The compound (I—2) is reacted with thiazolidine-2-thione in the presence of a hydrating agent (e.g. dicyclohexylcarbodiimide) to obtain the corresponding acylthiazolidine-2-thion derivative.

(e) The compound (I—2) is reacted with a thiol compound such as a substituted or unsubstituted thiophenol, 4,6-dimethyl-2-mercaptopyrimidine or 2-mercaptopyridine by the aid of a dehydrating agent (e.g. dicyclohexylcarbodiimide), or is converted into its active ester such as acid halide, mixed acid anhydride or acylimidazole derivative, followed by reacting with said thiol compound.

(f) The compound (I—2) is reacted with a hydroxyl compound such as N-hydroxysuccinimide, N-hydroxyphthalimide, a substituted or unsubstituted phenol or 2-pyrridone by the aid of a dehydrating agent (e.g. dicyclohexylcarbodiimide), or is converted into its active ester such as acid halide, mixed acid anhydride or acylimidazole derivative, followed by reacting with said hydroxyl compound.

Process D:—

The beta-lactam compound of the general formula:

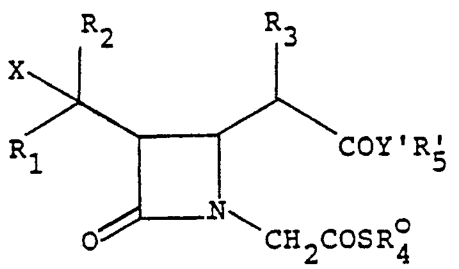

(I-6)

wherein $R_1$, $R_2$, $R_3$, $R_5'$, X and Y' are each as defined above and $R_4^o$ is a conventional thiolcarboxyl-protecting group can be obtained by reacting a compound of the general formula:

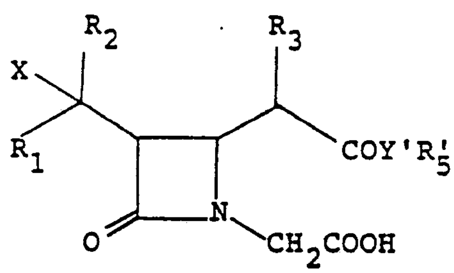

(I-5)

wherein $R_1$, $R_2$, $R_3$, $R_5$, X and Y' are each as defined above with a thiol of the general formula:

$$HSR_4^o$$

wherein $R_4$ is as defined above.

The reaction may be carried out by a per se conventional procedure for acylation of a thiol group.

Process E:—

The beta-lactam compound of the general formula:

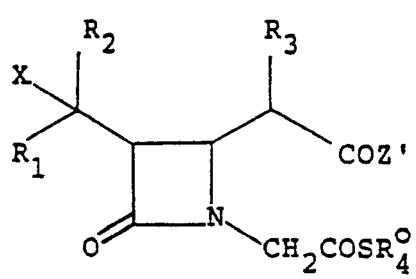

(I-7)

wherein $R_1$, $R_2$, $R_3$, $R_4^o$, X and Z' are each as defined above can be produced from the corresponding compound (I—6) according to the procedure as explained in Processes B and C.

Process F:—

The beta-lactam compound of the general formula:

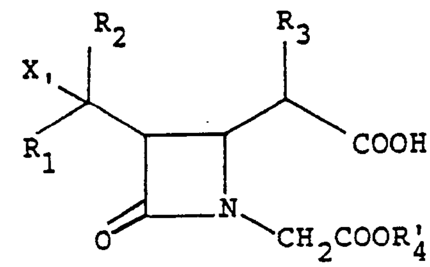

(I-2°)

wherein $R_1$, $R_2$, $R_3$ and $R_4'$ are each as defined above and X' is a hydrogen atom or a conventionally protected hydroxyl group can be produced by reacting the compound of the general formula:

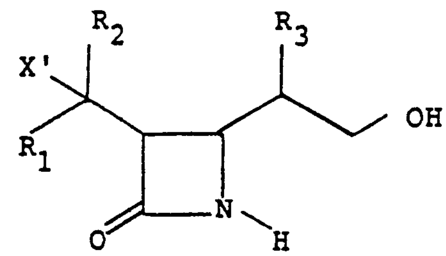

(IV)

wherein $R_1$, $R_2$, $R_3$ and X' are each as defined above with an acetic acid compound of the general formula:

$$M—CH_2COOR_4' \quad (III)$$

wherein $R_4$ and M are each as defined above in an inert solvent in the presence of a base and subjecting the resulting product of the general formula:

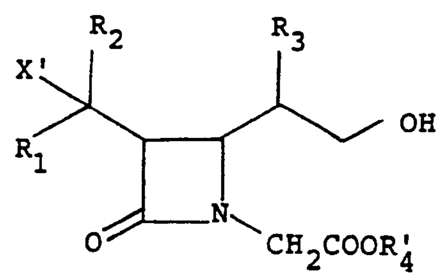

(V)

wherein $R_1$, $R_2$, $R_3$, $R_4'$ and X' are each as defined above to oxidation.

In the above process, the reaction at the first step may be carried out substantially in the same process as in Process A. The oxidation at the second step may be effected by a per se conventional procedure for conversion of a primary alcohol into the corresponding carboxylic acid, for instance, by treatment with an oxidizing agent (e.g. chromium (VI) oxide-sulfuric acid, chromium oxide-pyridine).

As stated above, the beta-lactam compounds (I) of the invention are useful as the intermediates for production of 1-alkylcarbapenem compounds. For instance, a compound of the general formula:

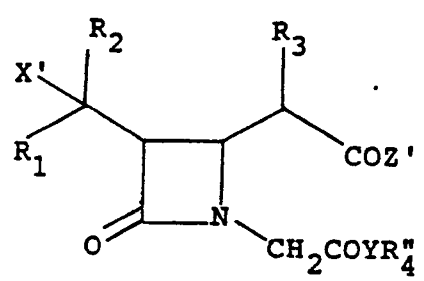

(I-8)

wherein $R_1$, $R_2$, $R_3$, Y and Z' are each as defined above, X' is a hydrogen atom or a coventionally protected hydroxyl group and $R_4''$ is a conventional carboxyl or thiolcarboxyl-protecting group, which covers the compounds (I—4) and (I—7) as well as the corresponding compounds derived therefrom, when the symbol X represents a hydroxyl group, by protection of such hydroxyl group in a per se conventional procedure, may be used as the starting material, which is converted into the 1-alkylcarbapenem compound (VII), (VIII) or (IX) in various ways, of which typical examples are shown below.

Procedure (1):-

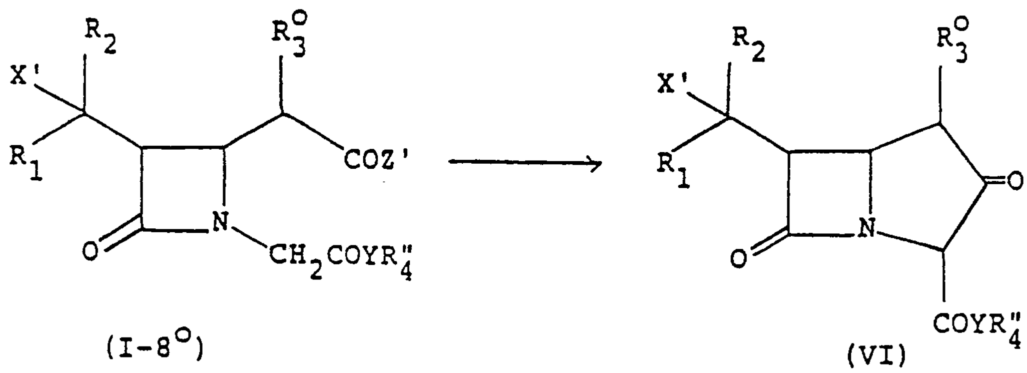

(I-8°) (VI)

wherein $R_1$, $R_2$, $R_4''$, X', Y and Z' are each as defined above and $R_3^o$ is a $C_{1-4}$-alkyl group.

The beta-lactam compound (I—8°) is treated with a base in an inert solvent to give the compound (VI). When $R_3^o$, $R_1$ and X are each a hydrogen atom and $R_2$ is a methyl group, the conversion of the compound (I—8') into the compound (VI) is known; see M. Hatanaka et al., Tetrahedron Letters, Vol. 25, No. 22 (1984), 2387—2390. When $R_3^o$ is a $C_{1-4}$-alkyl group, there can be obtained as the major product the compound (VI) retaining the steric configuration based on the asymmetric carbon atom at the 5-position bonding to the 4-position of the beta-lactam ring in the starting compound (I—8°). As the inert solvent, there may be used ethers (e.g. diethyl ether, tetrahydrofuran, dioxane, ethylene glycol dimethyl ether), aromatic hydrocarbons (e.g. benzene, toluene), acetonitrile, dimethylformamide, hexamethylphosphoric triamide (HMPT), t-butanol, etc. These solvents may be used solely or in combination. Preferred examples of the base are metal salts of amines (e.g. lithium diisopropylamide, lithium bis(trimethylsilyl)amide, sodium amide), metal salts of alcohols (e.g. potassium t-butoxide), alkali metal hydrides (e.g. sodium hydride, potassium hydride), sodium methylsulfinylmethide, etc.

The base is to be used in such an amount that the reaction can proceed smoothly, and it may be usually from 1.5 to 3 equivalents to the starting compound (I—8°). The reaction temperature may be accelerated or controlled by heating or cooling, and it may be normally from −75 to 50°C.

Recovery of the produced compound (VI) from the reaction mixture may be accomplished by application of a per se conventional procedure for post-treatment. However, post-treatment of the reaction mixture should be sometimes effected with special care, because, for instance, the alkyl group at the 1-position of the compound of the general formula:

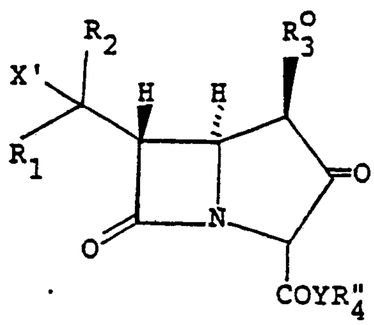

(VI-1)

wherein $R_1$, $R_2$, $R_3^o$, $R_4''$, X' and Y are each as defined above may be epimerized on treatment with a base or during concentration.

(1-a) The thus produced compound (VI) wherein Y is an oxygen atom can be converted into the corresponding antimicrobially active 1-alkylcarbepenem compound according to the following route:

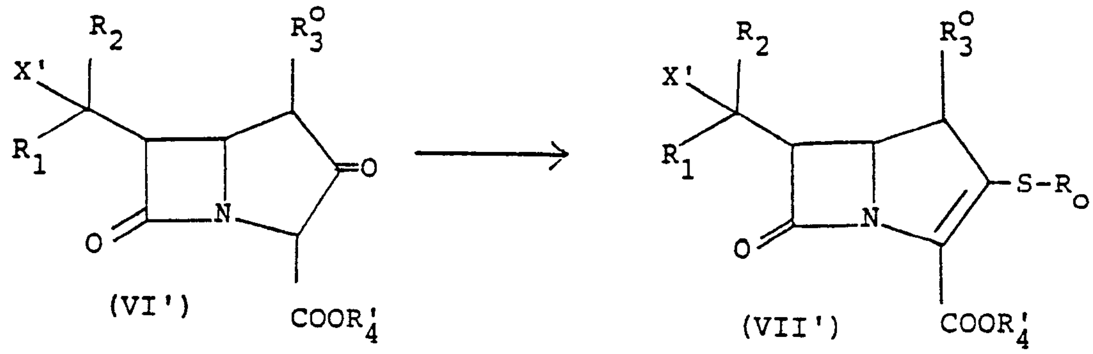

(VI')

(VII')

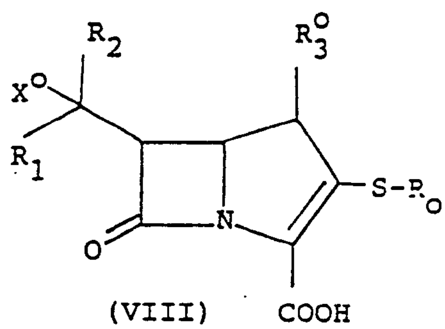

(VIII)

wherein $R_1$, $R_2$, $R_3^o$, $R_4'$ and X are each as defined above and $X^o$ is a hydrogen atom or a hydroxyl group and $R_o$ is an organic group.

The compound (VI') is first converted into the carbapenem compound (VII') by the procedure as described in U.S. Patent 4,350,631, European Patent 54,917 or Japanese Patent Publication (unexamined) No. 123182/82 or any similar procedure thereto. Then, the resulting carbapenem compound (VII') may be, if necessary, subjected to elimination of the hydroxyl-protecting group, elimination of the carboxyl-protecting group and/or elimination of the amino-protecting group to give the carbapenem compound (VIII).

Elimination of the protecting group may be accomplished by a per se conventional procedure, although it varies with the kind of the protecting group. When, for instance, the hydroxyl-protecting group and the nitrogen-protecting group in the compound (VII) are halogenated lower alkoxycarbonyl or ar(lower)alkoxycarbonyl or the carboxy-protecting group in the compound (VII) are halogenated lower alkyl, aryl(lower)alkyl or benzhydryl, it may be eliminated by application of an appropriate reduction. Such reduction may be effected using zinc with acetic acid, tetrahydrofuran or methanol in case of the protecting group being halo(lower)alkoxycarbonyl or halo(lower)alkyl, or using a catalyst such as platinum or palladium-on-carbon in case of the protecting group being ar(lower)alkyloxycarbonyl, ar(lower)alkyl or benzhydryl.

In the case using a catalyst as stated above, the reduction is normally effected in an inert solvent chosen from lower alkanols (e.g. methanol, ethanol), ethers (e.g. tetrahydrofuran, dioxane), organic acids (e.g. acetic acid), water and buffers (e.g. phosphate buffer, morpholinopropane-sulfonate buffer), etc. These solvents may be used solely or in combination. The reaction temperature may be usually from 0 to 100°C, preferably from 0 to 40°C. The hydrogen pressure may be an atmospheric or elevated one.

Still, such a protecting group as o-nitrobenzyloxycarbonyl or o-nitrobenzyl may be eliminated also by photo-reaction.

(1-b) The compound (VI) wherein Y is a sulfur atom can be converted into the corresponding carbapenem compound according to the following route:

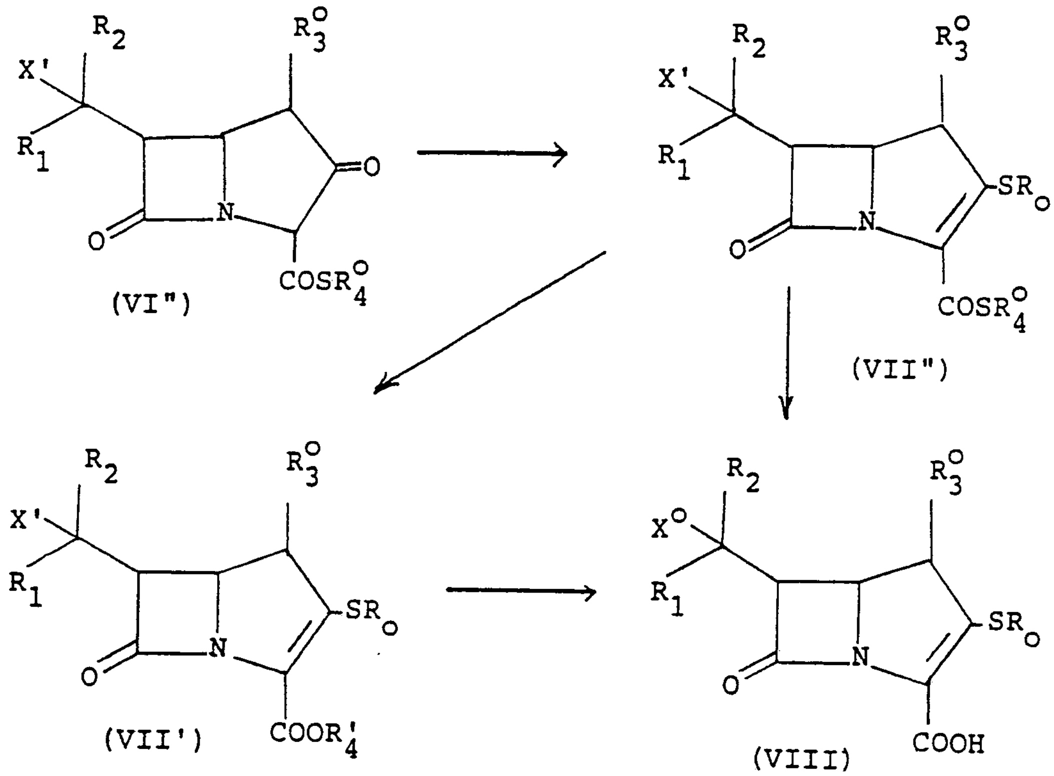

(VI") (VII") (VII') (VIII)

wherein $R_1$, $R_2$, $R_3^o$, $R_4^o$, $R_4'$, $R_o$, X' and X° are each as defined above.

The compound (VI'') is first converted into the carbapenem compound (VII'') in the same manner as in (1-a). Then, the carbapenem compound (VII'') may be, if necessary, subjected to elimination of various protecting groups. As to a thioester group, the application of a per se conventional hydrolytic procedure can successfully accomplish its elimination giving the carbapenem compound (VIII). Instead of application of the hydrolytic procedure, treatment with a silyl compound such as silanol may be applied to the carbapenem compound (VII'') so that the carbapenem compound (VIII) can be obtained directly. Alternatively, the carbapenem compound (VII'') may be treated with an alcohol in the presence of a silver salt (e.g. silver trifluoroacetate) to give the compound (VII'), which is then treated in the same manner as in (1-a) to give the carbapenem compound (VIII).

In the substituent $SR_o$ of the carbapenem compounds (VII') and (VII''), $R_o$ may be any one as heretofore used in connection with carbapenem compounds. Examples include substituted or unsubstituted alkyl or alkenyl having 1 to 10 carbon atoms; cycloalkyl, alkylcycloalkyl or cycloalkylalkyl in which the cycloalkyl group has 3 to 6 carbon atoms; aryl (e.g. phenyl), aralkyl wherein the aryl group is phenyl and the alkyl portion has 1 to 6 carbon atoms; heteroaryl, heteroarylalkyl or heterocycloalkyl, etc. These groups may be optionally bear thereon at least one substituent chosen from amino, mono-, di or trialkylamino, hydroxyl, alkoxy, mercapto, alkylthio, arylthio (e.g. phenylthio), sulfamoyl, amidino, guanidino, nitro, halo (e.g. chloro, bromo, fluoro), cyano and carboxyl. In said substituents having a hetero ring, the hetero atom(s) in the hetero ring may be chosen from oxygen, nitrogen and sulfur, and their number may be from 1 to 4. The alkyl moiety in said substituents may have 1 to 6 carbons atoms.

Procedure (2):-

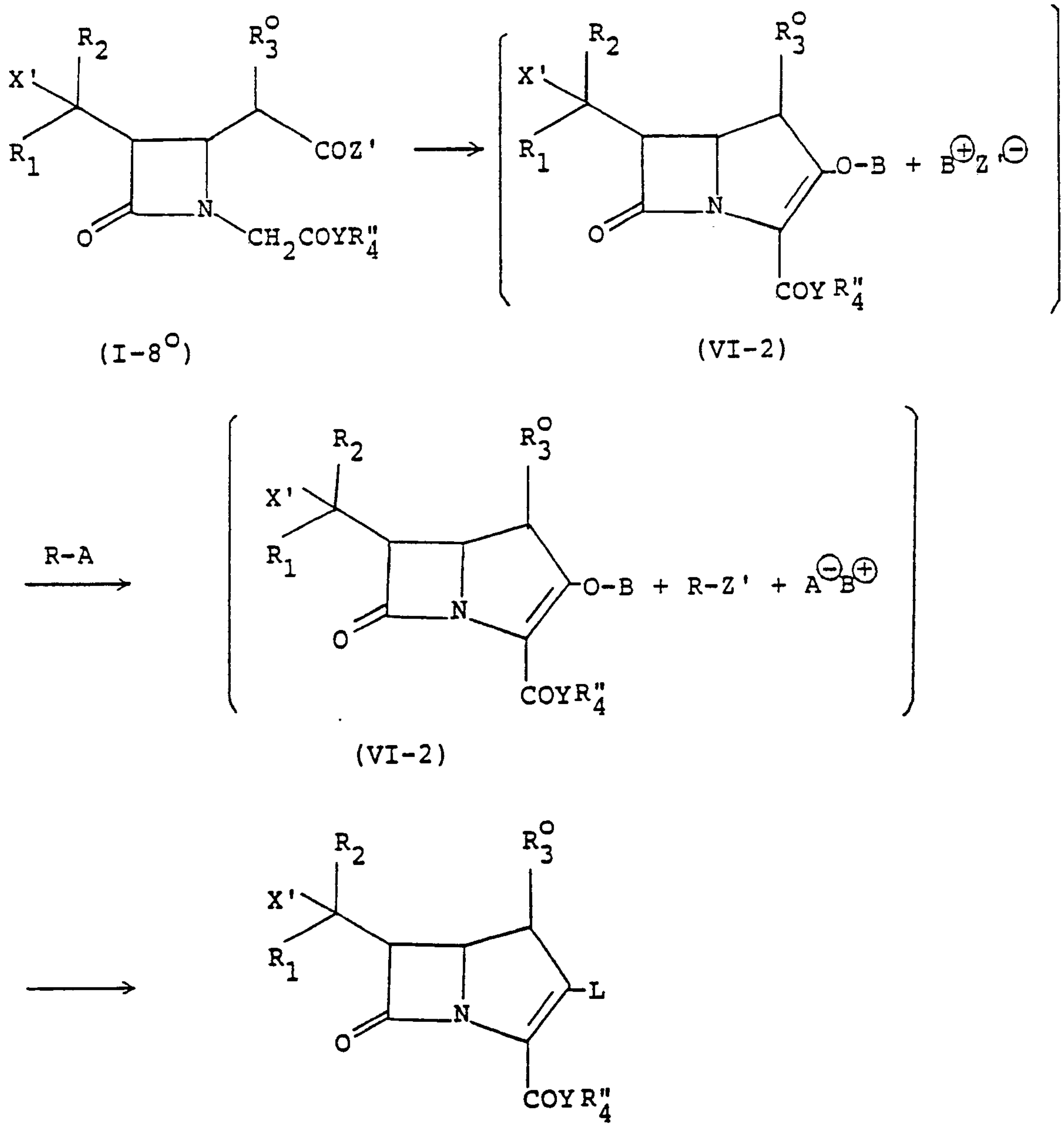


(I-8°) (VI-2)

(VI-2)

(IX)

wherein $R_1$, $R_2$, $R_3^o$, $R_4''$, X', Y and Z' are each as defined above and L is an active ester of a hydroxyl group, B is an alkali metal atom and R—A is an alkylating or acylating agent.

For direct production of the compound (IX) from the compound (I—8°), the latter is treated as in Procedure (1). Without isolation of the product, the reaction mixture is treated with an alkylating or acylating agent (e.g. iodomethane, iodopropane, allyl bromide, benzyl bromide, methyl p-toluene sulfonate) so as to catch the residue of the activating group such as an active ester residue, an active acid anhydride residue or a thiol residue, followed by treatment with a hydroxyl-activating agent such as an active esterifying agent for hydroxyl to give the carbapenem compound (IX). The treatment with the alkylating or acylating agent is preferably carried out in the presence of a base in an inert solvent.

As the active ester of a hydroxyl group represnted by the symbol L, there are exemplified substituted or unsubstituted arylsulfonic esters (e.g. benzenesulfonic esters, p-toluenesulfonic esters, p-nitrobenzenesulfonic esters, p-bromobenzenesulfonic esters), lower alkanesulfonic esters (e.g. methanesulfonic esters, ethanesulfonic esters), halo(lower)alkanesulfonic esters (e.g. trifluoromethanesulfonic esters), diarylphosphoric esters (e.g. diphenylphosphoric esters), halides (equal to esters with hydrogen halides) (e.g. chlorides, bromides, iodides), etc. Preferred are p-toluenesulfonic esters, methanesulfonic esters,

diphenylphosphoric esters, etc. Accordingly, any reagent which is reacted with the compound (VI—2) to give the active ester as exemplified above may be used as the active esterifying agent. Examples of the alkali metal atom represented by the symbol B are lithium, sodium, potassium, etc. As the base, there may be used the one as exemplified in Procedure (1) for production of the compound (VI).

When the symbol $R_3^o$ in the compound (I—8°) is a $C_{1-4}$-alkyl group, its treatment with a base in an inert solvent affords the enolate salt (VI—2), which retains the steric configuration on the basis of the asymmetric carbon atom at the 5-position of the compound (I—8°). Even after conversion of the enolate (VI—2) into the compound (IX), the steric configuration of the alkyl group represented by the symbol $R_3^o$ is unchanged. Thus, adoption of this Procedure (2) gives the carbapenem compound (IX) without epimerization. Still, the enolate (VI—2) in this case has a possibility of taking a chelate structure of the general formula:

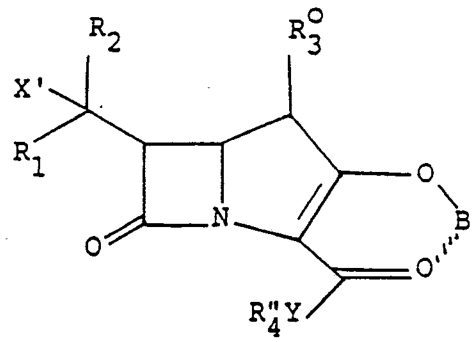

wherein $R_1$, $R_2$, $R_3$, $R_4''$, X', Y and B are each as defined above.

The active esterifying agent is to be used in an amount sufficient to effect the reaction smoothly, and its amount may be from 1 to 1.5 equivalents to the compound (I—8°). The reaction temperature may be usually from −78 to 60°C, preferably from −40 to 10°C.

Procedure (3):-

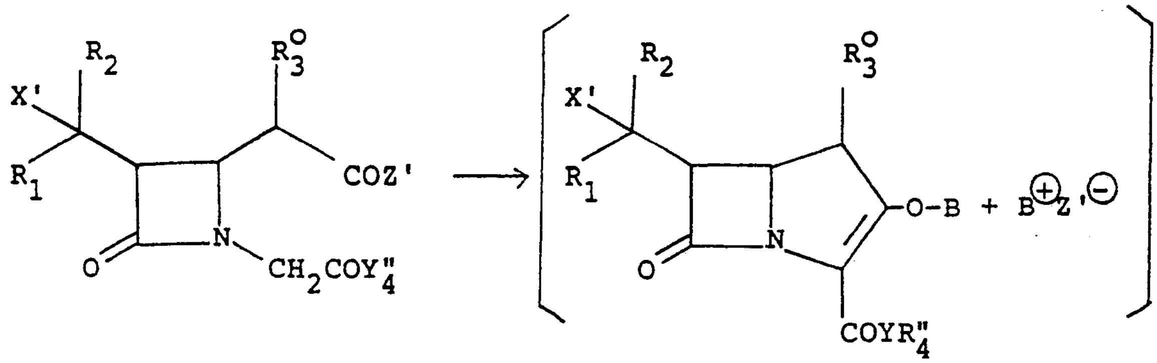

(I-8°)          (VI-2)

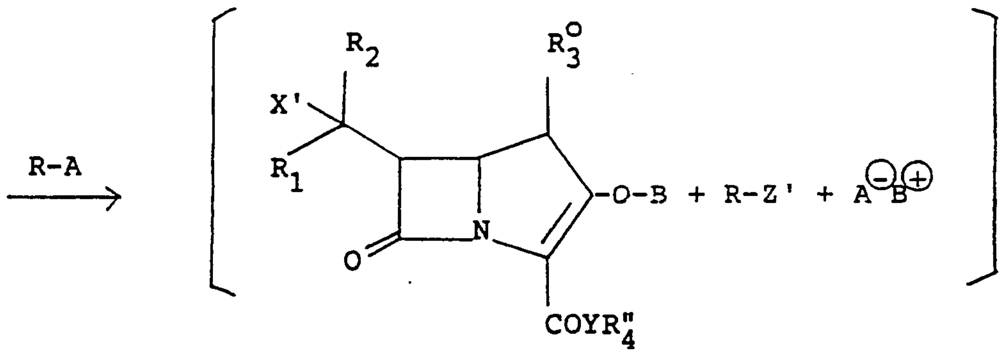


(VI-2)

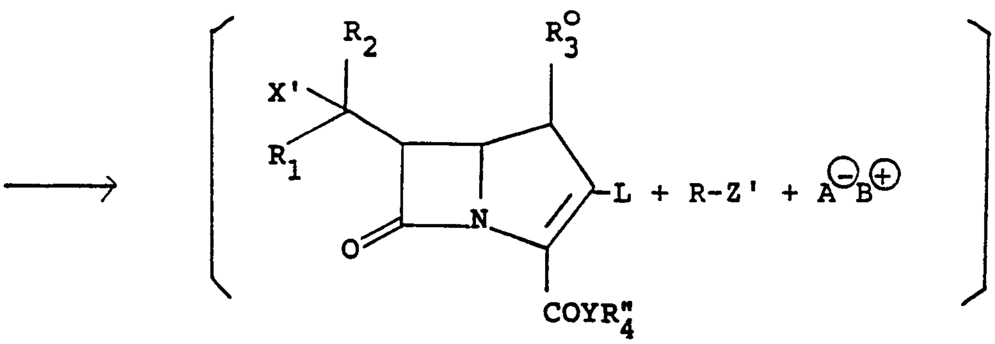

(IX)

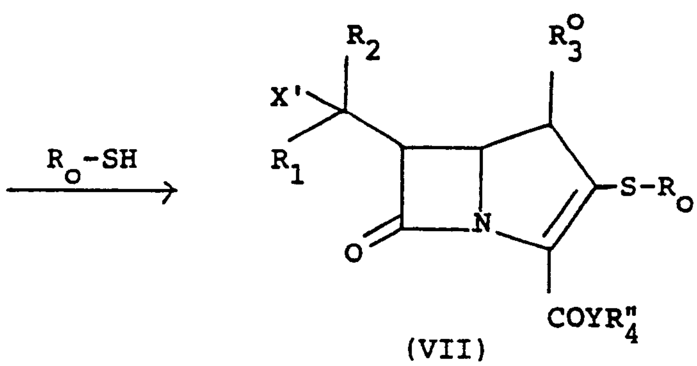


(VII)

wherein $R_1$, $R_2$, $R_3^o$, $R_4''$, R—A, B, X′, Y and Z′ are each as defined above.

When direct production of the carbapenem compound (VII) from the compound (I—8°) is desired, the latter may be converted into the carbapenem compound (IX) in the same manner as Procedure (2). Without isolation of the compound (IX), the reaction mixture is treated with a mercaptan compound of the general formula:

$$R_o—SH \qquad (X)$$

wherein $R_0$ is as defined above in the presence of a base to give the carbapenem compound (VII). The base to be used in the treatment with the mercaptan compound (X) may be the same as or different from that as used in the cyclization of the compound (I—8°) to the compound (IX). Likewise, the inert solvent to be used in said treatment may be the same as or different from that as used in said cyclization.

As the base, there may be used the same as exemplified in Procedure (1). Other examples of the base usable are organic amines such as triethylamine, diisopropylethylamine, 4-dimethylaminopyridine, 1,8-diazabicyclo[5.4.0]-7-undecene (DBU), 1,5-diazabicyclo[4.3.0]-5-nonene (DBN) and 1,4-diazabicyclo-[2.2.2]octane (DABCO). Preferred examples of the solvent which is used for smooth proceeding of the reaction are acetonitrile, dimethylformamide, dimethylsulfoxide, etc.

The base to be used together with the mercaptan compound (X) may be employed in such an amount as can assure the smooth proceeding of the reaction, and its amount may be in a large excess, preferably from 1 to 2 equivalents to the compounds (I—8°). The mercaptan compound (X) and the base may be introduced into the reaction system separately. Alternatively, the salts formed between them may be added to the reaction system.

Conversion of the beta-lactam compound (I—8°) into the carbapenem compound (IX) may be accomplished by carrying out the reactions as above explained in order. When desired, the carbapenem compound (IX) is subjected to hydrolysis or elimination of the protecting group so that the carbapenem compound (VII) can be obtained. The hydrolysis or the elimination of the protecting group may be carried out in the same manner as in Procedure (1-a) (i.e. conversion of the compound (VII') into the compound (VIII)) or (1-b) (i.e. conversion of the compound (VII'') into the compound (VIII)).

A typical example of the conversion from the beta-lactam compound (I—8°) into the 1-beta-methylcarbapenem compound is shown below:

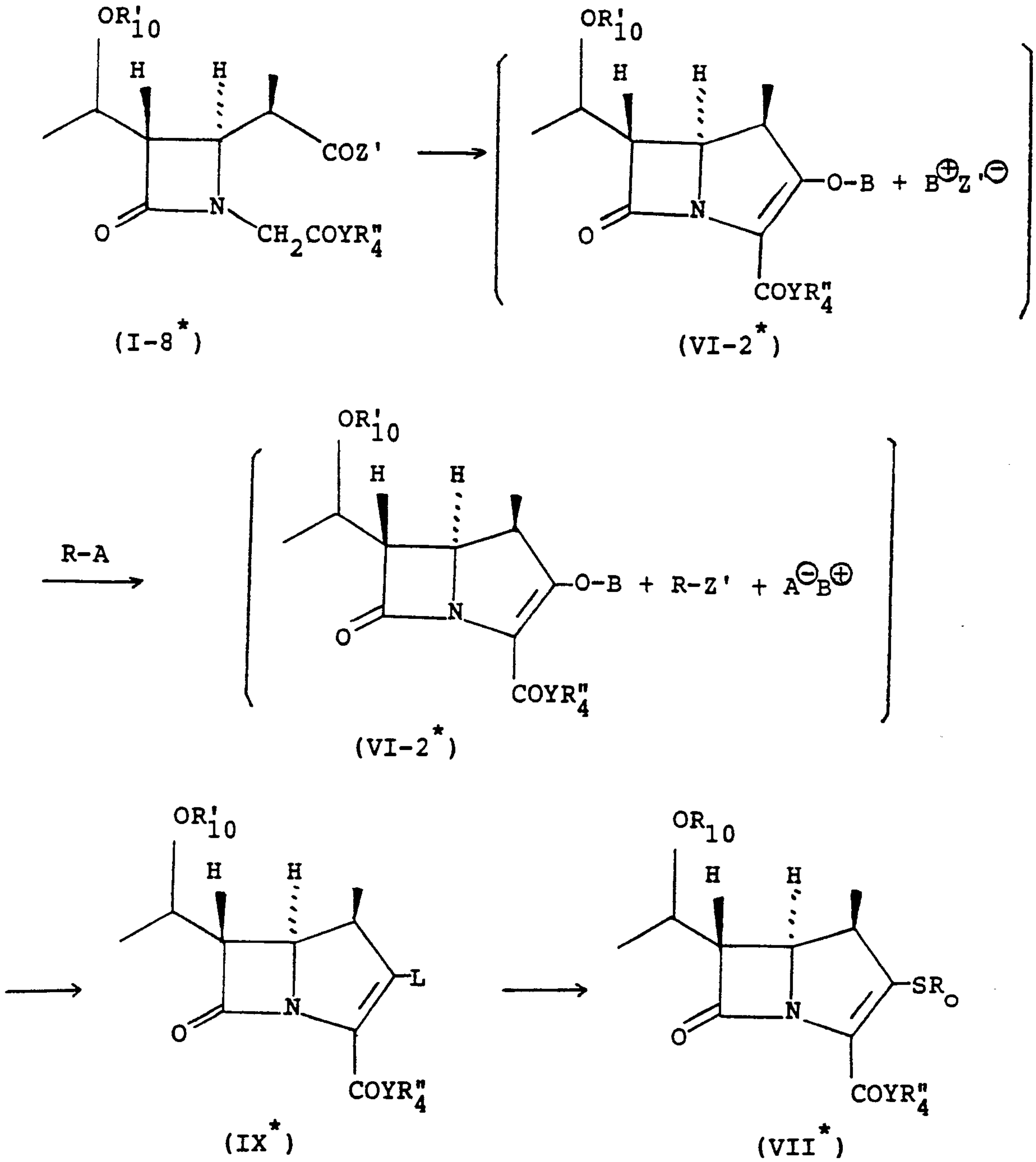


(I-8*) (VI-2*)

(VI-2*)

(IX*) (VII*)

wherein $R_4''$, Y, Z', B, R—A, L and $R_o$ are each as defined above and $R_{10}'$ is a hydroxyl-protecting group.

Namely, 1) the beta-lactam compound (I—8*) is treated with a base in an inert solvent, 2) the residue $Z'^-$ is caught with an alkylating or acylating agent and then 3) the resulting product is treated with a hydroxyl-activating group such as an active esterifying agent for hydroxyl. When desired, 4) the resultant product is reacted with the mercaptan compound (X) in the presence of a base or the salt of the mercaptan compound (X) with the base. These reactions may be carried out in a single reaction vessel in order to give the carbapenem compound (IX*) or (VII*).

Alternatively, the compound (I—8*) may be subjected to the reaction in 1), followed by post-treatment to give the compound of the general formula:

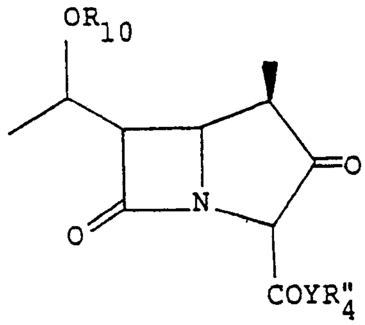

(VI*)

wherein $R_4''$, $R_{10}$ and Y are each as defined above. The beta-methyl group at the 1-position is apt to be epimerized when the product is stored in a highly concentrated solution or in a polar solvent such as acetonitrile. Thus, the stereo-specific production of the compound (IX*) from the compound (VI*) as once isolated in a large scale would have a technical problem. To the contrary, the conversion of not the compound (VI*) but the compound (VI—2'') into the compound (IX*) is advantageous, because the production of the compound (IX*) or (VII*) can be accomplished without epimerization of the methyl group at the 1-beta-position.

The compound (II) as the starting material in production of the beta-lactam compound (I) according to this invention can be produced in the manner as described in U.S. Patent 4,350,631, European Patent 54917 or Japanese Patent Publication (unexamined) No. 123182/82. For production of the compound (II) wherein Y is a sulfur atom, introduction of the $—SR_4$ group may be carried out by a conventional procedure as in the case of production of the compound (I—6). While the starting compound (IV) can be produced in the same manner as described in European Patent 10317 or Japanese Patent Publication (unexamined) No. 89285/80, it may be produced according to the route as shown in the following scheme:

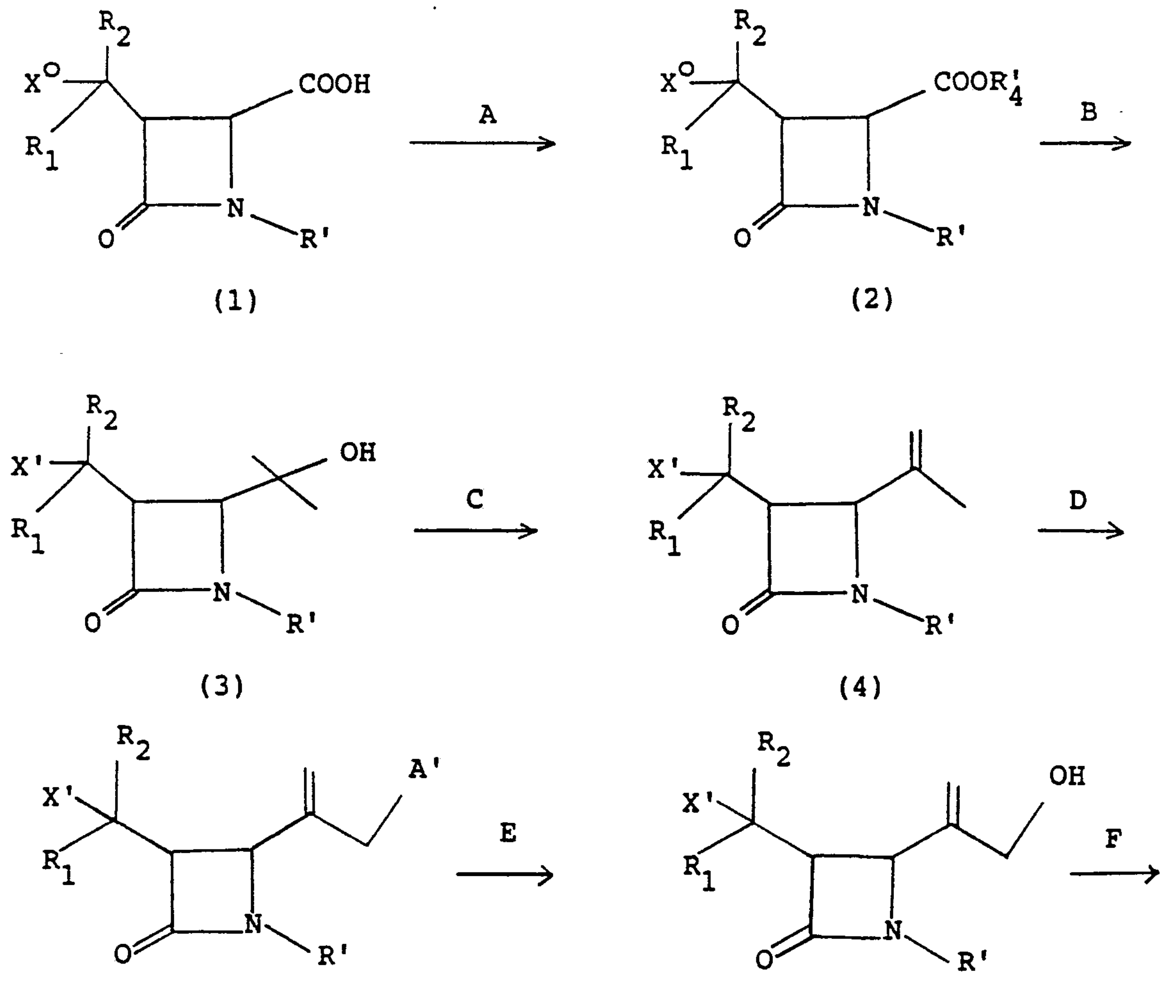


(1) (2) (3) (4) (5) (6)

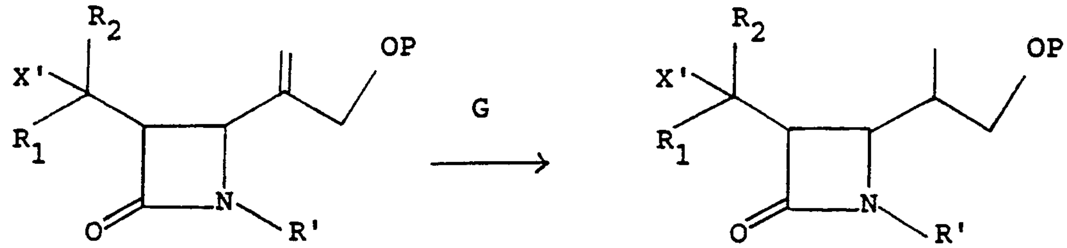


(7) (8)

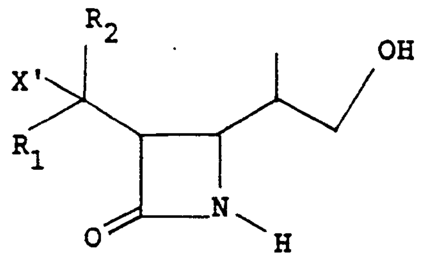

(IVa)

wherein $R_1$, $R_2$, $X^\circ$ and X′ are each as defined above, R′ is a nitrogen-protecting group, A′ is a halogen atom and P is a hydroxyl-protecting group.

In the above scheme, the step A is directed to conversion of the carboxylic acid (1) into the corresponding ester (2) The conversion is usually carried out by application of a per se conventional esterification procedure. For instance, the carboxylic acid (1) obtained by the process as described in Japanese Patent Publication (unexamined) No. 96060/83 may be reacted with an alkyl halide in the presence of an acid-eliminating agent or with an alkanol in the presence of a dehydrating agent to give the ester (2).

At the step B, the ester (2) is reduced by treatment with an organic metal compound such as magnesium halide or methyl lithium in an inert solvent, optionally followed by protection of a hydroxyl group in a per se conventional procedure to give the corresponding alcohol (3).

At the step C, the alcohol (3) is dehydrated by treatment with a dehydrating agent such as thionyl chloride or tosyl chloride in the presence or absence of a base to give the corresponding methylene compound (4).

At the step D, the methylene compound (4) is halogenated by treatment with a halogenating agent such as molecular halogen or N-halogenosuccinimide in an inert solvent to give the corresponding halogenated compound (5).

At the step E, the halogenated compound (5) is hydrolyzed by treatment with water in the presence of a low atomic valency ion salt of a heavy metal (e.g. copper, silver) to give the corresponding hydroxyl compound (6).

At the step F, the hydroxyl compound (6) is subjected to protection on the hydroxyl group to give the corresponding protected hydroxyl compound (7).

At the step G, the protected hydroxyl compound (7) is hydrogenated by catalytic hydrogenation to give the corresponding methyl compound (8).

At the step H, the methyl compound (8) is subjected to elimination of the hydroxyl-protecting group represented by the symbol P and elimination of the amino-protecting group represented by the symbol R′ simultaneously or stepwise to give the corresponding free compound (IVa).

In the beta-lactam compound (I), the carbon atoms at the 3- and 4-positions and the carbon atom bonding to the beta-lactam ring and in the substituent attached to the 4-position of such beta-lactam ring are all asymmetric carbon atoms. Further, in the case that all of $R_1$, $R_2$ and X are different one another (e.g. $R_1$ = methyl, $R_2$ = hydrogen, X = hydroxyl), the carbon atom bonding to the beta-lactam ring and in the substituent attached to the 3-position of such beta-lactam ring is an asymmetric carbon atom.

Accordingly, the beta-lactam compound of the formula (I) covers optical isomers and stereo isomers due to said asymmetric carbon atoms. Among those optical isomers and stereo isomers, the compounds of the following general formula:

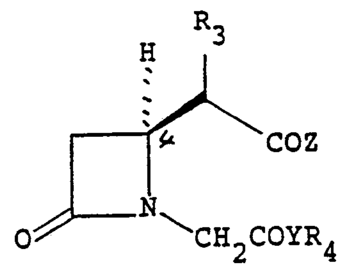

wherein $R_3$, $R_4$ and Y and Z are each as defined above are particularly preferred in having the same configuration as that of naturally occuring thienamycin on the carbon atom at the 4-position.

Practical and presently preferred embodiments of the invention are illustratively shown in the following Examples and References Examples. This invention is, however, not limited to these examples. In these examples, the abbreviations have the following meanings: TBDMS, t-butyldimethylsilyl; Ph, phenyl; tBu, t-butyl; PNB, p-nitrobenzyl; PMB, p-methoxybenzyl; Im, 1-imidazolyl; Bt, 1-benzotriazolyl; Ac, acetyl; PNZ, p-nitrobenzyloxycarbonyl; DAM, di-(p-anisyl)methyl; Z, benzyloxycarbonyl; Me, methyl.

Example 1-1

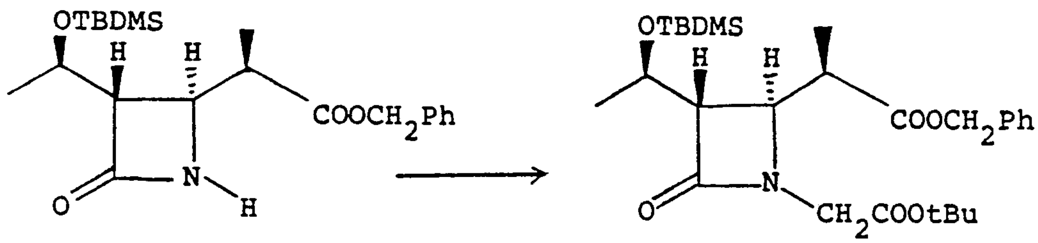

To a solution of (3S,4S)-3-[(1R)-1-t-butyldimethylsilyloxyethyl]-4-[(1R)-1-benzyloxycarbonylethyl]-azetidin-2-one (755 mg) in methylene chloride (10 ml), there were added successively t-butyl bromoacetate (1.88 g), 50% sodium hydroxide (620 mg) and triethylbenzylammonium chloride (220 mg), followed by stirring at room temperature for 2 hours. The reaction mixture was diluted with water and diethyl ether. The aqueous layer was separated from the organic layer and extracted two times with diethyl ether. The extracts were combined with the organic layer, washed with water two times and brine three times, dried over sodium sulfate and evaporated. The residue was purified by silica gel chromatography to give (3S,4S)-3 - [(1R) - 1 - t - butyldimethylsilyloxyethyl] - 4 - [(1R) - 1 - benzyloxycarbonylethyl] - 1 - (t - butyloxycarbonylmethyl)azetidin - 2 - one.

IR $\nu_{max}^{neat}(cm^{-1})$: 1755, 1730, 1450, 1400, 1380, 1360, 1242, 1220, 1150, 830, 765, 740, 685.

NMR δ ($CDCl_3$): 0.04 (3H, s), 0.07 (3H, s), 0.85 (9H, s), 1.23 (3H, d, J=6.3Hz), 1.24 (3H, d, J=6.9Hz), 1.44 (9H, s), 2.90 (1H, qd, J=6.9 and 3.6Hz), 2.99 (1H, dd, J=2.0 and 6.6Hz), 3.83 (2H, m), 5.10 (2H, s), 7.35 (5H, s).

Example 1-2

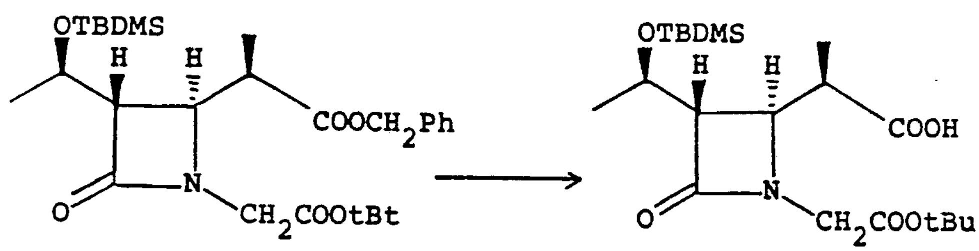

A solution of (3S,4S)-3-[(1R)-1-t-butyldimethylsilyloxyethyl]-4-[(1R)-1-benzyloxycarbonylethyl]-1-(t-butyloxycarbonylmethyl)azetidin-2-one (0.45 g) in 99.5% ethanol (6 ml) was subjected to hydrogenation at room temperature in the presence of 10% palladium-carbon (90 mg) under atomospheric pressure, followed by filtration to remove the catalyst. The filtrate was evaporated to give (3S,4S)-3-[(1R)-1-t-butyldimethylsilyloxyethyl]-4-[(1R)-1-carboxyethyl]-1-(t-butyloxycarbonylmethyl)azetidin-2-one.

IR $\nu_{max}^{neat}(cm^{-1})$: 1760, 1740, 1730, 1455, 1360, 1245, 1224, 1150, 830, 770, 745.

NMR δ ($CDCl_3$): 0.06 (3H, s), 0.08 (3H, s), 0.87 (9H, s), 1.24 (3H, d, J=6.3Hz), 1.25 (3H, d, J=7.3Hz), 1.48 (9H, s), 2.94 (1H, qd, J=7.1 and 3.0Hz), 3.04 (1H, dd, J=2.3 and 5.5Hz), 3.98 (2H, m), 4.00 (1H, m), 4.21 (1H, m).

Example 1-3(1)

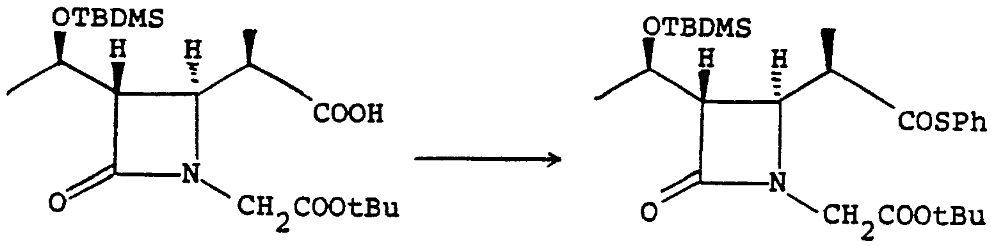

A mixture of (3S,4S)-3-[(1R)-1-t-butyldimethylsilyloxyethyl]-4-[(1R)-1-carboxyethyl]-1-(t-butyloxycarbonylmethyl)azetidin-2-one (1.29 g) and N,N'-carbonyldiimidazole (604 mg) in dry acetonitrile (25 ml) was stirred at room temperature for 1 hour. To the mixture, there were added successively a solution of thiophenol (410 mg) in dry acetonitrile (6 ml) and a solution of triethylamine (377 mg) in dry acetonitrile (6 ml). After stirring at room temperature for 0.5 hour, the reaction mixture was diluted with ethyl acetate and dilute hydrochloric acid. The aqueous layer was separated from the organic layer and extracted with ethyl acetate three times. The extracts were combined with the organic layer, washed with brine two times, dried over sodium sulfate and evaporated. The residue was purified by silica gel chromatography to give (3S,4S)-3 - [(1R) - 1 - t - butyldimethylsilyloxyethyl] - 4 - [(1R) - 1 - phenylthiocarbonylethyl] - 1 - (t - butyloxycarbonylmethyl)azetidin - 2 - one.

IR $\nu_{max}^{neat}(cm^{-1})$: 1760, 1740, 1705, 1367, 1250, 1227, 835, 770, 740.

Example 1-3(2)

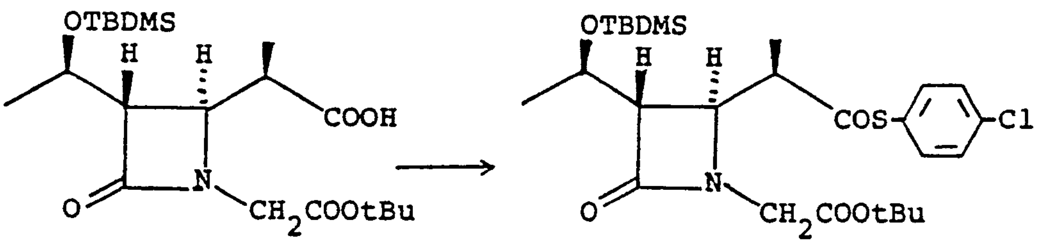

In the same manner as in Example 1-3(1) but replacing thiophenol by p-chlorothiophenol, there was obtained (3S,4S)-3-[(1R)-1-t-butyldimethylsilyloxyethyl]-4-[(1R)-1-p-chlorophenylthiocarbonylethyl]-1-(t-butoxycarbonylmethyl)azetidin-2-one.

IR $\nu_{max}^{neat}(cm^{-1})$: 1760, 1740, 1705, 1480, 1365, 1260, 1230, 1155, 1095, 838, 775.

NMR δ ($CDCl_3$): 0.10 (6H, s), 0.89 (9H, s), 1.26 (3H, d, J=6.3Hz), 1.31 (3H, d, J=6.9Hz), 1.43 (9H, s), 3.02 (1H, dd, J=2.3 and 6.9Hz), 3.14 (1H, qd, J=3.3 and 6.9Hz), 3.92 (2H, m), 7.34 (4H, m).

Example 1-3(3)

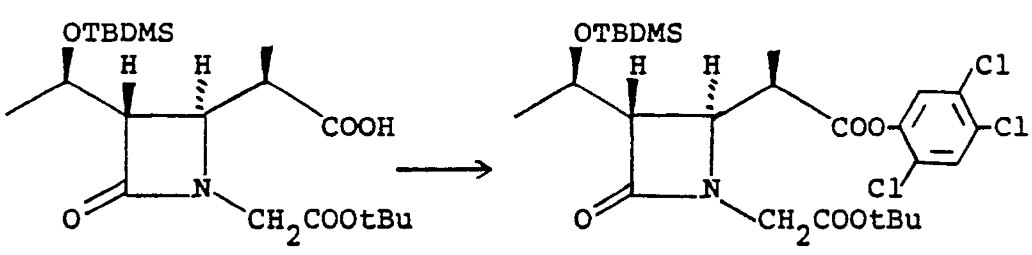

To a solution of (3S,4S)-3-[(1R)-1-t-butyldimethylsilyloxyethyl]-4-[(1R)-1-carboxyethyl]-1-(t-butyloxycarbonylmethyl)azetidin-2-one (100 mg) and 2,4,5-trichlorophenol (33 mg) in dry tetrahydrofuran (4 ml), there was added 1-ethyl-3-(3-dimethylaminopropyl)carbodiimide hydrochloride (96 mg) under ice-cooling, followed by stirring overnight. The reaction mixture was diluted with diethyl ether and water. The organic layer was separated from the aqueous layer, washed with brine, dried over sodium sulfate and distilled off to remove the solvent to give (3S,4S)-3-[(1R)-1-t-butyldimethylsilyloxyethyl]-4-[(1R)-1-(2,4,5-trichlorophenyloxy)carbonylethyl]-1-(t-butyloxycarbonylmethyl)azetidin-2-one.

IR $\nu_{max}^{neat}(cm^{-1})$: 1760, 1740 (sh), 1455, 1362, 1250, 1225.

Example 1-3(4)

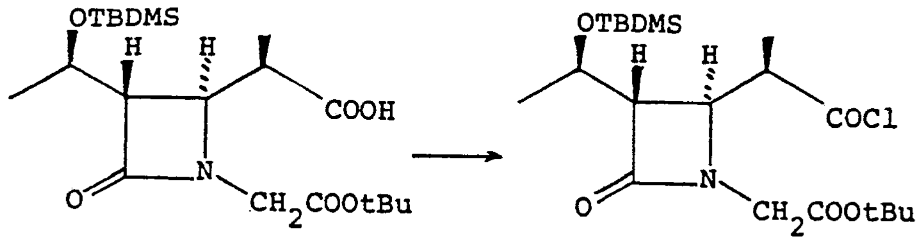

To a solution of (3S,4S)-3-[(1R)-1-t-butyldimethylsilyloxyethyl]-4-[(1R)-1-carboxyethyl]-1-(t-butyloxycarbonylmethyl)azetidin-2-one (98 mg) in dry methylene chloride (1 ml), there was added a solution of thionyl chloride (34 mg) in dry methylene chloride (0.5 ml) at room temperature. The resultant mixture was stirred at the same temperature for 1 hour and then boiled under reflux for 3 hours. After removal of the solvent, the residue was dissolved in dry toluene, again distilled off to remove the solvent and dried in vacuo to give (3S,4S)-3-[(1R)-1-t-butyldimethylsilyloxyethyl]-4-[(1R)-1-chlorocarbonylethyl]-1-(t-butyloxycarbonylmethyl)azetidin-2-one.

IR $\nu_{max}^{neat}(cm^{-1})$: 1800 (sh), 1740, 1450, 1360, 1244, 1220, 930, 770.

Example 1-3(5)

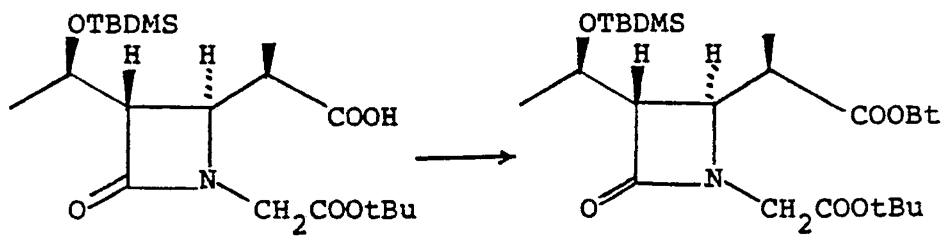

In the same manner as in Example 1-3(3), (3S,4S)-3-[(1R)-1-t-butyldimethylsilyloxyethyl]-4-[(1R)-1-carboxyethyl]-1-(t-butyloxycarbonylmethyl)azetidin-2-one (104 mg) was treated with 1-oxybenztriazole (53 mg) and 1-ethyl-3-(3-dimethylaminopropyl)carbodiimide hydrochloride (93 mg) to give (3S,4S)-3-[(1R)-1-t-butyldimethylsilyloxyethyl] - 4 - [(1R) - 1 - (1 - benzotriazolyloxy)carbonylethyl] - 1 - (t - butyloxycarbonylmethyl)azetidin - 2 - one.

IR $\nu_{max}^{neat}(cm^{-1})$: 1740 (sh), 1730, 1450, 1360, 1240, 1220, 822.

Example 1-3(6)

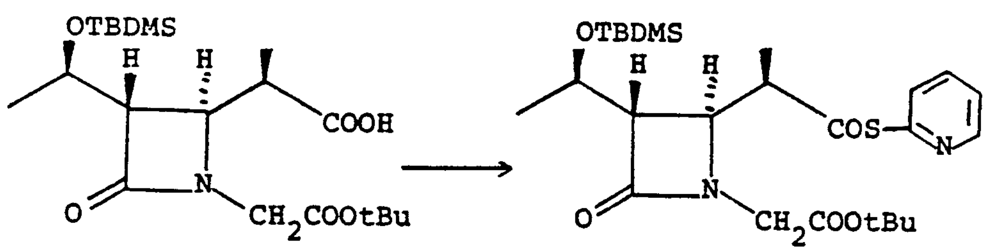

In the same manner as in Example 1-3(3), (3S,4S)-3-[(1R)-1-t-butyldimethylsilyloxyethyl]-4-[(1R)-1-carboxyethyl]-1-(t-butyloxycarbonylmethyl)azetidin-2-one (100 mg) was treated with 2-mercaptopyridine (35 mg) and 1-ethyl-3-(3-dimethylaminopropyl)carbodiimide hydrochloride (100 mg) to give (3S,4S)-3-[(1R) - 1 - t - butyldimethylsilyloxyethyl] - 4 - [(1R) - 1 - (2 - pyridylthio)carbonylethyl] - 1 - (t - butyloxycarbonylmethyl)azetidin - 2 - one.

IR $\nu_{max}^{neat}(cm^{-1})$: 1755, 1690, 1360, 1247, 1220, 1142, 830, 770.

Example 1-4

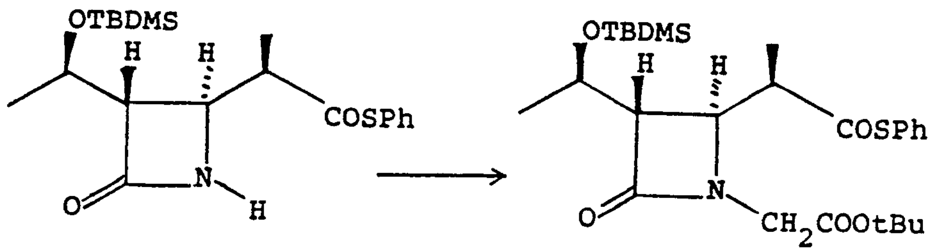

To a suspension of sodium hydride (31 mg) in dry dimethylformamide (4.3 ml), there were added successively t-butyl alpha-bromacetate (835 mg) and (3S,4S)-3-[(1R)-1-t-butyldimethylsilyloxyethyl]-4-[(1R)-1-phenylthicarbonylethyl]azetidin-2-one (0.42 g), and the resultant mixture was stirred at room temperature for 1 hour under a nitrogen stream. The reaction mixture was diluted with diethyl ether and adjusted to pH 6.86 with a phosphoric acid buffer solution. The aqueous layer was separated from the organic layer and extracted with diethyl ether three times. The extracts were combined with the organic layer, washed three times with brine, dried over sodium sulfate and evaporated. The residue was purified by silica gel chromatography to give (3S,4S)-3-[(1R)-1-t-butyldimethylsilyloxyethyl]-4-[(1R)-1-phenylthiocarbonylethyl]-1-(t-butyloxycarbonylmethyl)azetidin-2-one.

The IR spectrum of the product thus obtained was identical with that of the product in Example 1-3(1).

Example 1-5

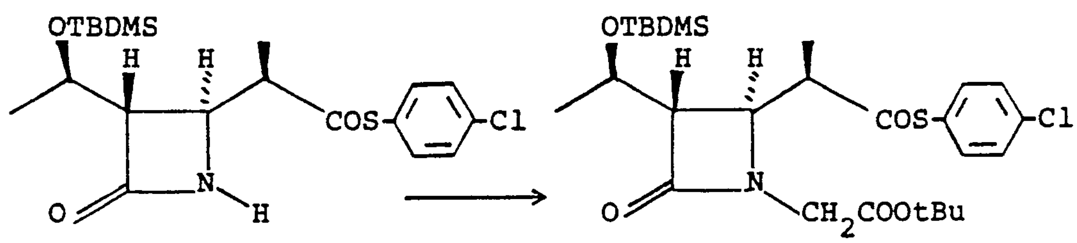

In the same manner as in Example 1-1, there was obtained (3S,4S)-3-[(1R)-1-t-butyldimethylsilyloxyethyl]-4-[(1R)-1-p-chlorophenylthiocarbonylethyl]-1-(t-butyloxycarbonylmethyl)azetidin-2-onefrom (3S,4S)-3-[(1R)-1-t-butyldimethylsilyloxyethyl]-4-[(1R)-1-p-chlorophenylthiocarbonylethyl)azetidin-2-one.

The IR spectrum and NMR spectrum of the product thus obtained were identical with those of the product in Example 1-3(2).

Example 2-1

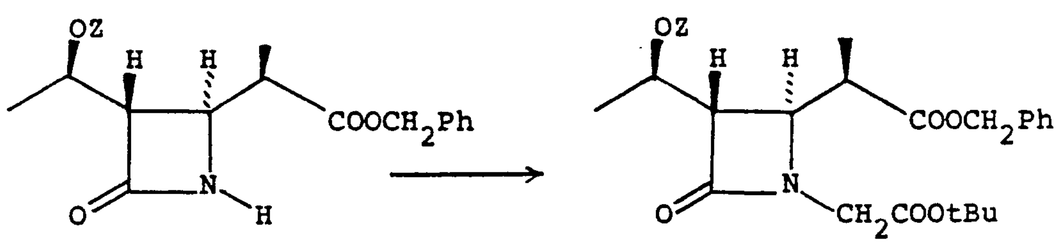

To a solution of (3S,4S)-3-[(1R)-1-t-benzyloxycarbonyloxyethyl]-4-[(1R)-1-benzyloxycarbonylethyl]azetidin-2-one (71.94 g) in dry dimethylformamide (700 ml), there were added successively t-butyl bromoacetate (68.25 g) and sodium hydride (9.24 g, 50% oil suspension) with ice-cooling, followed by stirring for 1 hour. The reaction mixture was diluted with a 10% aqueous ammonium chloride solution (50 ml), stirred for 30 minutes and extracted with toluene (2 liters). The extract was washed with brine, dried over sodium sulfate and evaporated. The residue was purified by silica gel chromatography to give (3S,4S)-3 - [(1R) - 1 - benzyloxycarbonyloxyethyl] - 4 - [(1R) - 1 - benzyloxycarbonylethyl] - 1 - (t - butyloxycarbonylmethyl)azetidin - 2 - one.

IR $\nu_{max}^{neat}(cm^{-1})$: 1765 (sh), 1740, 1455, 1370, 1268, 1160.

NMR δ ($CDCl_3$): 1.18 (3H, d, J=6.9Hz), 1.45 (3H, d, J=6.3Hz), 1.45 (9H, s), 2.86 (1H, m), 3.26 (1H, dd, J=2.0 and 9.0Hz), 3.55 (1H, d, J=18Hz), 4.04 (1H, dd, J=2.0 and 4.6Hz), 4.10 (1H, d, J=18Hz).

Example 2-2

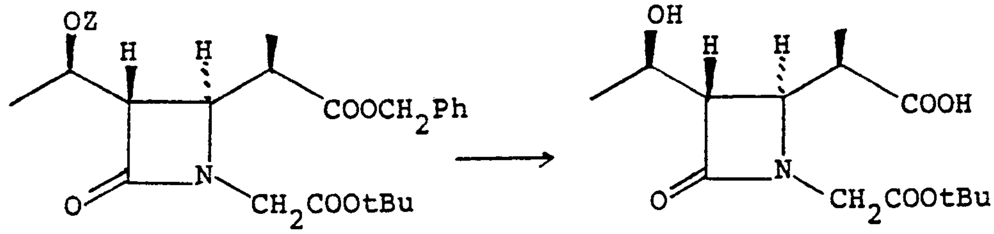

A solution of (3S,4S) - 3 - [(1R) - 1 - benzyloxycarbonyloxyethyl] - 4 - [(1R) - 1 - benzyloxycarbonylethyl] - 1 - (t - butyloxycarbonylmethyl)azetidin - 2 - one (81.50 g) in ethanol (800 ml) was subjected to hydrogenation at room temperature in the presence of 10% palladium-carbon (8.15 g) under atmospheric pressure, followed by filtration to remove the catalyst. The filtrate and the washings were combined and evaporated to give (3S,4S) - 3 - [(1R) - 1 - hydroxyethyl] - 4 - [(1R) - 1 - carboxyethyl] - 1 - (t - butyloxycarbonylmethyl)azetidin - 2 - one.

IR $\nu_{max}^{neat}$ ($cm^{-1}$): 1740, 1720, 1440, 1360.

NMR δ ($CDCl_3$): 1.28 (3H, d, J=6.9 Hz), 1.33 (3H, d, J=6.6 Hz), 2.84 (1H, m), 3.09 (1H, dd, J=2.0 and 6.6 Hz), 3.76 (1H, d, J=18 Hz), 4.03 (1H, dd, J=2.0 and 5.3 Hz).

Example 2-3

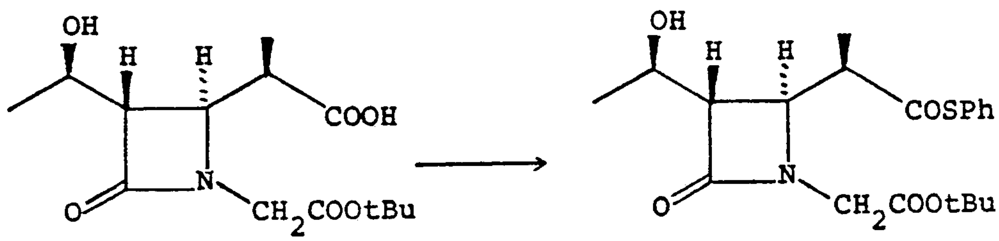

In the same manner as in Example 1-3(1), there was obtained (3S,4S) - 3 - [(1R) - 1 - hydroxyethyl] - 4 - [(1R) - 1 - phenylthiocarbonylethyl] - 1 - (t - butyloxycarbonylmethyl)azetidin - 2 - one from (3S,4S) - 3 - [(1R) - 1 - hydroxyethyl] - 4 - [(1R) - 1 - carboxyethyl] - 1 - (t - butyloxycarbonylmethyl-azetidin - 2 - one.

IR $\nu_{max}^{nujol}$ ($cm^{-1}$): 1745, 1725, 1290, 1230, 1140, 950, 750.

Example 3-1

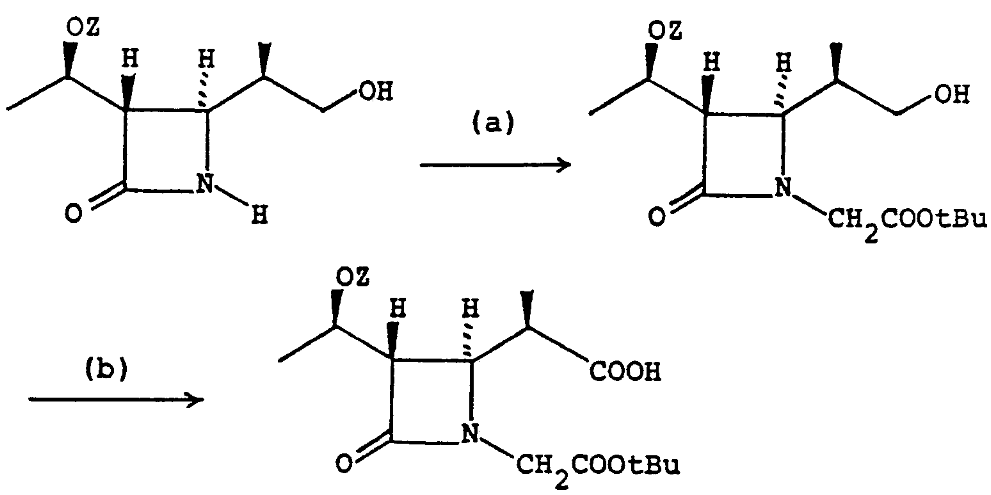


(a) To a solution of (3S,4R) - 3 - [(1R) - 1 - benzyloxycarbonyloxyethyl] - 4 - [(1R) - 1 - hydroxymethylethyl] - 2 - azetidinone (30.7 g) in acetone (300 ml), there were added t-butyl bromoacetate (33.0 g) and potassium carbonate (27.6 g), followed by stirring under reflux for 17 hours. The reaction mixture was

cooled down to room temperature and filtered to remove insoluble materials. The filtrate contained (3S,4R) - 3 - [(1R) - 1 - benzyloxycarbonyloxyethyl] - 4 - [(1R) - 1 - hydroxymethylethyl] - 1 - t - butoxycarbonylmethyl - 2 - azetidinone.

IR $\nu_{max}^{neat}$ ($cm^{-1}$): 1735, 1360, 1250, 1150, 1030, 955).

(b) The filtrate was diluted with water (15 ml) and treated with the Jones reagent, which was prepared from chromium trioxide (16.92 g), 98% sulfuric acid (26.52 g) and water (49.2 g), while ice-cooling for 1 hour. The reaction mixture was quenched with isopropanol and diluted with ethyl acetate (1 liter) and water (300 ml). The organic layer was washed with brine (300 ml × 4), dried over magnesium sulfate (50 g) and evaporated in vacuo to give an oily residue, which was purified by silica gel chromatography to give (3S,4S) - 3 - [(1R) - 1 - benzyloxycarbonyloxyethyl] - 4 - [(1R) - 1 - carboxyethyl] - 1 - t - butoxycarbonylmethyl - 2 - azetidinone (23.73 g, 54.5%).

IR $\nu_{max}^{neat}$ ($cm^{-1}$): 1735, 1450, 1365, 1250, 1150, 1040.

NMR δ ($CDCl_3$): 1.19 (3H, d, J=6.9 Hz), 1.46 (9H, s), 3.26 (1H, dd, J=2.3 and 8.6 Hz), 4.06 (1H, dd, J=2.3 and 4.0 Hz), 7.36 (5H, s).

Example 3-2

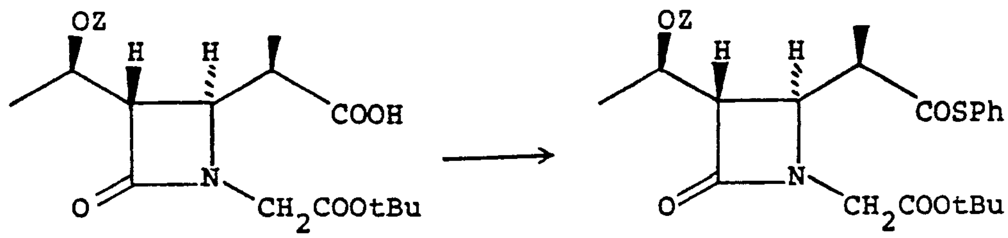

To a solution of (3S,4S) - 3 - [(1R) - 1 - benzyloxycarbonyloxyethyl] - 4 - [(1R) - 1 - carboxyethyl] - 1 - t - butoxycarbonylmethyl - 2 - azetidinone (23.75 g) in dry acetonitrile (237 ml), there was added N,N'-carbonyldiimidazole (10.55 g) under ice-cooling, followed by stirring for 0.5 hour. Thiophenol (7.21 g) and triethylamine (6.62 g) were then added thereto under ice-cooling, followed by stirring for 2.5 hours. The reaction mixture was diluted with ethyl acetate (500 ml) and washed with 1N hydrochloric acid (200 ml). The aqueous layer was separated from the organic layer and extracted with ethyl acetate (200 ml × 2). The extract was combined with the organic layer, washed with brine (300 ml × 3), dried over magnesium sulfate and evaporated in vacuo to give an oily residue, which was purified by silica gel chromatography to obtain (3S,4S) - 3 - [(1R) - 1 - benzyloxycarbonylethyl] - 4 - [(1R) - 1 - phenylthiocarbonylethyl] - 1 - t - butoxycarbonylmethyl - 2 - azetidinone (19.46 g, 67.64%).

IR $\nu_{max}^{neat}$ ($cm^{-1}$): 1760, 1735, 1700 1440, 1365, 1255, 1150, 1045, 950, 740.

NMR δ ($CDCl_3$): 1.25 (3H, d, J=6.9 Hz), 1.44 (9H, s), 1.47 (3H, d, J=6.2 Hz), 3.12 (1H, m), 4.13 (1H, dd, J=2.6 and 4.5 Hz), 7.36 (10H, m).

Example 4-1

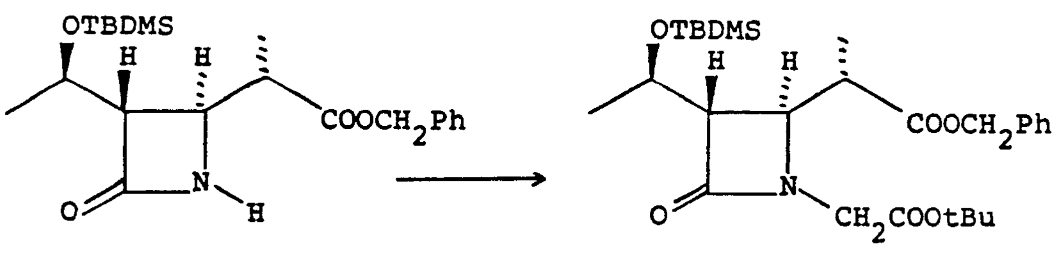

In the same manner as in Example 1-1, a solution of (3S,4S) - 3 - [(1R) - 1 - t - butyldimethylsilyloxyethyl] - 4 - [(1S) - 1 - benzyloxycarbonylethyl]azetidin - 2 - one (3.50 g) in methylene chloride (45 ml) was treated with t-butyl alpha-bromoacetate (8.73 g), 50% aqueous sodium hydroxide solution (2.86 g) and triethylbenzylammonium chloride (1.02 g) to give (3S,4S) - 3 - [(1R) - 1 - t - butyldimethylsilyloxyethyl] - 4 - [(1S) - 1 - benzyloxycarbonylethyl] - 1 - (t-butyloxymethyl)azetidine - 2 - one.

IR $\nu_{max}^{neat}$ ($cm^{-1}$): 1760 (sh), 1735, 1455, 1362, 1245, 1222, 835, 773.

Example 4-2

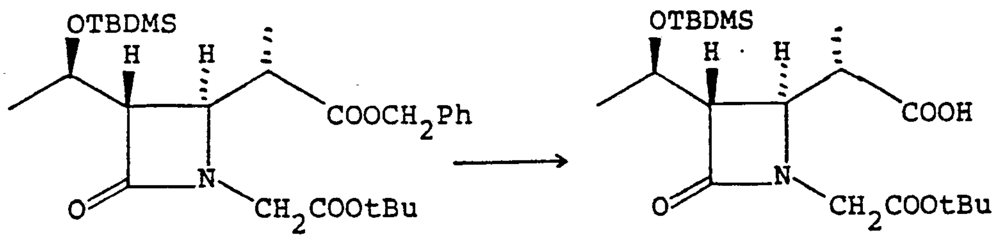

In the same manner as in Example 1-2, (3S,4S) - 3 - [(1R) - 1 - t - butyldimethylsilyloxyethyl] - 4 - [(1S) - 1 - benzyloxycarbonylethyl] - 2 - (t - butyloxycarbonylmethyl)azetidin - 2 - one was subjected to hydrogenation to give (3S,4S) - 3 - [(1R) - 1 - t - butyldimethylsilyloxyethyl] - 4 - [(1S) - 1 - carboxyethyl] - 1 - (t - butyloxycarbonylmethyl)azetidin - 2 - one.

IR $\nu_{max}^{nujol}$ ($cm^{-1}$): 3300 (broad), 1760 (sh), 1742, 1690, 990, 940, 825, 767.

Example 4-3(1)

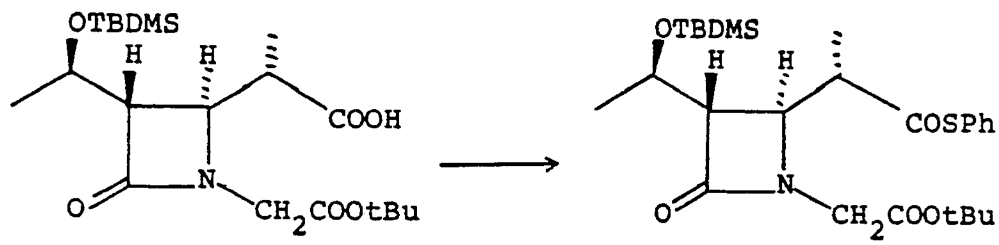

In the same manner as in Example 1-3(1) but replacing the starting material by (3S,4S) - 3 - [(1R) - 1 - t - butyldimethylsilyloxyethyl] - 4 - [(1S) - 1 - carboxyethyl] - 1 - t - butoxycarbonylmethyl - 2 - azetidinone, there was obtained (3S,4S) - 3 - [(1R) - 1 - t - butyldimethylsilyloxyethyl] - 4 - [(1S) - 1 - phenylthiocarbonylethyl] - 1 - t - butoxycarbonylmethyl - 2 - azetidinone.

IR $\nu_{max}^{neat}$ ($cm^{-1}$): 1760, 1740, (sh), 1700, 1365, 1250, 1225, 950, 830, 773, 740, 680.

Example 4-3(2)

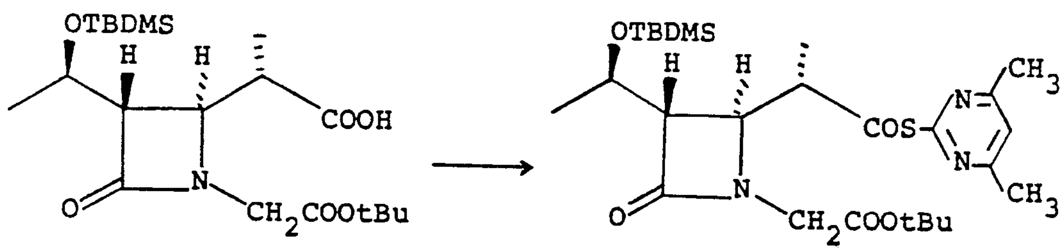

To a solution of (3S,4S) - 3 - [(1R) - 1 - t - butyldimethylsilyloxyethyl] - 4 - [(1S) - 1 - carboxyethyl] - 1 - t - butyloxycarbonylmethylazetidin - 2 - one (100 mg) in dry methylene chloride (  ml), there was added oxalyl chloride (37 mg), and the resultant mixture was stirred at room temperature for 2 hours in the presence of a catalytic amount of dimethylformammide. 4,6 - Dimethyl - 2 - mercaptopyrimidine (50 mg) and 4 - dimethylaminopyridine (44 mg) were added thereto, followed by stirring. The reaction mixture was diluted with methylene chloride, washed with dilute sulfuric acid, water and sodium bicarbonate and brine successively, dried over sodium sulfate and distilled off to remove the solvent. The residue was purified by silica gel chromatography to give (3S,4S) - 3 - [(1R) - 1 - t - butyldimethylsilyloxyethyl] - 4 - [(1S) - 1 - (4,6-dimethylpyrimidin) - 2 - ylthiocarbonyethyl] - 1 - t - butyloxycarbonylmethyl)azetidin - 2 - one.

IR $\nu_{max}^{neat}$ ($cm^{-1}$): 1760, 1740 (sh), 1580, 1362, 1247, 830, 770.

Example 4-3(3)

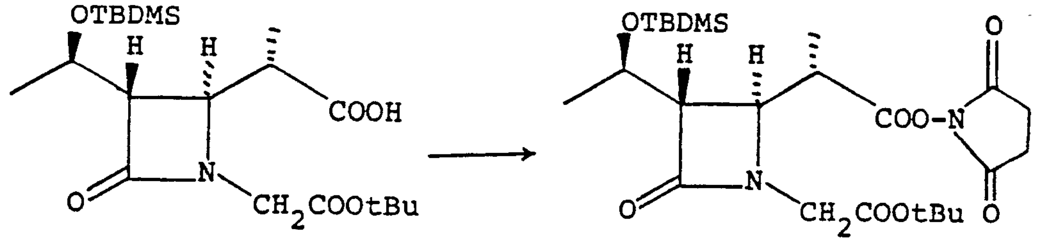

To a solution of (3S,4S) - 3 - [(1R) - 1 - t - butyldimethylsilyloxyethyl] - 4 - [(1S) - 1 - carboxyethyl] - 1 - (t - butyloxycarbonylmethyl)azetidin - 2 - one (100 mg) and N-hydroxysuccinimide (33 mg) in dry dimethylformamide (0.3 ml), there was added N,N'-dicyclohexylcarbodiimide (74 mg), followed by stirring at 0 to 5°C overnight. The reaction mixture was diluted with ethyl acetate, followed by addition of sulfuric acid. The aqueous layer was separated from the organic layer and extracted with ethyl acetate two times. The extracts were combined with the organic layer, washed with water four times and brine, dried over sodium sulfate and distilled off to remove the solvent. The residue was purified by silica gel chromatography to give (3S,4S) - 3 - [(1R) - 1 - t - butyldimethylsilyloxyethyl] - 4 - [(1S) - 1 - succinimidoxycarbonylethyl] - 1 - (t - butyloxycarbonylmethyl)azetidin - 2 - one.

IR $\nu_{max}^{neat}$ ($cm^{-1}$): 1805, 1760 (sh), 1740, 1455, 1360, 1200, 1145, 1055, 830, 762, 745.

Example 4-3(4)

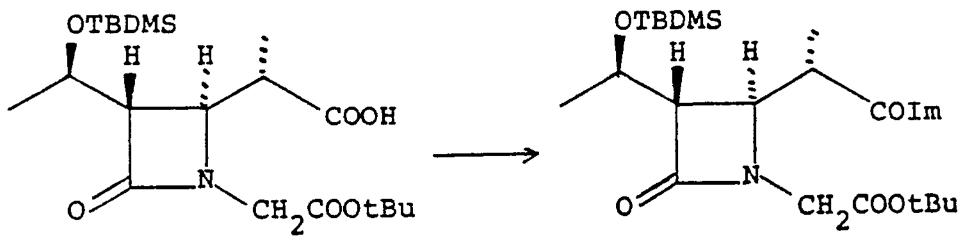

A solution of (3S,4S) - 3 - [(1R) - 1 - t - butyldimethylsilyloxyethyl] - 4 - [(1S) - 1 - carboxyethyl] - 1 - (t - butyloxycarbonylmethyl)azetidin - 2 - one (42 mg) and N,N'-carbonyldiimidazole (19 mg) in dry acetonitrile (0.9 ml) was stirred at room temperature for 1 hour and distilled off to remove the solvent. The residue was purified by silica gel chromatography to give (3S,4S) - 3 - [(1R) - 1 - t - butyldimethylsilyloxyethyl] - 4 - [(1S) - 1 - (1 - imidazolyl)carbonylethyl] - 1 - (t - butyloxycarbonylmethyl)azetidin - 2 - one.

IR $\nu_{max}^{neat}$ ($cm^{-1}$): 1760 (sh), 1735, 1382, 1360, 1225, 1145, 935, 827, 745.

Example 5-1

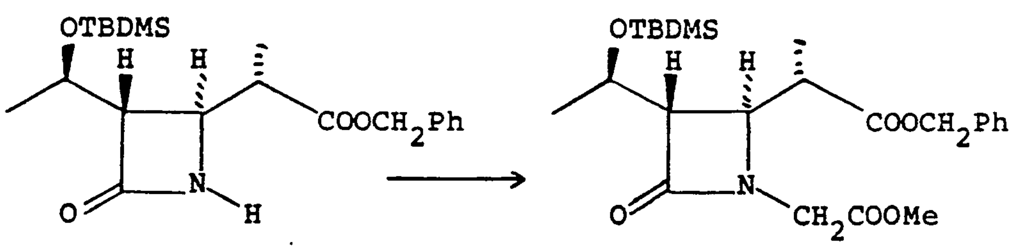

To a solution of (3S,4S) - 3 - [(1R) - 1 - t - butyldimethylsilyloxyethyl] - 4 - [(1S) - 1 - benzyloxycarbonylethyl]azetidin - 2 - one (1.56 g) in methylene chloride (20 ml), there were added successively methyl alpha-bromoacetate (916 mg), 50% aqueous sodium hydroxide solution (1.28 g) and triethylbenzylammonium chloride (455 mg), followed by stirring at room temperature for 2 hours. The reaction mixture was diluted with water. The aqueous layer was separated from the organic layer and extracted two times with diethyl ether. The extracts were combined with the organic layer, washed successively with water two times and brine three times, dried over sodium sulfate and evaporated. The residue was purified by silica gel chromatography to give (3S,4S) - 3 - [(1R) - 1 - t - butyldimethylsilyloxyethyl] - 4 - [(1S) - 1 -

benzyloxycarbonylethyl] - 1 - (methoxycarbonylmethyl) - azetidin - 2 - one.

IR $\nu_{max}^{neat}$ ($cm^{-1}$): 1760, 1740, 1460, 1407, 1360, 1250, 1215, 1180, 1140, 835, 770, 745.

NMR δ ($CDCl_3$): 0.07 (3H, s), 0.08 (3H, s), 0.87 (9H, s), 1.24 (3H, d, J=6.3 Hz), 1.25 (3H, d, J=7.2 Hz), 2.77 (1H, m), 3.63 (3H, s), 3.93 (2H, s), 3.96 (1H, dd, J=2.0 and 9.6 Hz), 4.19 (1H, m), 5.09 (2H, s), 7.36 (5H, s).

Example 5-2

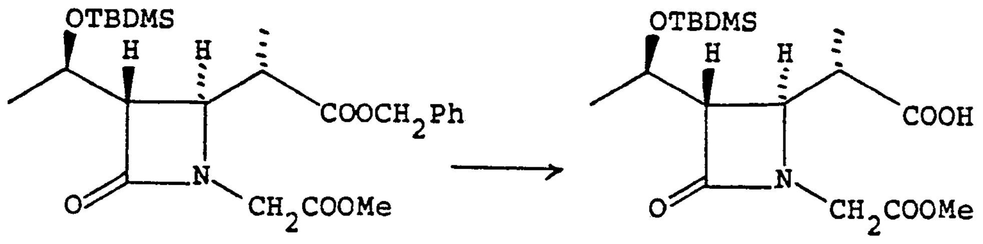

A solution of (3S,4S) - 3 - [(1R) - 1 - t - butyldimethylsilyloxyethyl] - 4 - [(1S) - 1 - benzyloxycarbonylethyl] - 1 - (methoxycarbonylmethyl) - azetidin - 2 - one (400 mg) in 99.5% ethanol (6 ml) was subjected to hydrogenation at room temperature in the presence of 10% palladium-carbon (80 mg) under atmospheric pressure, followed by filtration to remove the catalyst. The filtrate was evaporated to give (3S,4S) - 3 - [(1R) - 1 - t - butyldimethylsilyloxyethyl] - 4 - [(1S) - 1 - carboxyethyl] - 1 - (methoxycarbonylmethyl) - azetidin - 2 - one.

IR $\nu_{max}^{neat}$ ($cm^{-1}$): 1740, 1705, 1435, 1240, 1215, 1135, 830, 770.

Example 5-3

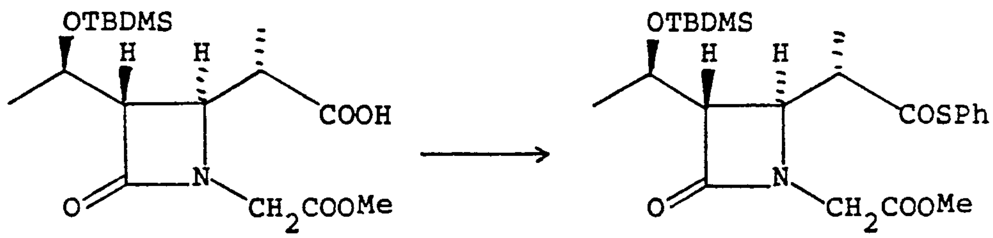

In the same manner as in Example 1-3(1) but replacing the starting material by (3S,4S) - 3 - [(1R) - 1 - t - butyldimethylsilyloxyethyl] - 4 - [(1S) - 1 - carboxyethyl] - 1 - (methoxycarbonylmethyl) - 2 - azetidinone, there was obtained (3S,4S) - 3 - [(1R) - 1 - t - butyldimethylsilyloxyethyl] - 4 - [(1S) - 1 - phenylthiocarbonylethyl] - 1 - methoxycarbonylmethyl - 2 - azetidinone.

IR $\nu_{max}^{neat}$ ($cm^{-1}$): 1750, 1695, 1437, 1405, 1247, 1202, 950, 830, 770, 740.

NMR δ ($CDCl_3$): 0.07 (3H, s), 0.09 (3H, s), 0.87 (9H, s), 2.88 (1H, dd, J=2.3 and 6.6 Hz), 3.03 (1H, m), 3.70 (3H, s), 4.02 (1H, dd, J=2.0 and 9.2 Hz), 4.19 (1H, m), 7.41 (5H, m).

Example 6-1

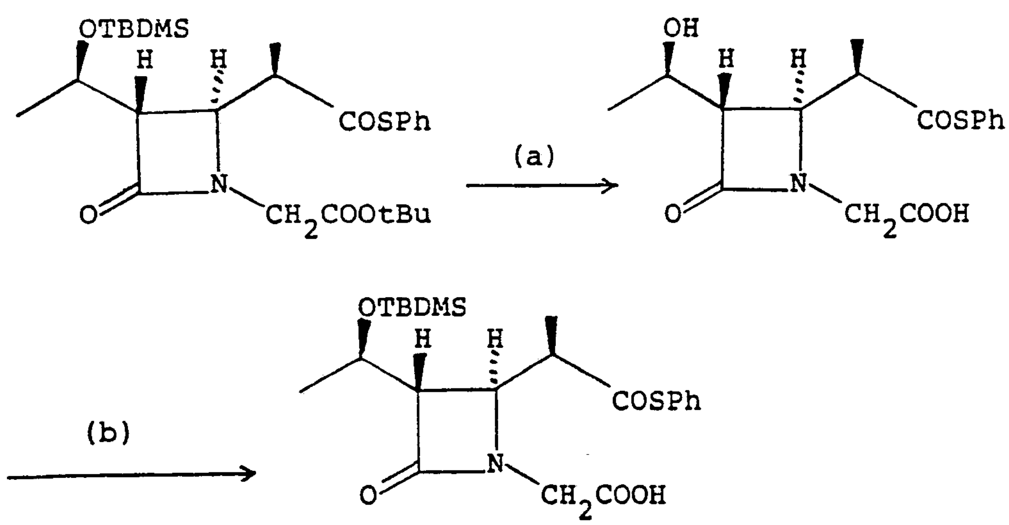


(a) To a solution of (3S,4S) - 3 - [(1R) - 1 - t - butyldimethylsilyloxyethyl] - 4 - [(1R) - 1 - phenylthiocarbonylethyl] - 1 - (t - butyloxycarbonylmethyl)azetidin - 2 - one (190 mg) in methanol (4.5 ml) was added 6N hydrochloric acid (1.5 ml), followed by stirring at room temperature for 15 minutes. The reaction mixture was diluted with chloroform and brine. The aqueous layer was separated from the organic layer and extracted with chloroform two times. The extracts were combined with the organic layer, dried over sodium sulfate and evaporated. The residue was dissolved in trifluoroacetic acid (1.0 ml) and anisole (0.1 ml). After stirring at room temperature for 25 minutes, the solvent was evaporated and removed azeotropically with dry toluene two times to give (3S,4S) - 3 - [(1R) - 1 - hydroxyethyl] - 4 - [(1R) - 1 - phenylthiocarbonylethyl] - 1 - (carboxymethyl)azetidin - 2 - one as a crude product.

(b) A mixture of the crude product as obtained above, t-butyldimethylchlorosilane (246 mg) and imidazole (151 mg) in dry dimethylformamide (2 ml) was stirred overnight. The resulting mixture was poured into water and extracted with ethyl acetate three times. The organic layer was washed successively with dilute sulfuric acid, water (five times) and brine (two times), dried over sodium sulfate and evaporated. The residue was purified by silica gel chromatography to give (3S,4S) - 3 - [(1R) - 1 - t - buthyldimethylsilyloxyethyl] - 4 - [(1R) - 1 - phenylthiocarbonylethyl] - 1 - (carboxymethyl)azetidin - 2 - one.

IR $\nu_{max}^{neat}$ ($cm^{-1}$): 3350 (broad), 1760 (sh), 1737, 1700, 1245, 1140, 830, 767, 742.

Example 6-2

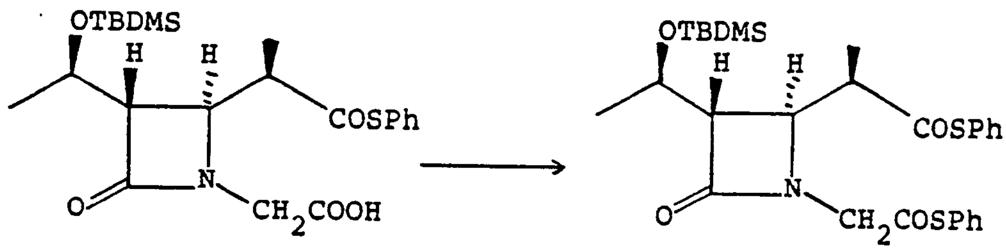

A mixture of (3S,4S) - 3 - [(1R) - 1 - t - butyldimethylsilyloxyethyl] - 4 - [(1R) - 1 - phenylthiocarbonylethyl] - 1 - (carboxymethyl)azetidin - 2 - one (154 mg) and N,N'-carbonyldiimidazole (66 mg) in dry acetonitrile (2.9 ml) was stirred at room temperature for 1 hour. Thiophenol (56 mg) in dry acetonitrile (1 ml) and triethylamine (52 mg) in dry acetonitrile (0.5 ml) were added thereto, followed by stirring at room temperature for 30 minutes. The reaction mixture was diluted with ethyl acetate, poured into dilute sulfuric acid and extracted with ethyl acetate three times. The organic layer was washed successively with dilute sulfuric acid and brine (two times), dired over sodium sulfate and evaporated. The residue was purified by silica gel chromatography to give (3S,4S) - 3 - [(1R) - 1 - t - butyldimethylsilyloxyethyl] - 4 - [(1R) - 1 - phenylthiocarbonylethyl] - 1 - (phenylthiocarbonylmethyl)azetidin - 2 - one.

IR $\nu_{max}^{neat}$ ($cm^{-1}$): 1760, 1700, 1478, 1440, 1250, 1140, 835, 773, 740, 682.

NMR δ ($CDCl_3$): 0.08 (3H, s), 0.10 (3H, s), 0.89 (9H, s), 1.27 (3H, d, J=6.3 Hz), 1.32 (3H, d, J—7.3 Hz), 3.11 (1H, dd, J—2.0 and 6.9 Hz), 3.20 (1H, m), 4.24 (1H, m), 4.30 (2H, m), 7.41 (10H, s).

Example 7-1

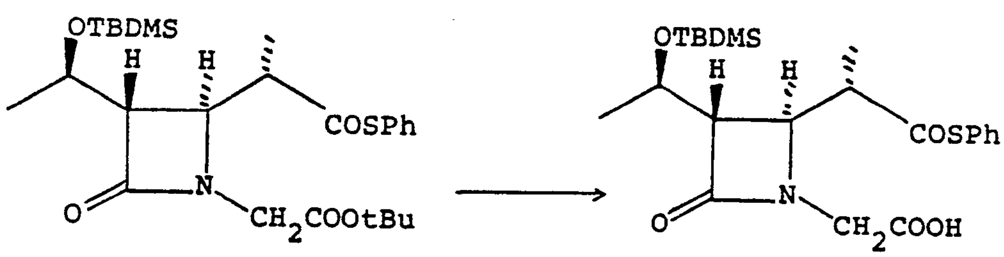

In the same manner as in Example 6-1, there was obtained (3S,4S) - 3 - [(1R) - 1 - t - butyldimethylsilyloxyethyl] - 4 - [(1S) - 1 - phenylthiocarbonylethyl] - 1 - (carboxymethyl)azetidin - 2 - one from (3S,4S) - 3 - [(1R) - 1 - t - butyldimethylsilyloxyethyl] - 4 - [(1S) - 1 - phenylthiocarbonylethyl] - 1 - (t-butyloxycarbonymethyl)azetidin - 2 - one.

IR $\nu_{max}^{neat}$ ($cm^{-1}$): 3350 (broad), 1760 (sh), 1740, 1250, 940, 825, 770, 740, 680.

Example 7-2

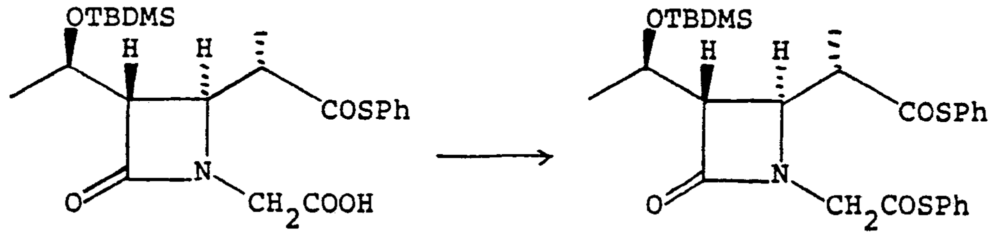

In the same manner as in Example 6-2 but replacing (3S,4S) - 3 - [(1R) - 1 - t - butyldimethylsilyloxyethyl] - 4 - [(1R) - 1 - phenylthiocarbonylethyl] - 1 - (carboxymethyl)azetidin - 2 - one by (3S,4S) - 3 - [(1R) - 1 - t - butyldimethylsilyloxyethyl] - 4 - [(1S) - 1 - phenylthiocarbonylethyl] - 1 - (carboxymethyl)-azetidin - 2 - one, there was obtained (3S,4S) - 3 - [(1R) - 1 - t - butyldimethylsilyloxyethyl] - 4 - [(1S) - 1 - phenylthiocarbonylethyl] - 1 - (phenylthiocarbonylmethyl)azetidin - 2 - one.

IR $\nu_{max}^{neat}$ ($cm^{-1}$): 1760, 1705, 1480, 1442, 1250, 955, 835, 742, 682.

Example 8-1

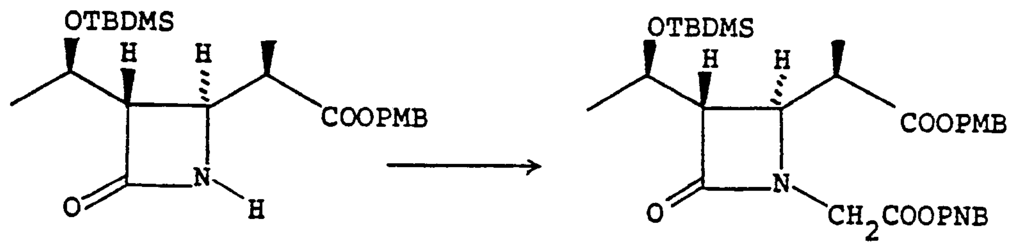

To a solution of (3S,4S) - 3 - [(1R) - 1 - t - butyldimethylsilyloxyethyl] - 4 - [(1R) - 1 - p - methoxybenzyloxycarbonylethyl]azetidin - 2 - one (1.12 g) in methylene chloride (14 ml), there were successively added p-nitrobenzyl alpha-bromoacetate (1.09 g), 50% aqueous sodium hydroxide solution (0.85 g) and triethylbenzylammonium chloride (303 mg), followed by stirring at room temperature for 30 minutes. The reaction mixture was diluted with water and extracted with a mixture of diethyl ether and methylene chloride (3:1) three times. The organic layer was washed successively with water two times and brine three times, dried over sodium sulfate and evaporated. The residue was purified by silica gel chromatography to give (3S,4S) - 3 - [(1R) - 1 - t - butyldimethylsilyloxyethyl] - 4 - [(1R) - 1 - p - methoxybenzyloxycarbonylethyl] - 1 - (p - nitrobenzyloxycarbonylmethyl)azetidin - 2 - one.

IR $\nu_{max}^{neat}$ ($cm^{-1}$): 1760, 1742, 1607, 1515, 1458, 1342, 1241, 1170, 830, 747.

NMR δ ($CDCl_3$): 0.01 (3H, s), 0.05 (3H, s), 0.83 (9H, s), 2.86 (1H, qd, J=7.2 and 3.0 Hz), 3.00 (1H, dd, J=2.3 and 6.6 Hz), 3.80 (3H, s), 5.01 (2H, m), 5.20 (2H, s), 6.88 (2H, d, J=8.6 Hz), 7.49 (2H, d, J=8.9 Hz), 8.22 (2H, d, J=8.6 Hz).

Example 8-2

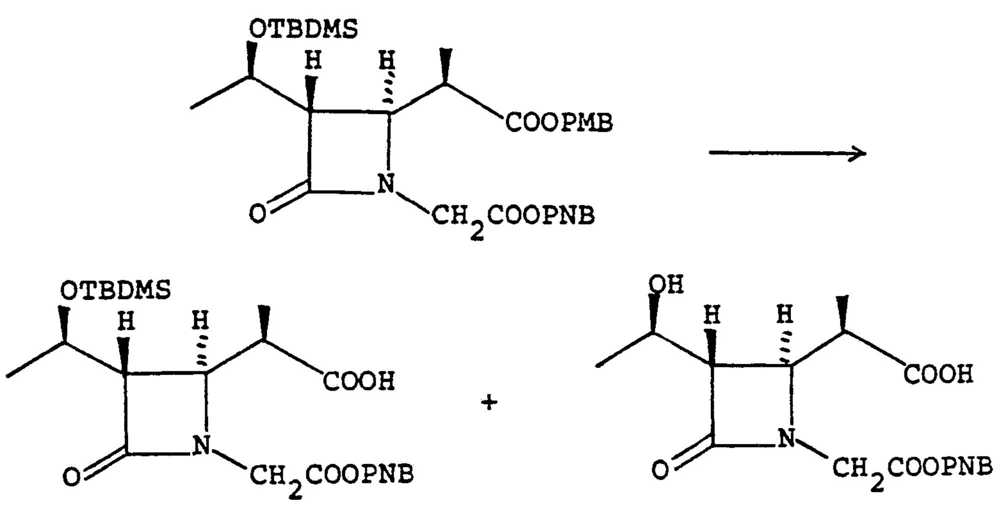

To a solution of (3S,4S) - 3 - [(1R) - 1 - t - butyldimethylsilyloxyethyl] - 4 - [(1R) - 1 - p -

methoxybenzyloxycarbonylethyl] - 1 - (p - nitrobenzyloxycarbonylmethyl)azetidin - 2 - one (142 mg) in dry methylene chloride, there was $BF_3$-$Et_2O$ complex (163 mg) under ice-cooling, followed by stirring at room temperature. The reaction mixture was poured into a cold aqueous sodium bicarbonate solution, acidified with dilute hydrochloric acid and extracted with ethyl acetate three times. The organic layer was washed successively with dilute hydrochloric acid and brine, dried over sodium sulfate and evaporated. The residue was purified by silica gel chromatography to give (3S,4S) - 3 - [(1R) - 1 - t - butyldimethylsilyloxyethyl] - 4 - [(1R) - 1 - carboxyethyl] - 1 - (p - nitrobenzyloxycarbonylmethyl)azetidin - 2 - one (Compound A) and (3S,4S) - 3 - [(1R) - 1 - hydroxyethyl] - [(1R) - 1 - carboxyethyl] - 1 - (p - nitrobenzyloxycarbonylmethyl)azetidin - 2 - one (Compound B).

Compound A:—
IR $\nu_{max}^{neat}$ ($cm^{-1}$): 3100 (broad), 1760, 1730, 1520, 1342, 1245, 1180, 830, 770.
NMR δ ($CDCl_3$): 0.03 (3H, s), 0.07 (3H, s), 0.85 (9H, s), 1.25 (3H, d, J=6.3 Hz), 1.26 (3H, d, J=7.3 Hz), 2.91 (1H, qd, J=3.0 and 7.3 Hz), 3.05 (1H, dd, J=2.3 and 5.9 Hz), 4.13 (2H, m), 5.26 (2H, s), 7.52 (2H, d, J=8.9 Hz), 8.23 (2H, d, J=8.9 Hz).

Compound B:—
IR $\nu_{max}^{neat}$ ($cm^{-1}$): 3430 (broad), 1760, 1735, 1705, 1520, 1345, 1180, 745.

Example 9-1

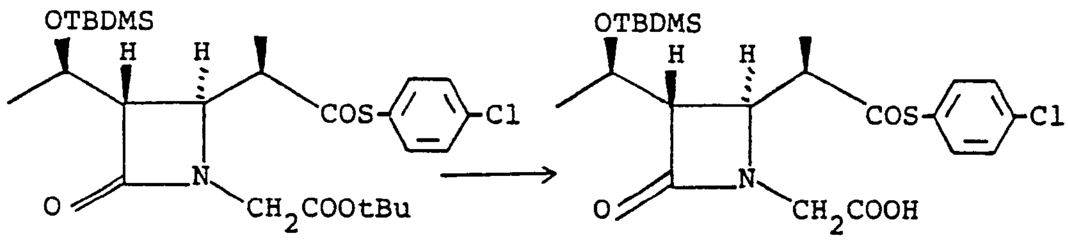

To a solution of (3S,4S) - 3 - [(1R) - 1 - t - butyldimethylsilyloxyethyl] - 4 - [(1R) - 1 - p - chlorophenylthiocarbonylethyl] - 1 - (t - butyloxycarbonylmethyl)azetidin - 2 - one (200 mg) in dry methylene chloride (1.5 ml), there was added $BF_3$—$ET_2O$ complex (263 mg), followed by stirring at room temperature for 1 hour. After evaporation of the solvent, the residue was dissolved in methanol (0.5 ml), diluted with brine and extracted with ethyl acetate. The organic layer was washed with brine, dried over sodium sulfate and evaporated to give (3S,4S) - 3 - [(1R) - 1 - hydroxyethyl] - 4 - [(1R) - 1 - p - chlorophenylthiocarbonylethyl] - 1 - (carboxymethyl)azetidin - 2 - one as a crude product. The crude product, t-butyldimethylsilyl chloride (246 mg) and imidazole (151 mg) were dissolved in dry dimethylformamide (2.5 ml) and allowed to stand at room temperature overnight. The reaction mixture was poured into cold brine, adjusted with 1M potassium hydrogensulfate to pH 2 and extracted with diethyl ether three times. The organic layer was washed with brine two times, dried over sodium sulfate and evaporated. The residue was purified by silica gel chromatography to give (3S,4S) - 3 - [(1R) - 1 - t - butyldimethylsilyloxyethyl] - 4 - [(1R) - 1 - p - chlorophenylthiocarbonylethyl] - 1 - (carboxymethyl)-azetidin - 2 - one.
IR $\nu_{max}^{neat}$ ($cm^{-1}$): 3300 (broad), 1760, 1740, 1700, 1480, 1382, 1250, 1140, 1087, 830, 775.

Example 9-2

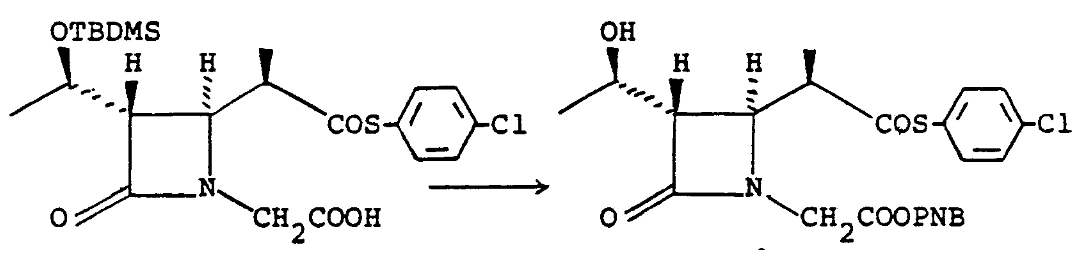

To a mixture of (3S,4S) - 3 - [(1R) - 1 - t - butyldimethylsilyloxyethyl) - 4 - [(1R) - 1 - p - chlorophenylthiocarbonylethyl] - 1 - carboxymethylazetidin - 2 - one (70 mg) and p-nitrobenzyl alcohol (24 ml) in ethyl acetate (0.3 ml), there was added a solution of N,N'-dicyclohexylcarbodiimide (30 mg) in dry ethyl acetate (0.2 ml), and the resultant mixture was stirred at 5 to 10°C overnight. The precipitated N,N'-dicyclohexylurea was collected by filtration and washed with ethyl acetate. The washing was combined with the filtrate, washed with water and brine, dried over sodium sulfate and distilled off to remove the

solvent. The residue was purified by silica gel chromatography to give (3S,4S) - 3 - [(1R) - 1 - t - butyldimethylsilyloxyethyl] - 4 - [(1R) - 1 - p - chlorophenylthiocarbonylethyl] - 1 - (1 - nitrobenzyloxycarbonylmethyl)azetidin - 2 - one.

IR $\nu_{max}^{neat}$ ($cm^{-1}$): 1760, 1750, 1700, 1602, 1520, 1478, 1343, 1250, 1180, 1090, 835, 775, 742.

NMR δ ($CDCl_3$): 0.07 (3H, s), 0.08 (3H, s), 0.88 (9H, s), 1.27 (3H, d, J=6.3 Hz), 1.31 (3H, d, J=7.3 Hz), 3.01 (1H, dd, J=2.6 and 7.1 Hz), 3.14 (1H, qd, J=2.6 and 7.3 Hz), 4.12 (2H, m), 4.17 (2H, m), 5.20 (2H, m), 7.34 (4H, m), 7.44 (2H, d, J=8.6 Hz), 8.17 (2H, d, J=8.9 Hz).

Example 10-1

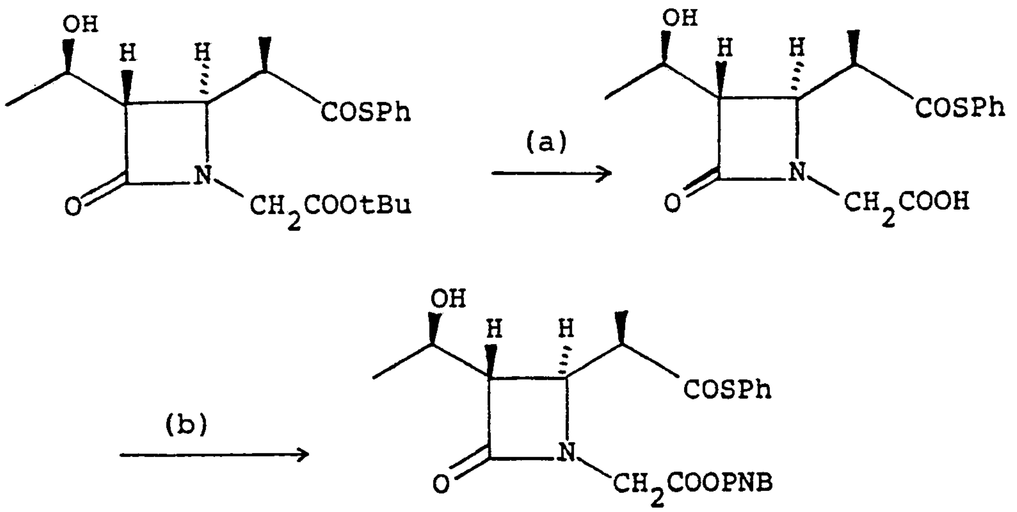


(a) (3S,4S) - 3 - [(1R) - 1 - Hydroxyethyl] - 4 - [(1R) - 1 - phenylthiocarbonylethyl] - 1 - (t - butyloxycarbonylmethyl)azetidin - 2 - one (72.0 g) was dissolved in trifluoroacetic acid (500 ml) under ice-cooling, followed by stirring for 2 hours. The reaction mixture was evaporated in vacuo below 50°C. The residue was then dissolved in toluene (250 ml) and evaporated off to remove the solvent.

(b) To a solution of the residue in dry acetonitrile (720 ml), there were added triethylamine (43.25 g) and p-nitrobenzyl bromide (92.33 g), followed by stirring at room temperature for 1 hour. The reaction mixture was diluted with ethyl acetate (1.5 liters), washed with a 20% aqueous sodium chloride solution several times, dried over sodium sulfate and evaporated. The residue was purified by silica gel chromatography to give (3S,4S) - 3 - [(1R) - 1 - hydroxyethyl] - 4 - [(1R) - 1 - phenylthiocarbonylethyl] - 1 - (p - nitrobenzyloxycarbonylmethyl)azetidin - 2 - one.

IR $\nu_{max}^{CHCl_3}$ ($cm^{-1}$): 1740, 1680, 1600, 1515, 1360, oxycarbonylmethyl)azetidin-2-one.

IR $\nu_{max}^{CHCl_3}$ ($cm^{-1}$): 1740, 1680, 1600, 1515, 1360, 1250, 1180, 950, 740.

Example 10-2

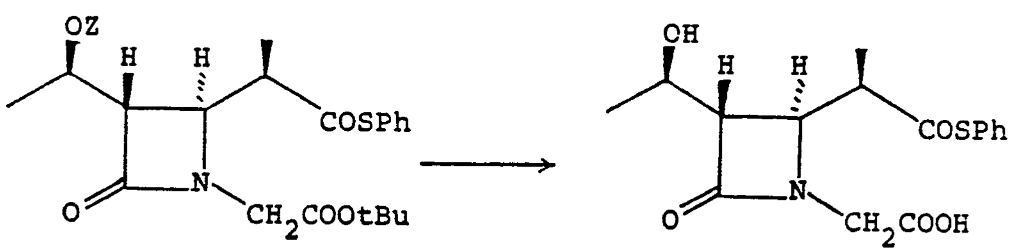

To a solution of (3S,4S) - 3 - [(1R) - 1 - benzyloxycarbonyloxyethyl] - 4 - [(1R) - 1 - phenylthiocarbonylethyl] - 1 - t - butoxycarbonylmethylazetidin - 2 - one (8.5 g) in 1,2-dichloroethane (185 ml), there was dropwise added to a solution of boron tribromide (26.4 g) in 1,2-dichloroethane (100 ml) at −10°C for 20 minutes, followed by stirring at the same temperature for 1 hour. Sodium bicarbonate (40 g) and ice-water (600 g) were added thereto while stirring, and the resultant mixture was diluted with ethyl acetate (200 ml). The aqueous layer was acidified with 2N hydrochloric acid (200 ml), extracted with ethyl acetate and combined with the organic layer. The combined organic layer was washed with brine (200 ml × 3), dried over magnesium sulfate and evaporated in vacuo to give (3S,4S) - 3 - [(1R) - 1 - hydroxyethyl] - 4 - [(1R) - 1 - phenylthiocarbonylethyl] - 1 - carboxymethylazetidin - 2 - one (10.4 g, 87.9%).

IR $\nu_{max}^{Nujol}$ ($cm^{-1}$): 1740, 1710, 1690, 1210, 1130, 1070, 940, 740.

Example 10-3

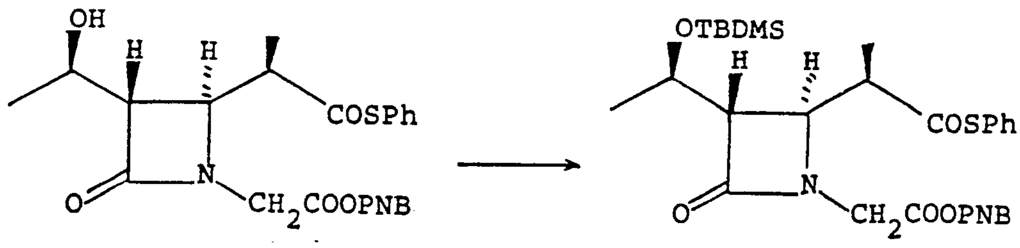

To a solution of (3S,4S) - 3 - [(1R) - 1 - hydroxyethyl] - 4 - [(1R) - 1 - phenylthiocarbonylethyl] - 1 - (p - nitrobenzyloxycarbonylmethyl]azetidin - 2 - one (52.36 g) in dry dimethylformamide (262 ml), there were added imidazole (16.6 g) and t-butyldimethylchlorosilane (23.38 g), followed by stirring at room temperature for 5 hours. The reaction mixture was diluted with ethyl acetate (1 liter), washed with a 20% aqueous sodium chloride solution. The aqueous layer was separated from the organic layer and extracted with ethyl acetate (500 ml). The extract was combined with the organic layer, washed with a 20% aqueous sodium chloride solution two times, dried over sodium sulfate and evaporated. The residue was purified by silica gel chromatography to give (3S,4S) - 3 - [(1R) - 1 - t - butyldimethylsilyloxyethyl] - 4 - [(1R) - 1 - phenylthiocarbonylethyl] - 1 - (p - nitrobenzyloxycarbonylmethyl]azetidin - 2 - one.

IR $\nu_{max}^{neat}$ ($cm^{-1}$): 1755, 1690, 1600, 1515, 1340, 1250, 1180, 835.

NMR δ ($CDCl_3$): 0.08 (3H, s), 0.09 (3H, s), 0.89 (9H, s), 1.28 (3H, d, J=6.0 Hz), 1.32 (3H, d, J=7.3 Hz), 3.01 (1H, dd, J=2.3 and 7.3 Hz), 3.16 (1H, dd, J=2.3 and 7.3 Hz), 3.96 (1H, d, J=17.8 Hz), 4.17 (2H, m), 4.31 (1H, d, J=17.8 Hz), 5.20 (2H, $AB_q$, J=13.5 Hz), 7.25—7.45 (5H), 8.12 (2H, d, J=8.9 Hz).

Example 10-4

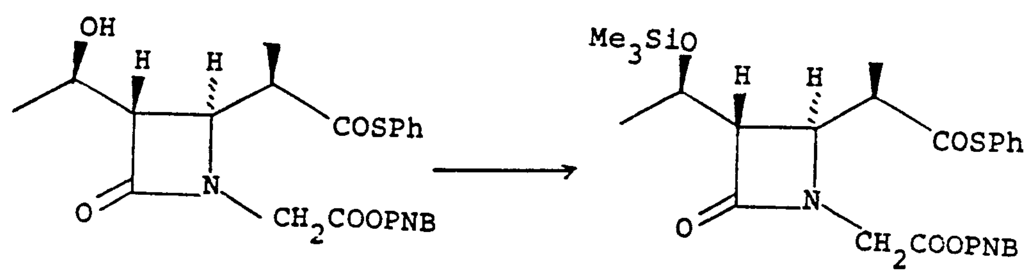

In the same manner as in Example 10-3, there was obtained (3S,4S) - 3 - [(1R) - 1 - trimethylsilyloxyethyl] - 4 - [(1R) - 1 - phenylthiocarbonylethyl] - 1 - (p - nitrobenzyloxycarbonylmethyl]azetidin - 2 - one from (3S,4S) - 3 - [(1R) - 1 - hydroxyethyl] - 4 - [(1R) - 1 - phenylthiocarbonylethyl] - 1 - (p - nitrobenzyloxycarbonylmethyl]azetidin - 2 - one.

IR $\nu_{max}^{neat}$ ($cm^{-1}$): 1760, 1695, 1600, 1520, 1440, 1340, 1250, 1180, 950, 840, 740.

NMR δ ($CDCl_3$): 0.13 (9H, s), 3.04 (1H, dd, J=2.3 and 7.6 Hz), 3.15 (1H, dq, J=2.3 and 7.0 Hz), 3.92 (1H, d, J=18.1 Hz), 4.38 (1H, d, J=18.1 Hz), 5.21 (2H, $AB_q$, J=13.5 Hz), 8.12 (2H, d, J=8.9 Hz).

Reference Example 1-1

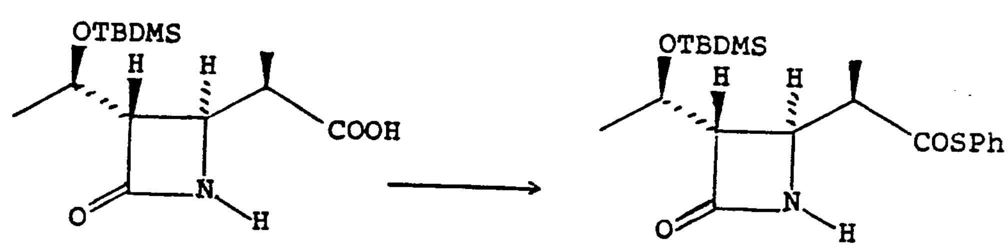

A mixture of (3S,4S) - 3 - [(1R) - 1 - t - butyldimethylsilyloxyethyl] - 4 - [(1R) - 1 - carboxyethyl]azetidin - 2 - one (301 mg) and N,N'-carbonyldiimidazole (194 mg) in dry acetonitrile (8.6 ml) was stirred at room temperature for 1 hour. To the reaction mixture, there were successively added thiophenol (132 mg) in dry acetonitrile (2 ml) and triethylamine (121 mg) in dry acetonitrile (2 ml), followed by stirring at room temperature for 30 minutes. The resulting mixture was diluted with ethyl acetate and washed with brine. The aqueous layer was separated from the organic layer and extracted with ethyl acetate two times. The extracts were combined with the organic layer, washed with brine, dried over sodium sulfate and

distilled off to remove the solvent. The residue was purified by silica gel chromatography to give (3S,4S) - 3 - [(1R) - 1 - t - butyldimethylsilyloxyethyl] - 4 - [(1R) - phenylthiocarbonylethyl]azetidin - 2 - one.

IR $\nu_{max}^{neat}$ ($cm^{-1}$): 3200 (broad), 1760, 1700, 1370, 1250, 1140, 955, 830, 773, 740, 680.

Reference Example 1-2

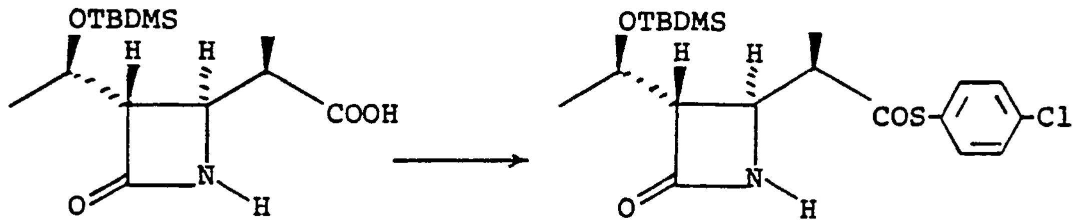

A mixture of (3S,4S) - 3 - [(1R) - 1 - butyldimethylsilyloxyethyl] - 4 - [(1R) - 1 - carboxyethyl]azetidin - 2 - one (400 mg) and N,N'-carbonyldiimidazole (259 mg) in dry acetonitrile (11 ml) was stirred at room temperature for 1 hour. To the resulting mixture, there were successively added p-chlorothiophenol (231 mg) in dry acetonitrile (3.2 ml) and triethylamine (162 mg) in dry acetonitrile (2.3 ml), followed by stirring at room temperature for 30 minutes. The reaction mixture was diluted with ethyl acetate and washed with brine. The aqueous layer was separated from the organic layer and extracted with ethyl acetate two times. The extracts were combined with the organic layer, washed with dilute hydrochloric acid, brine, aqueous sodium bicarbonate solution and brine, dried over sodium sulfate and distilled off to remove the solvent. The residue was purified by silica gel chromatography to give (3S,4S) - 3 - [(1R) - 1 - t - butyldimethylsilyloxyethyl] - 4 - [(1R) - 1 - p - chlorophenylthiocarbonylethyl]azetidin - 2 - one.

IR $\nu_{max}^{neat}$ ($cm^{-1}$): 3250 (broad), 1770, 1750, 1700, 1478, 1247, 1140, 1090, 820, 770.

NMR δ ($CDCl_3$): 0.07 (6H, s), 0.88 (9H, s), 1.18 (3H, d, J=6.3 Hz), 1.33 (3H, d, J=6.9 Hz), 2.97 (1H, m), 3.02 (1H, m), 3.93 (1H, dd, J=2.0 and 5.3 Hz), 4.22 (1H, m), 5.86 (1H, broad, s), 7.36 (4H, m).

Reference Example 1-3

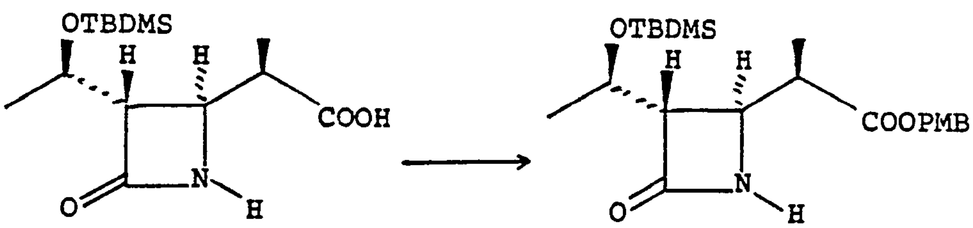

A mixture of (3S,4S) - 3 - [(1R) - 1 - t - butyldimethylsilyloxyethyl] - 4 - [(1R) - 1 - carboxylethyl]-azetidin - 2 - one (1.00 g), triethylamine (369 mg) and p-methoxybenzyl chloride (779 mg) in dry dimethylformamide (1 ml) was stirred at 70°C for 2 hours and 40 minutes. The reaction mixture was poured into ice-water, acidified with dilute hydrochloric acid to pH 2 to 3 and extracted with diethyl ether three times. The organic layer was washed with cold 1N aqueous sodium hydroxide solution, water and brine in this order, dried over sodium sulfate and distilled off to remove the solvent. The residue was purified by silica gel chromatography to give (3S,4S) - 3 - [(1R) - 1 - t - butyldimethylsilyloxyethyl] - 4 - [(1R) - 1 - p - methoxybenzyloxycarbonylethyl]azetidin - 2 - one.

IR $\nu_{max}^{neat}$ ($cm^{-1}$): 3225 (broad), 1760, 1740, 1605, 1505, 1458, 1240, 1160, 1030, 950, 825, 767.

NMR δ ($CDCl_3$): 0.05 (6H, s), 0.86 (9H, s), 1.13 (3H, d, J=6 Hz), 1.21 (3H, d, J=7 Hz), 2.70 (1H, m), 2.95 (1H, dd, J=2 and 4 Hz), 3.81 (3H, s), 3.89 (1H, dd, J=2 and 5 Hz), 4.16 (1H, m), 5.05 (2H, s), 5.96 (1H, broad, s), 6.87 (2H, d, J=9 Hz), 7.27 (2H, d, J=9 Hz).

Reference Example 2-1

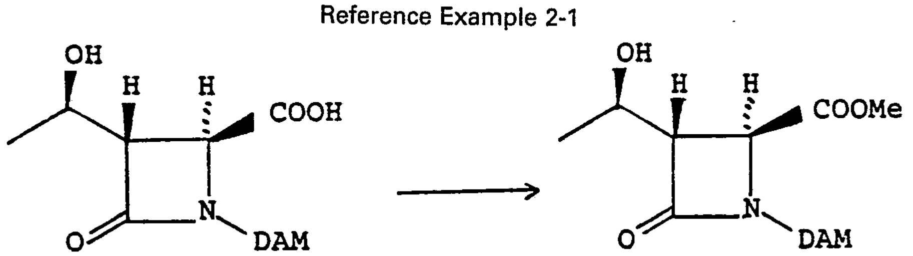

To a solution of (3S,4S) - 4 - carboxy - 3 - (1 - (R) - hydroxyethyl) - 1 - di(p - anisyl)methyl - 2 -

azetidinone (34 g) in methanol (310 ml), there was added 98% sulfuric acid (2.9 g). The resultant mixture was heated at 65°C for 3 hours, cooled down to 40°C, neutralized with 8% aqueous sodium hydroxide solution (15 ml) and concentrated to make a one third volume. The concentrate was diluted with 1,2-dichloroethane (105 ml) and washed with water. The aqueous layer was separated from the organic layer and extracted with 1,2-dichloroethane (105 ml). The extract was combined with the organic layer, washed with water and dried over anhydrous sodium sulfate. After filtration, the filtrate was concentrated in vacuo to give (3S,4S) - 4 - methoxycarbonyl - 3 - (1 - (R) - hydroxyethyl) - 1 - di(p - anisyl)methyl - 2 - azetidinone. m.p., 102—104°C.

Reference Example 2-2

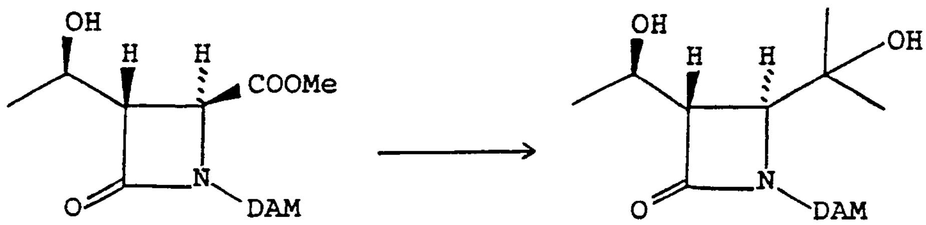

To a solution of (3S,4S) - 4 - methoxycarbonyl - 3 - (1 - (R) - hydroxyethyl) - 1 - di(p - anisyl)-methyl - 2 - azetidinone (32.5 g) in dry tetrahydrofuran (310 ml), there was added dropwise a 1M suspension of methyl magnesium bromide in tetrahydrofuran (370 g) at 0—5°C, and the suspension was stirred at the same temperature as above for 1 hour. 20% Hydrochloric acid (350 ml) was poured into the suspenson at 20—25°C, and the resultant mixture was stirred for 1 hour, followed by extraction with ethyl acetate (110 ml). The aqueous layer was reextracted with ethyl acetate (110 ml). The extracts were combined together, washed successively with brine, a saturated sodium bicarbonate solution and water and dried over anhydrous sodium sulfate. After filtration, the filtrate was concentrated in vacuo to give (3S,4S) - 4 - (1 - hydroxy - 1 - methylethyl) - 3 - (1 - (R) - hydroxyethyl) - 1 - di(p - anisyl)methyl - 2 - azetidinone. m.p., 154—156°C.

Reference Example 2-3

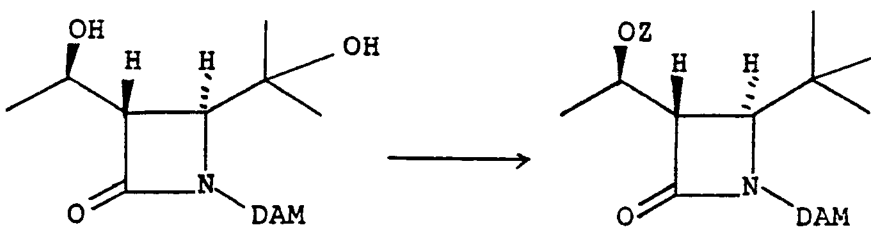

(3S,4S) - 4 - (1 - Hydroxy - 1 - methylethyl) - 3 - (1 - (R) - hydroxyethyl) - 1 - di(p - anisyl)methyl - 2 - azetidinone (26 g) and 4-dimethylaminopyridine (16 g) were dissolved in dry dichloromethane (200 ml). Benzyl chloroformate (20 g) was added dropwise thereto over a period of 1 hour with ice-cooling, and the resultant mixture was stirred for 2 hours and warmed to room temperature, followed by stirring at the same temperature as above for 10 hours. 5% Hydrochloric acid (100 ml) was poured into the reaction mixture with ice-cooling, and the resulting mixture was stirred for 0.5 hour and allowed to stand. The organic layer was washed successively with water, a saturated sodium bicarbonate solution and brine and dried over anhydrous sodium sulfate. After filtration, the filtrate was concentrated in vacuo to give (3S,4S) - 4 - (1 - hydroxy - 1 - methylethyl) - 3 - (1 - (R) - benzyloxycarbonyloxyethyl) - 1 - di(p - anisyl)methyl - 2 - azetidinone.

IR $\nu_{max}^{neat}$ ($cm^{-1}$): 3450, 1750, 1615, 1515, 1250, 1180, 1030.

NMR δ ($CDCl_3$): 1.13 (6H, s), 1.38 (3H, d, J=6 Hz), 3.70 (3H, s), 3.75 (3H, s), 5.10 (2H, s), 5.55 (1H, bs), 7.29 (5H, s).

Reference Example 2-4

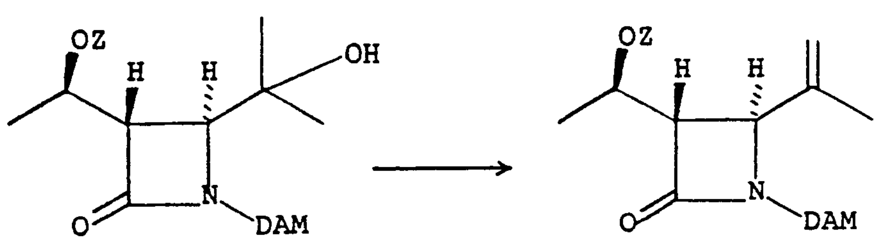

A solution of (3S,4S) - 4 - (1 - hydroxy - 1 - methylethyl) - 3 - (1 - (R) - benzyloxycarbonyloxyethyl) - 1 - di(p - anisyl)methyl - 2 - azetidinone (30 g) in dry toluene (350 ml) was treated with thionyl chloride (9.0 g) at 20—30°C for 5 hours in the presence of pyridine (10 ml). Water (100 ml) was added to quench the reaction at 10—25°C. The organic layer was separated, washed with water and dried over anhydrous sodium sulfate. After filtration, the filtrate was concentrated in vacuo to give an oily residue, which was crystallized from a mixture of cyclohexane and ethyl acetate to yield (3S,4S) - 4 - (1 - methylethyl) - 3 - (1 - (R) - benzyloxycarbonyloxyethyl) - 1 - di(p - anisyl)methyl - 2 - azetidinone. m.p., 117—118°C.

Reference Example 2-5

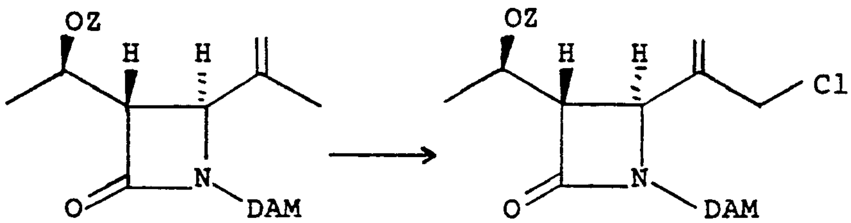

(3S,4S) - 4 - (1 -Methylethenyl) - 3 - (1 - (R) - benzyloxycarbonyloxyethyl) - 1 - di(p - anisyl)-methyl - 2 - azetidinone (200 g) was dissolved in ethyl acetate (3 liters), and a solution of chlorine in carbon tetrachloride (3.85%, 870 g) was added dropwise thereto at room temperature over a period of 15 minutes, followed by stirring for 1 hour. Water (1 liter) and then 10% aqueous sodium thiosulfate solution (50 ml) were poured into the reaction mixture, which was stirred for 0.5 hour and allowed to stand. The organic layer was washed successively with a saturated sodium bicarbonate solution and brine and dried over anhydrous sodium sulfate. After filtration, the filtrate was concentrated in vacuo to give (3S,4S) - 4 - (1 - chloromethylethenyl) - 3 - (1 - (R) - benzyloxycarbonyloxyethyl) - 1 - di(p - anisyl)methyl - 2 - azetidinone. m.p., 84—85°C.

Reference Example 2-6

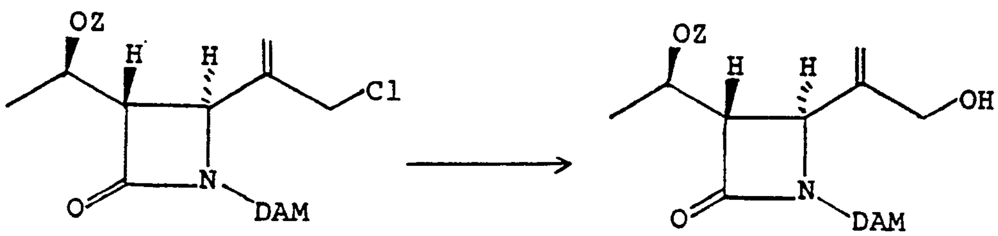

To a solution of (3S,4S) - 4 - (1 - chloromethylethyl) - 3 - (1 - (R) - benzyloxycarbonyloxyethyl) - 1 - di(p - anisyl)methyl - 2 - azetidinone (20 g) in dimethylsulfoxide (160 ml), there were successively added water (40 ml), cuprous oxide (6.76 g) and p-toluenesulfonic acid (7.6 g), and the resultant mixture was warmed to 50 to 55°C and stirred for 2 hours at the same temperature. After cooling down to room temperature, 1% aqueous phosphoric acid (90 ml) and ethyl acetate (200 ml) were poured into the reaction mixture, followed by stirring for 0.5 hour. An insoluble material was removed by filtration over celite and washed 3 times with ethyl acetate (20 ml). The filtrate and the washings were combined together, and the aqueous layer was separated from the organic layer and extracted with ethyl acetate (200 ml). The organic layer and the extract were combined together, washed with brine and dried over anhydrous sodium sulfate. After filtration, the filtrate was concentrated in vacuo, and the concentrate was crystallized from a mixture of toluene and n-hexane (1:1) to give crystals of (3S,4S) - 4 - (1 - hydroxymethylethenyl) - 3 - (1 - (R) - benzyloxycarbonyloxyethyl) - 1 - di(p - anisyl)methyl - 2 - azetidinone. m.p., 118—120°C.

Reference Example 2-7

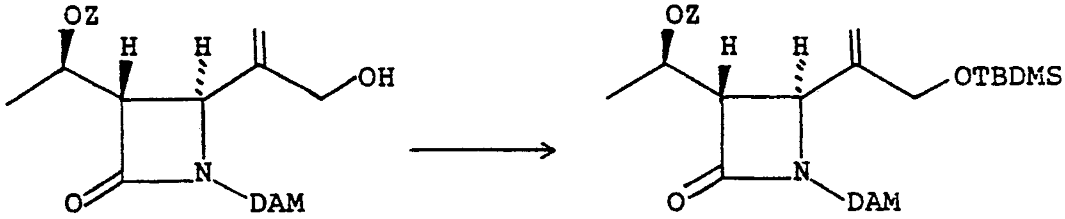

A solution of (3S,4S) - 4 - (1 - hydroxymethylethenyl) - 3 - (1 - (R) - benzyloxycarbonyloxyethyl) -

1 - di(p - anisyl)methyl - 2 - azetidinone (20 g) and imidazole (5.6 g) in dry dimethylformamide (45 ml) was treated with t-butyldimethylchlorosilane (6.77 g) at room temperature for 2 hours. The reaction mixture was diluted with cold water (200 ml) and ethyl acetate (150 ml). The aqueous layer was extracted with ethyl acetate (150 ml). The combined extracts were washed successively with 5% hydrochloric acid solution (80 ml × 2) and brine (80 ml), and dried over anhydrous sodium sulfate. After filtration, the filtrate was concentrated in vacuo, and the concentrate was crystallized from isopropanol to give crystals of (3S,4S) - 4 - (1 - t - butyldimethylsilyloxymethylethenyl) - 3 - (1 - (R) - benzyloxycarbonyloxyethyl) - 1 - di(p - anisyl)methyl - 2 - azetidinone. m.p., 90—92°C.

Reference Example 2-8

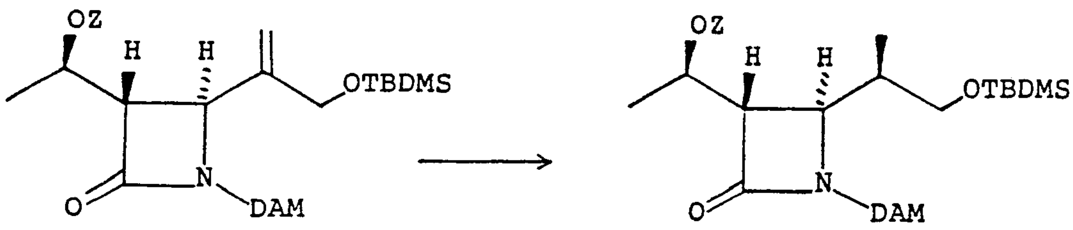

To a solution of (3S,4S) - 4 - (1 - t - butyldimethylsilyloxymethylethenyl) - 3 - (1 - (R) - benzyloxycarbonyloxyethyl) - 1 - di(p - anisyl)methyl - 2 - azetidinone (20 g) in acetonitrile (200 ml), there were added 5% platinium on activated carbon (4.0 g) and water (4 ml) under nitrogen atmosphere. The mixture was stirred at 10°C in a hydrogen gas flow until 2.2 equivalents of hydrogen had been taken up. The catalyst was removed by filtration and washed with ethyl acetate. The filtrate and the washings were combined together and concentrated in vacuo to give (3S,4S) - 4 - (1 - t - butyldimethylsilyloxymethylethenyl) - 3 - (1 - (R) - benzyloxycarbonyloxyethyl) - 1 - di(p - anisyl)methyl - 2 - azetidinone as an oil.

High performance liquid chromatography (HPLC) [Lichrosorb[R] RP—18], eluting with 85% acetonitrile/water (1 ml/min) and NMR spectra indicated that this product was a mixture of 4-(1-(R)-t-butyldimethylsilyloxyethyl) compound and the corresponding (S)-compound is a ratio of 7.7:1. The above mixture was recrystallized from a mixture of n-hexane and ethyl acetate (10:1) to yield the (R)-compound. m.p. 78—81°C.

NMR δ ($CDCl_3$): 0.01 (6H, s), 0.87 (9H, s), 1.40 (3H, d, J=6 Hz), 3.31 (1H, dd, J=2.2 and 7.0 Hz), 3.44 (2H, d, J=5.3 Hz), 3.73 (3H, s), 3.76 (3H, s), 5.07 (1H, m), 5.17 (2H, s), 7.38 (5H, s).

Reference Example 2-9

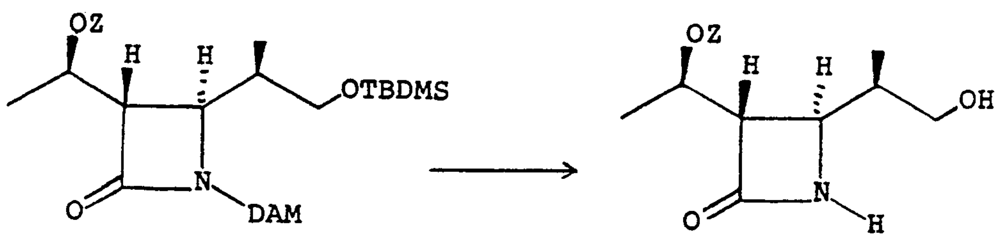

To a solution of (3S,4R) - 4 - (1 - (R) - t - butyldimethylsilyloxymethylethyl) - 3 - (1 - (R) - benzyloxycarbonyloxyethyl) - 1 - di(p - anisyl)methyl - 2 - azetidinone (20 g) in dry dichloromethane (200 ml), there were added 1,3-dimethoxybenzene (7.8 g) and boron trifluoride etherate (23 g) at 10—20°C, and the resultant mixture was stirred at room temperature for 3 hours, followed by heating under reflux for 3—5 hours. The reaction mixture was cooled down to 10—15°C, washed successively with brine (200 ml × 2), 2.5% aqueous sodium bicarbonate solution (200 ml) and brine (200 ml) and dried over anhydrous sodium sulfate. After filtration, the filtrate was concentrated in vacuo to give an oily residue, which was purified by silica gel chromatography to yield (3S,4S) - 4 - (1 - (R) - hydroxymethylethyl) - 3 - (1 - (R) - benzyloxycarbonyloxyethyl) - 2 - azetidinone.

IR $\nu_{max}^{neat}$ ($cm^{-1}$): 3350, 1750, 1740, 1455, 1382, 1260, 1030.

NMR δ ($CDCl_3$): 0.95 (3H, d, J=7.0 Hz), 1.48 (3H, d, J=6.5 Hz), 3.14 (1H, dd, J=2 and 9 Hz), 3.55 (1H, d, J=2 Hz), 5.15 (2H, s), 6.05 (1H, broad, s), 7.37 (5H, s).

Reference Example 2-10

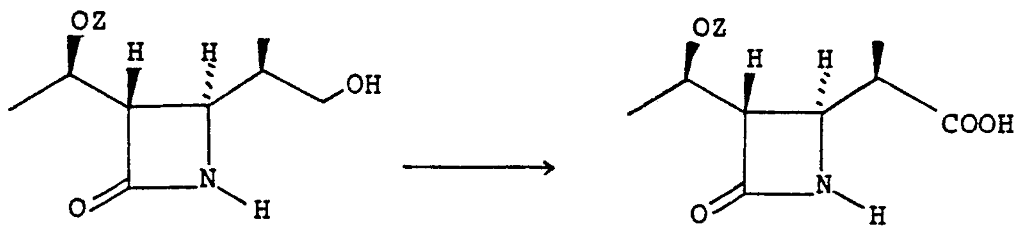

A solution of (3S,4S) - 4 - (1 - (R) - hydroxymethylethyl) - 3 - (1 - (R) - benzyloxycarbonyloxyethyl) - 2 - azetidinone (6.1 g) in acetone (60 ml) was treated with the Jones reagent, prepared from chromium trioxide (2.78 g), 98% sulfuric acid (4.4 g) and water (8.1 ml), at 10—20°C for 1 hour. The reaction mixture was quenched with isopropanol (0.5 ml) at 10—20°C for 15 minutes, diluted with ethyl acetate (122 ml) and washed with water (135 ml). The aqueous layer was separated from the organic layer and extracted with ethyl acetate (61 ml). The ethyl acetate extracts and the organic layer were combined together and extracted with 5% aqueous sodium bicarbonate solution (30 ml). The extract was washed with dichloromethane (60 ml) and acidified with 10% hydrochloric acid solution (20 ml) with ice-cooling. The acidic solution was extracted twice with dichloromethane (60 ml). The extracts were washed with brine and dried over anhydrous sodium sulfate. After filtration, the filtrate was concentrated in vacuo to give (3S,4S) - 4 - (1 - (R) - carboxyethyl) - 3 - (1 - (R) - benzyloxycarbonyloxyethyl) - 2 - azetidinone.

IR $\nu_{max}^{neat}$ ($cm^{-1}$): 3270, 1740, 1460, 1385, 1270, 750.

NMR δ ($CDCl_3$): 1.19 (3H, d, J=7.0 Hz), 1.40 (3H, d, J=6.2 Hz), 2.67 (1H, m), 3.22 (1H, broad, d, J=7.5 Hz), 3.84 (1H, broad, d, J=5.5 Hz), 5.14 (2H, s), 6.57 (1H, broad, s), 7.35 (5H, s), 7.63 (1H, broad, s).

Reference Example 2-11

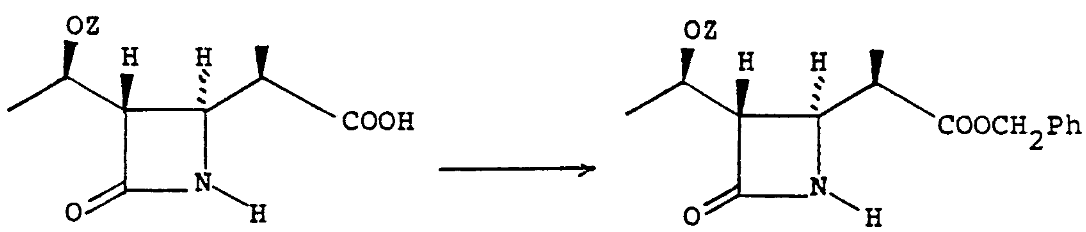

To a solution of 4 - (1 - (R) - carboxyethyl) - 3 - (1 - (1R) - benzyloxyethyl)azetidin - 2 - one (51.69 g) in acetone (510 ml), there were added anhydous potassium carbonate (89.0 g) and benzyl bromide (30.3 g), followed by stirring at 60°C for 1.5 hours. The reaction mixture was cooled to room temperature and filtered off to remove insoluble materials. The filtrate and the washings were combined and evaporated. The residue wsa purified by silica gel chromatography to give (3S,4S) - 3 - [(1R) - benzyloxycarbonyloxyethyl) - 4 - [(1R) - 1 - benzyloxycarbonylethyl]azetidin - 2 - one.

IR $\nu_{max}^{neat}$ ($cm^{-1}$): 1760 (sh), 1735, 1450, 1380, 1260, 1155.

NMR δ ($CDCl_3$): 1.22 (3H, d, J=6.9 Hz), 1.39 (3H, d, J=6.3 Hz), 2.71 (1H, q, J=6.9 Hz), 3.19 (1H, dd, J=2.0 and 7.9 Hz), 3.83 (1H, dd, J=2.0 and 6.3 Hz), 5.92 (1H, s).

Reference Example 3-1

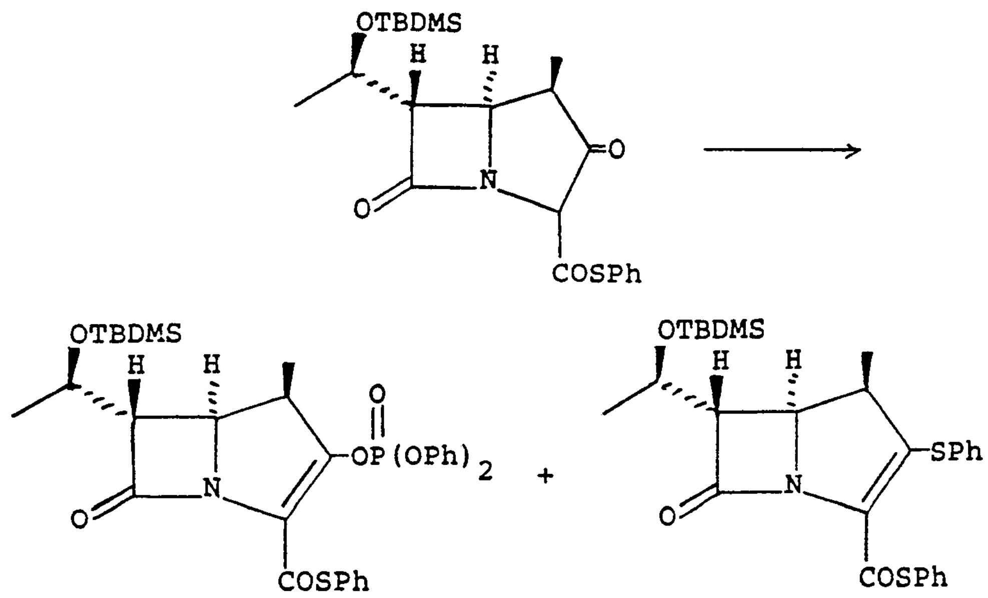

To a solution of (4R,5R,6S,8R) - 4 - methyl - 6 - (1 - t - butyldimethylsilyloxyethyl) - 1 - azabicyclo-[3.2.0]hept - 3,7 - dione - 2 - carboxylic acid phenylthioester (about 0.22 mmol) containing thiophenol in dry acetonitrile (0.8 ml), there were added a solution of diisopropylethylamine (59 mg) in dry acetonitrile (0.5 ml) and a solution of diphenyl chlorophosphate (124 mg) in dry acetonitrile (0.5 mg) under a nitrogen stream while ice-cooling, followed by stirring for 1 hour. The reaction mixture was diluted with diethyl ether and a phosphate buffer solution (pH, 6.86). The aqueous layer was separated from the organic layer and extracted with diethyl ether two times. The extracts were combined with the organic layer, washed successively with a 0.1M potassium dihydrogen phosphate solution (three times), water (two times) and brine, dried over sodium sulfate and evaporated. The residue was purified by silica gel chromatography to give (4R,5R,6S,8R) - 3 - (diphenylphosphoryloxy) - 4 - methyl - 6 - [1 - t - butyldimethylsilyloxyethyl] - 1 - azabicyclo[3.2.0]hept - 2 - en - 7 - one - 2 - carboxylic acid phenylthioester (Compound A) and (4R,5R,6S,8R) - 3 - phenylthio - 4 - methyl - 6 - (1 - t - butyldimethylsilyloxyethyl) - 1 - azabicyclo-[3.2.0]hept - 2 - en - 7 - one - 2 - carboxylic acid phenylthioester (Compound B).

Compound A:—
IR $\nu_{max}^{neat}$ ($cm^{-1}$): 1778, 1673, 1607, 1582, 1487, 1198, 1182, 1002, 962, 935, 767, 740, 680.
NMR δ ($CDCl_3$): 0.09 (3H, s), 0.11 (3H, s), 0.91 (9H, s), 1.21 (3H, d, J=7.3 Hz), 1.22 (3H, d, J=6.0 Hz), 3.29 (1H, dd, J=3.0 and 5.0 Hz), 3.52 (1H, m), 4.28 (2H, m).

Compound B:—
IR $\nu_{max}^{CHCl_3}$ ($cm^{-1}$): 1780, 1660 (sh), 1647, 1520, 1478, 1285, 1260, 1115, 1018, 945, 837.
NMR δ ($CDCl_3$): 0.09 (3H, s), 0.13 (3H, s), 0.92 (9H, s), 0.95 (3H, d, J=7.6 Hz), 1.17 (3H, d, J=6.3 Hz), 3.07 (1H, m), 3.20 (1H, dd, J=2.6 and 4.3 Hz), 4.31 (1H, dd, J=2.8 and 9.8 Hz), ~ 4.3 (1H, m), 7.3—7.6 (10H, m).

Reference Example 3-2(1)

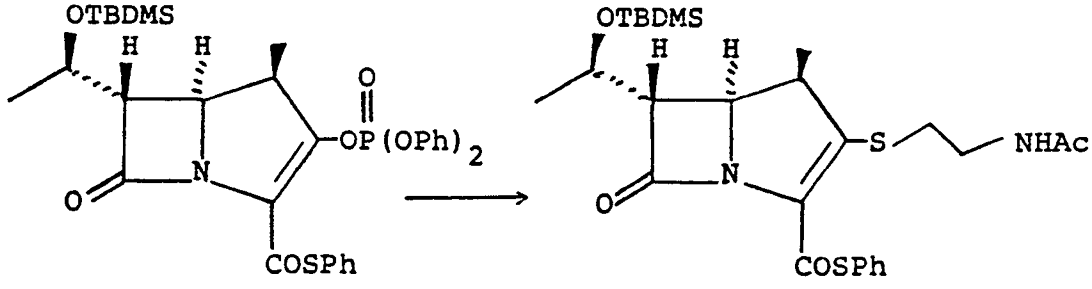

To a solution of (4R,5R,6S,8R) - 3 - (diphenylphosphoryloxy) - 4 - methyl - 6 - (1 - t - butyldimethylsilyloxyethyl) - 1 - azabicyclo[3.2.0]hept - 2 - en - 7 - one - 2 - carboxylic acid phenylthioester (10 mg) in

dry acetonitrile (0.1 ml), there were added a solution of diisopropylethylamine (5.2 ml) in dry acetonitrile (0.2 ml) and a solution of N-acetylcysteamine (4.8 mg) in dry acetonitrile (0.2 ml) at −30°C under a nitrogen stream. The reaction mixture was warmed gradually to −20°C and diluted with diethyl ether and a phosphate buffer solution (pH, 6.86). The aqueous layer was separated from the organic layer and extracted with diethyl ether two times. The extracts were combined with the organic layer, washed successively with a phosphate buffer solution (pH, 6.86), a 0.1M potassium dihydrogen phosphate solution and brine, dried over sodium sulfate and evaporated. The residue was purified by silica gel chromatography to give (4R,5R,6S,8R) - 3 - (2 - acetaminoethylthio) - 4 - methyl - 6 - [1 - t - butyldimethylsilyloxyethyl) - 1 - azabicyclo[3.2.0]hept - 2 - en - 7 - one - 2 - carboxylic acid phenylthio-ester.

IR $\nu_{max}^{CHCl_3}$ (cm$^{-1}$): 3460, 1765, 1665, 1250, 1102, 830.

NMR δ ($CDCl_3$): 0.11 (3H, s), 0.14 (3H, s), 0.94 (9H, s), 1.25 (3H, d, J=7.3 Hz), 1.25 (3H, d, J=6.3 Hz), 1.97 (3H, s), 5.9 (1H, broad s), 7.3—7.6 (5H, m).

Reference Example 3-2(2)

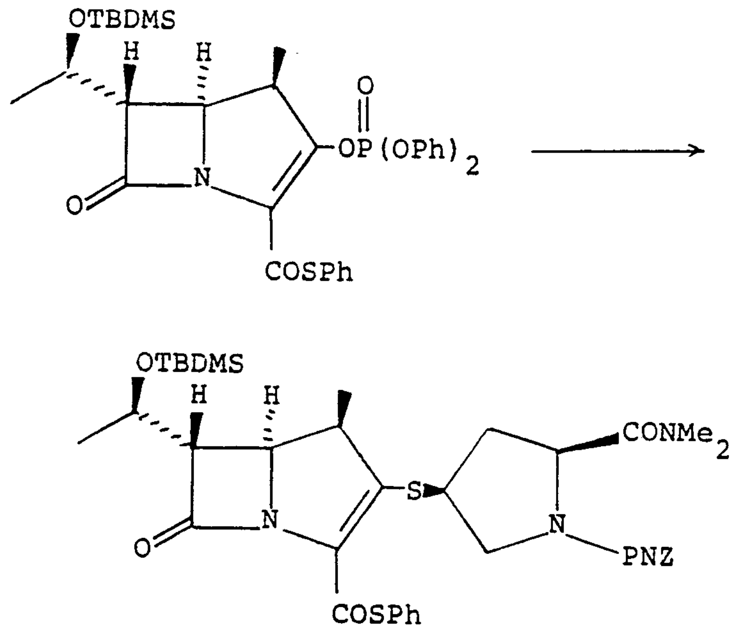

In the same manner as in Reference Example 3-2(1), (4R,5R,6S,8R) - 3 - (diphenylphosphoryloxy) - 4 - methyl - 6 - (1 - t - butyldimethylsilyloxyethyl) - 1 - azabicyclo[3.2.0]hept - 2 - en - 7 - one - 2 - carboxylic acid phenylthio ester (6 mg), diisopropylamine (1.4 mg) and (2S,4S,) - 1 - p - nitrobenzyloxycarbonyl - 4 - mercaptopyrrolidine (4 mg) were treated to give (4R,5R,6S,8R,2'S,4'S) - 3 - [(1 - p - nitrobenzyloxycarbonyl - 2 - dimethylaminocarbonylpyrrolidin) - 4 - ylthio] - 4 - methyl - 6 - (1 - t - butyldimethylsilyloxyethyl) - 1 - azabicyclo[3.2.0]hept - 2 - en - 7 - one - 2 - carboxylic acid phenylthioester.

IR $\nu_{max}^{CHCl_3}$ (cm$^{-1}$): 1767, 1700, 1650, 1518, 1340, 1100.

Reference Example 3-2(3)

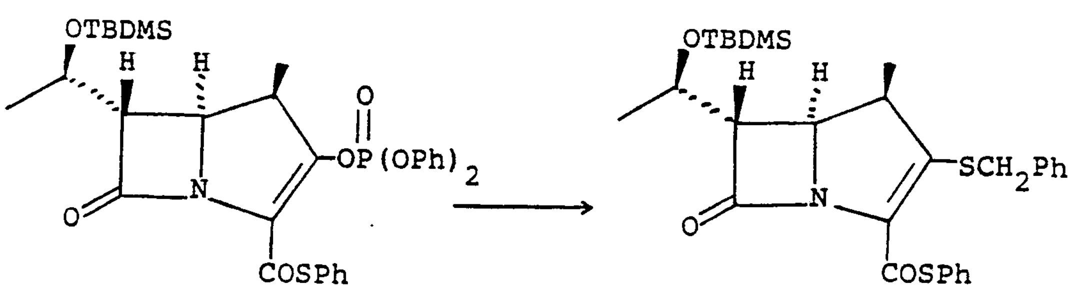

In the same manner as in Reference Example 3-2(1), (4R,5R,6S,8R) - 3 - (diphenylphosphoryloxy) - 4 - methyl - 6 - (1 - t - butyldimethylsilyloxyethyl) - 1 - azabicyclo[3.2.0]hept - 2 - en - 7 - one - 2 -

carboxylic acid phenylthio ester (20 mg), diisopropylethylamine (5.2 mg) and benzylmercaptan (5 mg) were treated to give (4R,5R,6S,8R) - 3 - benzylthio - 4 - methyl - 6 - (1 - t - butyldimethylsilyloxyethyl) - 1 - azabicyclo[3.2.0]hept - 2 - en - 7 - one - 2 - carboxylic acid phenylthio ester.

IR $\nu_{max}^{CHCl_3}$ ($cm^{-1}$): 1770, 1660 (sh), 1640, 1298, 1266, 1250, 1142, 1102, 835.

NMR δ ($CDCl_3$): 0.10 (3H, s), 0.13 (3H, s), 0.92 (9H, s), 3.24 (1H, dd, J=2.6 and 5.3 Hz), 3.37 (1H, m), 4.09 (1H, m), 4.2—4.4 (2H, m), 7.30 (5H, s), 7.3—7.6 (5H, m).

Reference Example 4

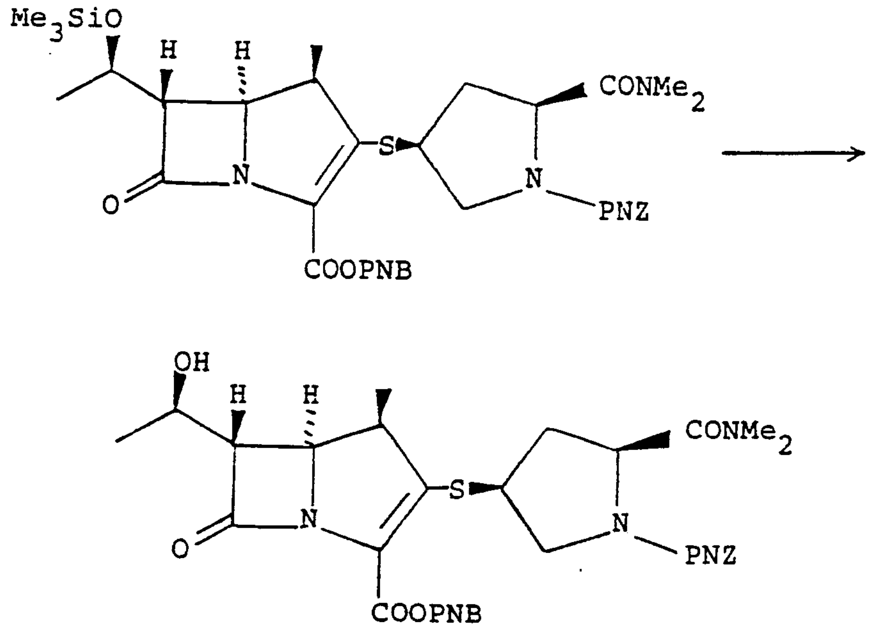

To a solution of (4R,5S,6S,8R,2'S,4'S) - p - nitrobenzyl - 3 - [4 - (1 - p - nitrobenzyloxycarbonyl - 2 - dimethylaminocarbonylpyrrolidinyl)thio] - 4 - methyl - 6 - (1 - trimethylsilyloxyethyl) - 1 - azabicyclo-[3.2.0]hept - 2 - en - 7 - one - 2 - carboxylate (1.0 g) in dry tetrahydrofuran (10 ml), there was added a phosphate buffer solution (pH 3; 8 ml), and the resultant mixture was vigorously stirred at room temperature for 2.5 hours. The reaction mixture was diluted with ethyl acetate (50 ml), washed with brine, dried over magnesium sulfate and evaporated in vacuo to give (4R,5S,6S,8R,2'S,4'S) - p - nitrobenzyl - 3 - [4 - (1 - p - nitrobenzyloxycarbonyl - 2 - dimethylaminocarbonylpyrrolidinyl)thio] - 4 - methyl - 6 - (1 - hydroxyethyl) - 1 - azabicyclo[3.2.0]hept - 2 - en - 7 - one - 2 - carboxylate.

IR $\nu_{max}^{neat}$ ($cm^{-1}$): 1760, 1705, 1645, 1520, 1402, 1342, 1135, 1110.

NMR δ ($CDCl_3$): 1.30 (3H, d, J=7.0 Hz), 1.35 (3H, d, J=6.5 Hz), 2.99 (3H, s), 3.02 (3H, d, J=15 Hz), 5.21 (2H, s), 5.20 and 5.43 (2H, $AB_q$, J=14 Hz), 7.51 (2H, d, J=8.5 Hz), 7.64 (2H, d, J=8.5 Hz), 8.20 (4H, d, J=8.5 Hz).

Reference Example 5-1

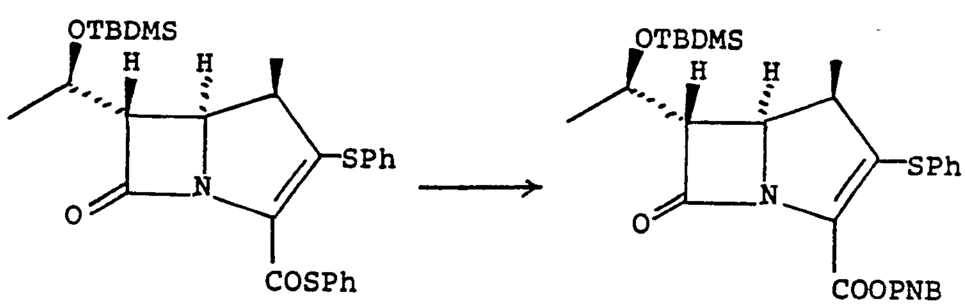

(4R,5S,6S,8R) - 3 - Phenylthio - 4 - methyl - 6 - [(1R) - 1 - t - butyldimethylsilyloxyethyl] - 1 - azabicyclo[3.2.0]hept - 2 - en - 7 - one - 2 - carboxylic acid phenylthio ester (17 mg) and p-nitrobenzyl alcohol (24 mg) were dissolved in dry methylene chloride (0.6 ml). In the dark, silver trifluoroacetate (7 mg) and 1,8-diazabicyclo[5.4.0]-7-undecene (5 mg) in dry methylene chloride (0.3 ml) were added thereto successively at room temperature. The resultant mixture was stirred for 4.3 hours and diluted with a 0.1M

phosphate buffer solution (pH 7; 3 ml) and methylene chloride. After removal of any insoluble materials by filtration, the filtrate was extracted with methylene chloride two times. The organic layer was washed successively with a 2.5% sodium dihydrogen phosphate solution and brine, dried over sodium sulfate and magnesium sulfate and evaporated. The residue was purified by silica gel chromatography to give (4R,5S,6S,8R) - 3 - phenylthio - 4 - methyl - 6 - [(1R) - 1 - t - butyldimethylsilyloxyethyl] - 1 - azabicyclo[3.2.0]hept - 2 - en - 7 - one - 2 - carboxylic acid p-nitrobenzyl ester.

IR and NMR spectra of this compound were identical to those of the compound as in Example 11-4.

Reference Example 5-2(1)

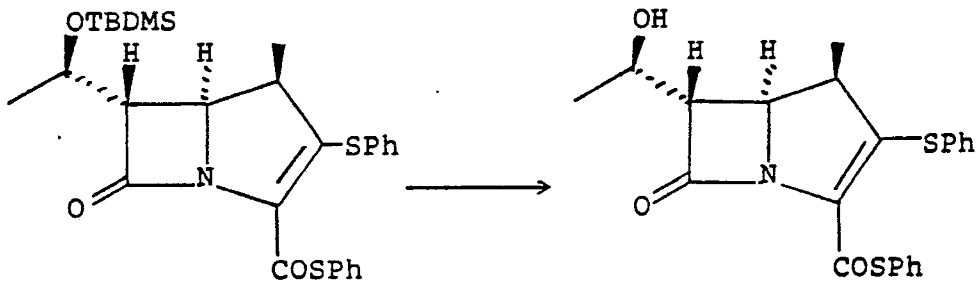

To a solution of (4R,5S,6S,8R) - 3 - phenylthio - 4 - methyl - 6 - [(1R) - 1 - t - butyldimethylsilyloxyethyl] - 1 - azabicyclo[3.2.0]hept - 2 - en - 7 - one - 2 - carboxylic acid phenylthio ester (20 mg) in dry tetrafuran (0.3 ml), there was dropwise added a 0.27 M solution of tetra-n-butylammonium fluoride (0.14 ml) in tetrahydrofuran under a nitrogen stream with ice-cooling, followed by stirring for 1 hour. The reaction mixture was diluted with a phosphate buffer solution (pH 7) and extracted with methylene chloride three times. The organic layer was washed with brine, dried over sodium sulfate and evaporated in vacuo to give an oily residue, which was purified by silica gel chromatography to give (4R,5S,6S,8R) - 3 - phenylthio - 4 - methyl - 6 - [(1R) - 1 - hydroxyethyl] - 1 - azabicyclo[3.2.0]hept - 2 - en - 7 - one - 2 - carboxylic acid phenylthio ester.

IR $\nu_{max}^{CHCl_3}$ ($cm^{-1}$): 3600 (broad), 1775, 1660 (sh), 1645, 1300, 1273.

NMR δ ($CDCl_3$): 0.98 (3H, d, J=7.3 Hz), 1.35 (3H, d, J=6.3 Hz), 3.11 (1H, m), 3.23 (1H, dd, J=2.3 and 6.9 Hz), 4.27 (1H, dd, J=2.6 and 9.2 Hz), — 4.3 (1H, m), 7.3—7.6 (10H, m).

Reference Example 5-2(2)

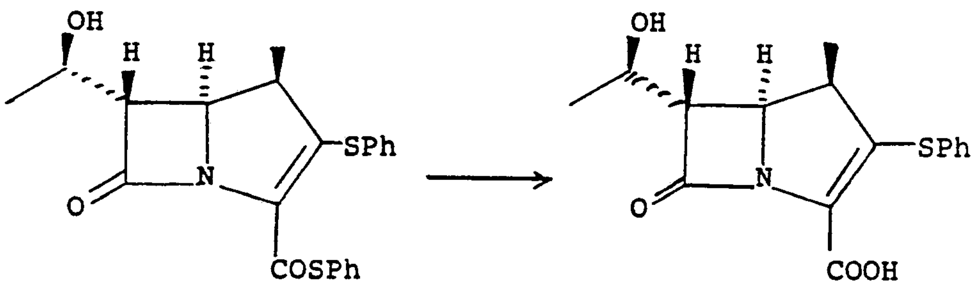

To a mixture of (4R,5S,6S,8R) - 3 - phenylthio - 4 - methyl - 6 - (1 - hydroxyethyl) - 1 - azabicyclo-[3.2.0]hept - 2 - en - 7 - one - 2 - carboxylic acid phenylthio ester (3 mg) and trimethylsilanol (14 mg) in dry toluene (0.1 ml), there were added silver trifluoroacetate (1.6 mg) and a solution of 1,8-diazabicyclo-[5.4.0]-7-undecene (1.1 mg) in dry toleune (0.1 ml) at room temperature, followed by stirring at 80°C for 15 minutes. The reaction mixture was cooled down to room temperature and diluted with a 0.1M phosphate buffer solution (pH 7; 1 ml) and methylene chloride. After removal of insoluble materials by filtration, the filtrate was extracted with methylene chloride two times. The aqueous layer was stirred under reduced pressure to remove any organic solvent. By identification of HPLC (Lichrosorb® RP—18; MeOH (pH 7.0—7.2)/0.005 M phosphate buffer (3:7); 0.8 ml/minute) and TLC (silica gel; $CHCl_3$/MeOH/acetic acid (200:50:1), (4R,5S,6S,8R) - 3 - phenylthio - 4 - methyl - 6 - (1 - hydroxyethyl) - 1 - azabicyclo-[3.2.0]hept - 2 - en - 7 - one - 2 - carboxylic acid, i.e. the compound in Reference Example 6-(2), was found to be present in the aqueous layer.

Reference Example 6-1

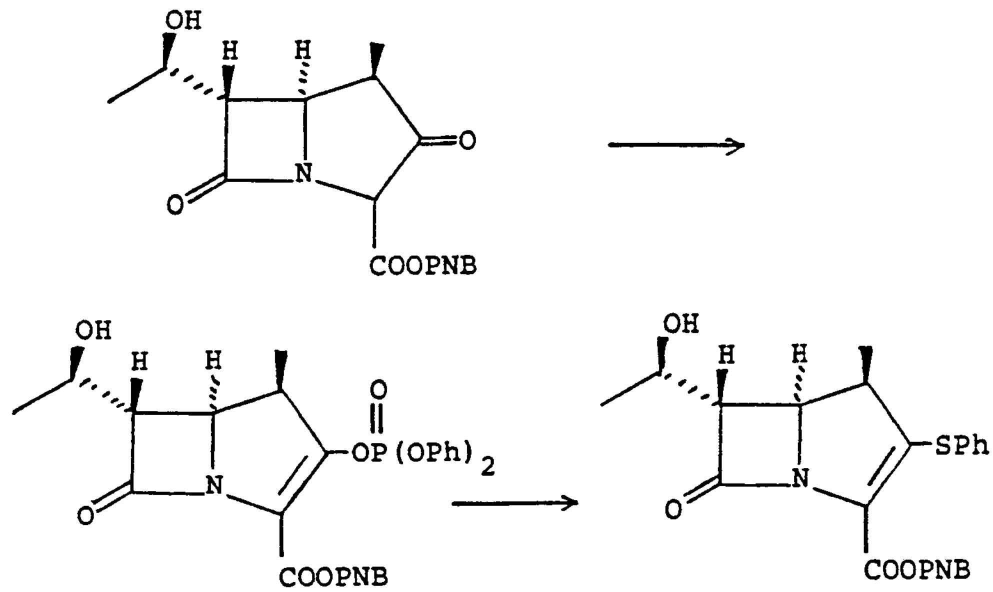

To a solution of (4R,5S,6S,8R) - 4 - methyl - 6 - (1 - hydroxyethyl) - 1 - azabicyclo[3.2.0]hept - 3,7 - dione - 2 - carboxylic acid p-nitrobenzyl ester (100 mg) in dry acetonitrile (1 ml), there were added a solution of diphenylchlorophosphate (67 mg) in dry acetonitrile (0.5 ml) and a solution of diisopropylethylamine (32 mg) in dry acetonitrile (0.5 ml) under a nitrogen stream while ice-cooling. The resultant mixture was stirred at the same temperature for 30 minutes. The reaction mixture was cooled down to −30°C and then thiophenol (37 mg) and diisopropylethylamine (44 mg) in dry acetonitrile (0.4 ml) were added thereto successively. The resulting mixture was stirred at the same temperature for 25 minutes and further for 15 minutes under ice-cooling. The mixture was diluted with ethyl acetate, washed successively with brine, an aqueous solution of potassium dihydrogen phosphate and brine, dried over a mixture of magnesium sulfate and potassium carbonate and evaporated. The residue was purified by silica gel chromatography to give (4R,5S,6S,8R) - 3 - phenylthio - 4 - methyl - 6 - (1 - hydroxyethyl) - 1 - azabicyclo[3.2.0]hept - 2 - en - 7 - one - 2 - carboxylic acid p-nitrobenzyl ester.

IR $\nu_{max}^{neat}$ ($cm^{-1}$): 3480 (broad), 1764, 1707, 1520, 1342, 1215, 1140.

NMR δ ($CDCl_3$): 0.97 (3H, d, J=7.3 Hz), 1.31 (3H, d, J=6.3 Hz), 3.10 (1H, m), 3.21 (1H, dd, J=2.8 and 6.8 Hz), 4.18 (1H, dd, J=2.8 and 9.4 Hz), 4.23 (1H, m), 5.42 (2H, m), 7.3—7.6 (5H, m), 7.69 (2H, d, J=8.9 Hz), 8.24 (2H, d, J=8.9 Hz).

Reference Example 6-2

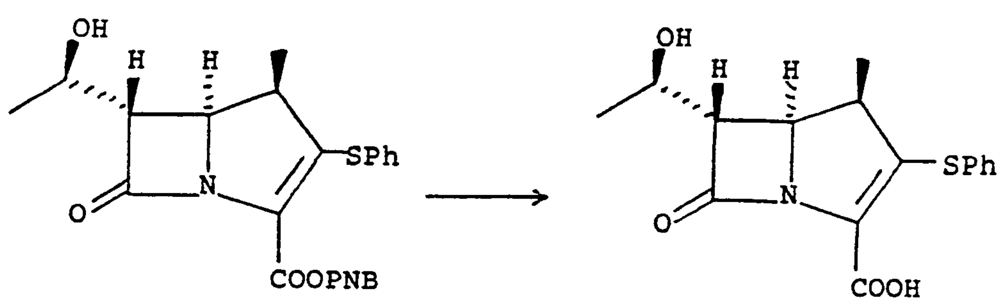

To a solution of (4R,5S,6S,8R)-3-phenylthio-4-methyl-6-(1-hydroxyethyl)-1-azabicyclo[3.2.0]hept-2-en-7-one-2-carboxylic acid p-nitrobenzyl ester (37 mg) in tetrahydrofuran (2 ml), a morpholinopropanesulfonic acid buffer solution (pH 7.0; 2 ml) was added, and the resultant mixture was subjected to hydrogenation at room temperature in the presence of 10% palladium-carbon (56 mg) under atmospheric pressure for 4.5 hours. After filtration, the filtrate was stirred under reduced pressure to remove tetrahydrofuran. The filtrate was washed with methylene chloride and stirred again under reduced pressure to remove any organic solvent. The residue was purified by polymer chromatography (CHP-20P), and the fractions eluted with 2% and 5% tetrahydrofuran-water gave (4R,5S,6S,8R)-3-phenylthio-4-methyl-6-(1-hydroxyethyl)-1-azabicyclo[3.2.0]hept-2-en-7-one-2-carboxylic acid.

UV $\lambda_{max}^{H_2O}$ (nm): 306.

IR $\nu_{max}^{KBr}$ ($cm^{-1}$): 3425 (broad), 1745, 1595, 1400.

NMR δ ($D_2O$) (ppm): 0.90 (3H, d, J=7.3 Hz), 1.18 (3H, d, J=6.3 Hz), 3.00 (1H, m), 3.32 (1H, dd, J=2.6 and 5.9 Hz), 4.09 (1H, dd, J=2.6 and 9.2 Hz), 4.16 (1H, m), 7.3—7.6 (5H, m).

Example 11-1(1)

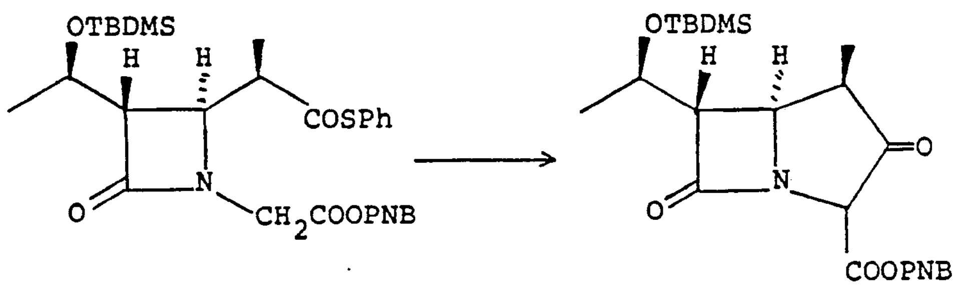

A solution of (3S,4S)-3-[(1R)-1-t-butyldimethylsilyloxyethyl]-4-[(1R)-1-phenylthiocarbonylethyl]-1-p-nitrobenzyloxycarbonylmethyl-2-azetidinone (70 mg) in dry toluene (0.5 ml) was dropwise added to a suspension of sodium hydride (12.5 mg) 50% in oil) and dry tetrahydrofuran (0.1 ml) under ice-cooling, and the resultant mixture was stirred for 30 minutes. p-Toluenesulfonic acid monohydrate (57 mg) was added thereto, and the reuslting mixture was further stirred for 10 minutes. The reaction mixture was diluted with cold ethyl acetate (20 ml), washed with brine, dried over magnesium sulfate and evaporated to give (4R,5R,6S,8R) - p - nitrobenzyl - 4 - methyl - 6 - (1 - t - butyldimethylsilyloxyethyl) - 1 - azabicyclo[3.2.0]hept - 3,7 - dione - 2 - carboxylate as an oil.

IR $\nu_{max}$ ($cm^{-1}$): 1760, 1605, 1520, 1460, 1350, 1250, 1220, 1110, 1045, 835, 780, 738.

NMR δ ($CDCl_3$): 1.21 (3H, d, J=7.6 Hz), 1.29 (3H, d, J=6.3 Hz), 2.80 (1H, m), 3.22 (1H, dd, J=2.3 and 6.3 Hz), 4.18 (1H, dd, J=2.3 Hz), 4.29 (1H, m), 4.72 (1H, s), 5.30 (2H, $AB_q$, J=13.2 Hz), 7.54 (2H, d, J=8.9 Hz), 8.24 (2H, d, J=8.9 Hz).

Example 11-1(2)

To a solution of (3S,4S)-3-[(1R)-1-t-butyldimethylsilyloxyethyl]-4-[(1R)-1-phenylthiocarbonylethyl]-1-p-nitrobenzyloxycarbonylmethyl-2-azetidinone (60 mg) in a mixture of benzene-$d_6$ and tetrahydrofuran-$d_8$ (4:1) (0.6 ml) there was added sodium hydride (11 mg; 50% in oil) under ice-cooling, followed by stirring for 1 hour. The NMR spectrum of the reaction mixture was measured and shown below in contrast to that as in Example 11-1(1).

NMR spectra data:—

| Example No. | Solvent | 5-H | 6-H |
|---|---|---|---|
| 11-1(2) | $C_6D_6$-$THFd_8$ | 3.08, d | 3.93, d |
| | (4:1) | J=6.3 Hz | J=8.9 Hz |
| 11-1(1) | $C_6D_6$-$THFd_8$ | 2.98, dd | 4.08, dd |
| | (4:1) | J=2.6 and 5.3 Hz | J=2.6 and 7.9 Hz |

Example 11-1-(3)

In the same manner as in Example 11-1(2) but replacing the solvent by a mixture of benzene-$d_6$ and dimethylsulfoxide-$d_6$ (9:1), the NMR spectrum of the reaction mixture was measured and shown below in contrast to that as in Example 11-1(1).

NMR spectra data:—

| Example No. | Solvent | 5—H | 6-H |
|---|---|---|---|
| 11-1(3) | $C_6D_6$-$DMSOd_6$ | 3.16, d | 4.00, d |
| | (9:1) | J=7.3 Hz | J=7.6 Hz |
| 11-1(1) | $C_6D_6$-$DMSOd_6$ | 3.08, dd | 4.10, dd |
| | | J=2.6 and 5.0 Hz | J=2.6 and 7.9 Hz |

Example 11-2

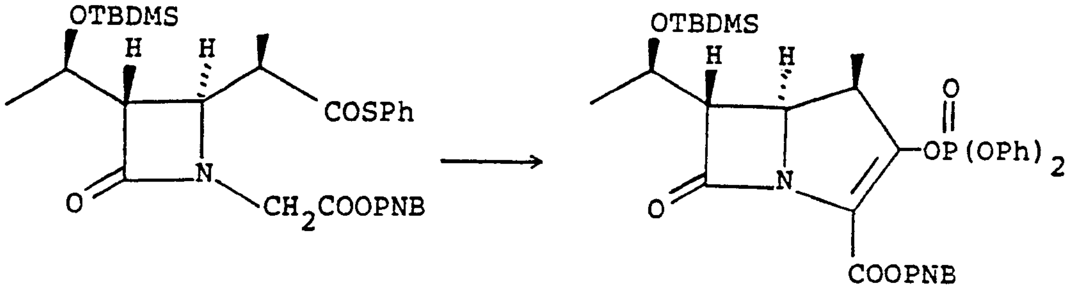

A solution of (3S,4S)-3-[(1R)-1-t-butyldimethylsilyloxyethyl]-4-[(1R)-1-phenylthiocarbonylethyl]-1-p-nitrobenzyloxycarbonylmethyl-2-azetidinone (117 mg) in a mixture of dry toluene and dry tetrahydrofuran (1:1) (1.2 ml) was dropwise added to a suspension of sodium hydride (22 ml; 50% in oil) in a mixture of dry toluene and dry tetrahydrofuran (1:1) (0.2 ml) at −20°C, followed by stirring at the same temperature for 1 hour. A 2M solution (0.1 ml) of iodomethane in tetrahydrofuran was added thereto, and stirring was continued for 30 minutes. A solution of diphenylchlorophosphate (56 ml) in dry toluene (0.1 ml) was added to the mixture at the same temperature, followed by stirring for 1.5 hours. The resultant mixture was diluted with ethyl acetate (20 ml), washed with brine, dried over a mixture of magnesium sulfate and potassium carbonate (10:1) and evaporated in vacuo to give an oily residue, which was purified by silica gel chromatography to obtain (4R,5R,6S,8R)-p-nitrobenzyl-3-diphenylphosphoryloxy-4-methyl-6-(1-t-butyldimethylsilyloxyethyl)-1-azabicyclo[3.2.0]hept-2-en-7-one-2-carboxylate (115 mg).

IR $\nu_{max}^{neat}$ ($cm^{-1}$): 1775, 1725, 1630, 1585, 1518, 1482, 1340, 1285, 1185, 1160, 938, 825, 770.

NMR δ ($CDCl_3$): 0.06 (3H, s), 0.07 (3H, s), 0.86 (9H, s), 1.20 (3H, d, J=7.9 Hz), 1.23 (3H, d, J=6.3 Hz), 3.29 (1H, dd, J=3.0 and 6.0 Hz), 3.43 (1H, m), 4.22 (2H, m), 5.28 (2H, $AB_q$, J=13.5 Hz), 7.56 (2H, d, J=8.9 Hz), 8.14 (2H, d, J=8.9 Hz).

Example 11-1(3)

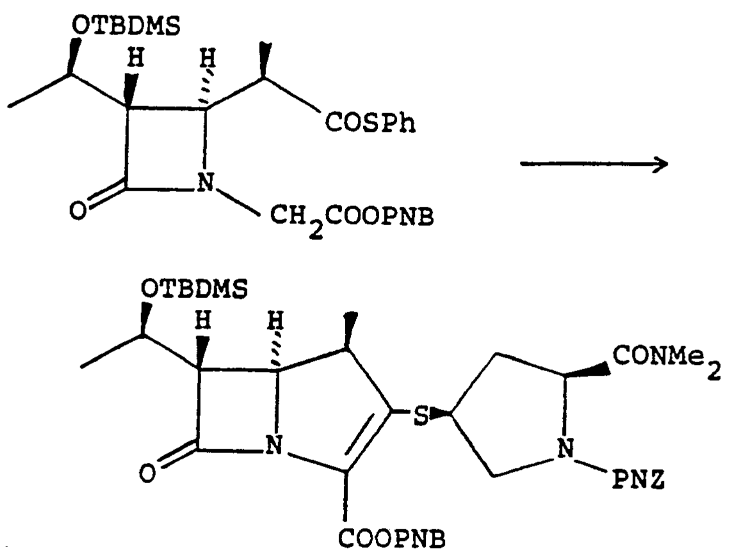

A solution of (3S,4S)-3-[(1R)-1-t-butyldimethylsilyloxyethyl]-4-[(1R)-1-phenylthiocarbonylethyl]-1-p-nitrobenzyloxycarbonylmethyl-2-azetidinone (415 mg) in a mixture of dry toluene and dry tetrahydrofuran (4:1) (4 ml) was dropwise added to a suspension of sodium hydride (75 ml; 50% in oil) in a mixture of dry toluene and dry tetrahydrofuran (4:1) (0.75 ml) at −20°C, followed by stirring at the same temperature for 1 hour. A 0.5 M solution (1.49 ml) of iodomethane in tetrahydrofuran was added thereto, and stirring was continued for 30 minutes. A solution of diphenyl chlorophosphate (218.5 mg) in dry toluene (2.2 ml) was added to the mixture at the same temperature, followed by stirring for 2 hours. Thereafter, (2S,4S)-1-p-nitrobenzyloxycarbonyl-2-dimethylaminocarbonyl-4-mercaptopyrrolidine (237.5 mg) and sodium hydride (32.3 mg; 50% in oil) were added thereto, and stirring was continued for 2 hours. The resultant mixture was diluted with ethyl acetate (50 ml), washed with brine, dried over magnesium sulfate and evaporated in vacuo to give an oily residue, which was purified by silica gel chromatography to obtain (4R,5S,6S,8R,2'S,4'S) - p - nitrobenzyl - 3 - [4 - (1 - p - nitrobenzyloxycarbonyl - 2 - dimethylaminocarbonyl-pyrrolidinyl)thio] - 4 - methyl - 6 - (1 - t - butyldimethylsilyloxyethyl) - 1 - azabicyclo[3.2.0]hept - 2 - en - 7 - one - 2 - carboxylate (329 mg).

IR $\nu_{max}^{neat}$ ($cm^{-1}$): 1775. 1715, 1660, 1610, 1525, 1400, 1345, 1210, 1140, 1110, 835, 755.

Example 11-4

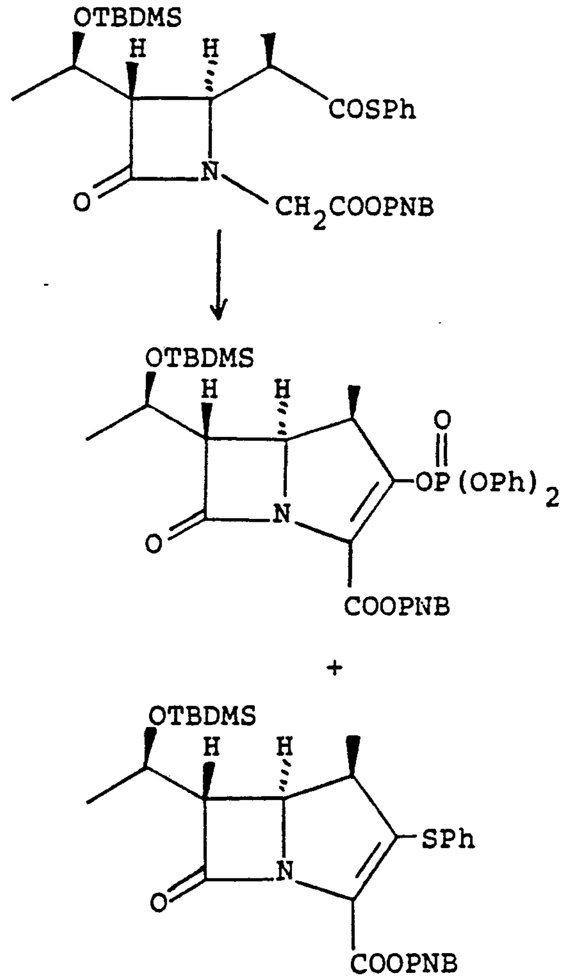

A solution of (3S,4S)-3-[(1R)-1-t-butyldimethylsilyloxyethyl]-4-[(1R)-1-phenylthiocarbonylethyl]-1-p-nitrobenzyloxycarbonylmethyl-2-azetidinone (69 mg) in dry toluene (0.6 ml) was dropwise added to a suspension of sodium hydride (12.5 ml; 50% in oil) in dry tetrahydrofuran under ice-cooling, and the suspension was stirred for 30 minutes. Diphenyl chlorophosphate (67 mg) was added thereto under ice-cooling, followed by stirring for 1 hour. The resultant mixture was diluted with ethyl acetate (10 ml), washed with brine, dried over a mixture of magnesium sulfate and potassium carbonate (10:1) and evaporated in vacuo to give an oily residue, which was purified by silica gel chromatography to obtain (4R,5S,6S,8R) - p - nitrobenzyl - 3 - phenylthio - 4 - methyl - 6 - (1 - t - butyldimethylsilyloxyethyl) -1 - azabicyclo[3.2.0]hept - 2 - en - 7 - one - 2 - carboxylate (37 mg) (Compound A) and (4R,5R,6S,8R) - p - n itrobenzyl - 3 - diphenylphosphoryloxy) - 4 - methyl - 6 - ( 1 - t - butyldimethylsilyloxyethyl) - 1 - azabicyclo-[3.2.0]hept - 2 - en - 7 - one - 2 - carboxylate (34 mg) (Compound B).

Compound A:—

IR $\nu_{max}^{neat}$ ($cm^{-1}$): 1765, 1707, 1522, 1378, 1350, 1340, 1140.

NMR δ ($CDCl_3$): 0.06 (6H, s), 0.84 (9H, s), 0.95 (3H, d, J=7.3 Hz), 1.17 (3H, d, J=6.3 Hz), 3.06 (1H, m), 3.19 (1H, dd, J=2.9 and 5.0 Hz), 4.22 (2H, m), 5.40 (2H, $AB_q$, J=13.9 Hz), 7.3—7.6 (5H, m), 7.69 (2H, d, J=8.9 Hz), 8.23 (2H, d, J=8.9 Hz).

Compound B:—

The IR and NMR spectra data were identical to those of the compound obtained as in Example 11-2.

Example 12

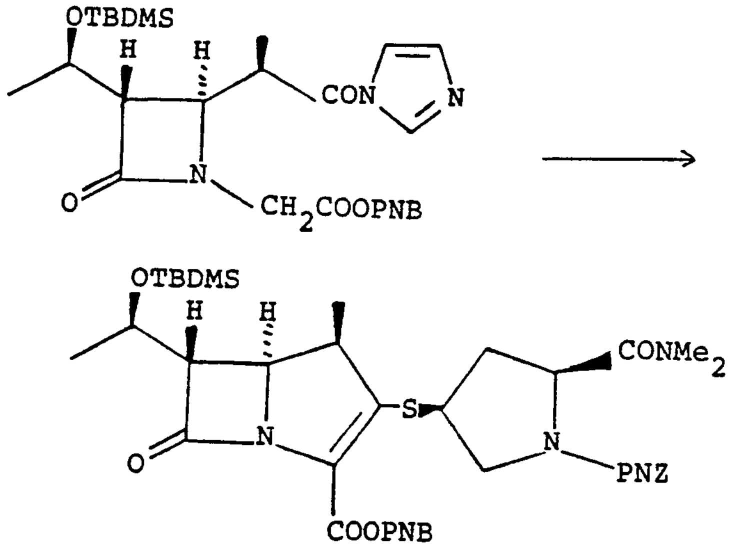

To a solution of (3S,4S)-3-[(1R)-1-t-butyldimethylsilyloxyethyl]-4-[(1R)-1-imidazolylcarbonylethyl]-1-p-nitrobenzyloxycarbonylmethyl-2-azetidinone (52 mg) in a mixture of dry toluene and dry tetrahydrofuran (4:1) (0.5 ml), there were added sodium hydride (10 mg; 50% in oil) and dry dimethylformamide (0.05 ml) under ice-cooling, followed by stirring for 1 hour. Diphenyl chlorophosphate (60 mg) was added thereto, and stirring was continued or 2 hours. To the resultant mixture, (2S,4S)-1-p-nitrobenzyloxycarbonyl-2-dimethylaminocarbonyl-4-mercaptopyrrolidine (35.3 mg) and sodium hydride (5 mg) 50% in oil) were added under ice-coling, followed by stirring for 1.5 hours. The reaction mixture was diluted with ethyl acetate (10 ml), washed with brine, dried over magnesium sulfate and evaporated in vacuo to give an oily residue, which was purified by thin layer chromatography on silica gel to obtain (4R,5S,6S,8R,2'S,4'S)-p-nitrobenzyl - 3 - [4 - (1 - p - nitrobenzyloxycarbonyl - 2 - dimethylaminocarbonylpyrrolidinyl)thio] - 4 - m ethyl - 6 - (1 - t - butyldimethylsilyloxyethyl) - 1 - azabicyclo[3.2.0]hept - 2 - en - 7 - one - 2 - carboxylate.

The IR and NMR spectra data of this compound were identical to those of the compound as in Example 11-3.

Example 13

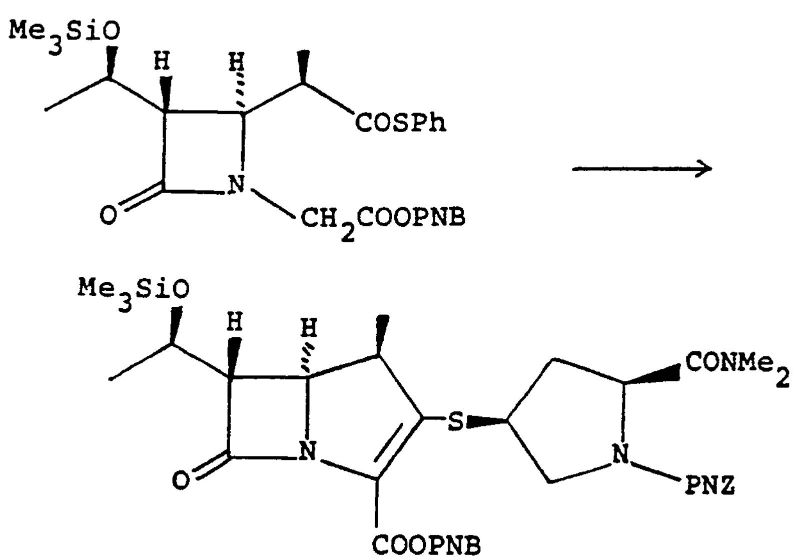

trimethylsilyloxyethyl] - 4 - [(1R) - 1 - phenylthiocarbonylethyl] - 1 - p - nitrobenzyloxycarbonylmethyl - 2 - azetidinone, there was obtained (4R,5S,6S,8R,2'S,4'S) - p - nitrobenzyl - 3 - [4 - (1 - p - nitrobenzyloxycarbonyl - 2 - dimethylaminocarbonylpyrrolidinyl)thio] - 4 - methyl - 6 - ( 1 - trimethylsilyloxyethyl) - 1 - azabicyclo[3.2.0]hept - 2 - en - 7 - one - 2 - carboxylate.

IR $\nu_{max}^{neat}$ ($cm^{-1}$): 1765, 1705, 1650, 1600, 1512, 1395, 1335, 1200, 1130, 1100, 840, 740.

Example 14-1

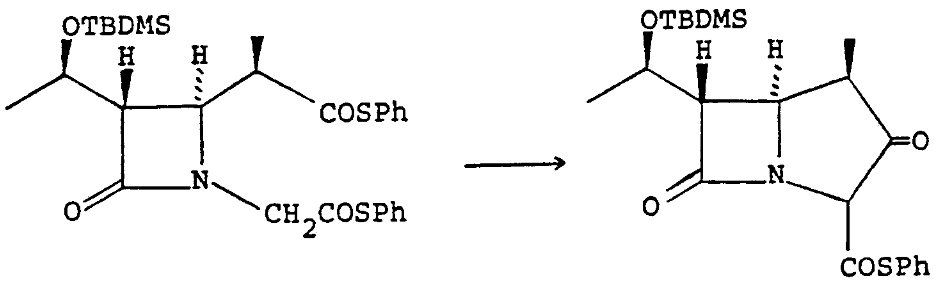

To a solution of (3S,4S)-3-[(1R)-1-t-butyldimethylsilyloxyethyl]-4-[(1R)-1-phenylthiocarbonylethyl]-1-(phenylthiocarbonylmethyl)azetidin-2-one (20 mg) in dry dimethylformamide (0.2 ml), there was added sodium hydride (2.6 mg), followed by stirring at room temperature for 20 minutes. The reaction mixture was diluted with diethyl ether, followed by addition of a phosphate buffer solution (pH, 6.86). The aqueous layer was separated from the organic layer and extracted with diethyl ether two times. The extracts were combined with the organic layer, washed successively with water three times and brine two times, dried over sodium sulfate an distilled off to remove the solvent. The residue was purified by silica gel chromatography to give (4R,5R,6S,8R)-4-methyl-6-(1-t-butyldimethylsilyloxyethyl)-1-azabicyclo[3.2.0]hept-3,7-dione-2-carboxylic acid phenylthio ester.

IR $\nu_{max}^{neat}$ ($cm^{-1}$): 1780 (sh), 1760, 1750 (sh), 1710, 1250, 1140, 1062, 830, 775, 742.

Example 14-2

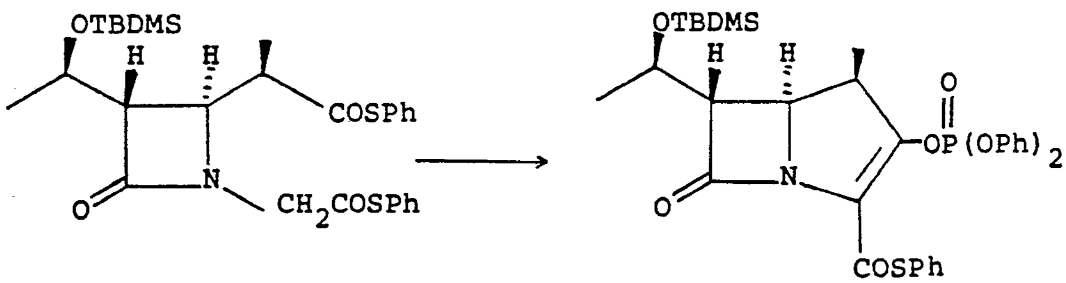

To a solution of (3S,4S)-3-[(1R)-1-t-butyldimethylsilyloxyethyl]-4-[(1R)-1-phenylthiocarbonylethyl]-1-phenylthiocarbonylmethyl-2-azetidinone (60 mg) in dry acetonitrile (1 ml), there was added sodium hydride (13 mg; 50% in oil) under ice-cooling, followed by stirring for 15 minutes. A solution of diphenyl chlorophosphate (57.5 mg) in dry acetonitrile (0.3 ml) was added thereto, and stirring was continued for 1.5 hours while ice-cooling. The reaction mixture was diluted with ethyl acetate (10 ml), washed with brine, dried over a mixture of magnesium sulfate and potassium carbonate (10:1) and evaporated in vacuo to give an oily residue, which was purified by thin layer chromatography on silica gel to obtain (4R,5S,6S,8R)-3-diphenylphosphoryloxy - 4 - methyl - 6 - (1 - t - butyldimethylsilyloxyethyl) - 1 - azabicyclo[3.2.0]hept - 2 - en - 7 - one - 2 - carboxylic acid phenylthio ester (55 mg).

The IR and NMR spectra data of this compound were identical to those of the compound as in Reference Example 3-1.

Example 15

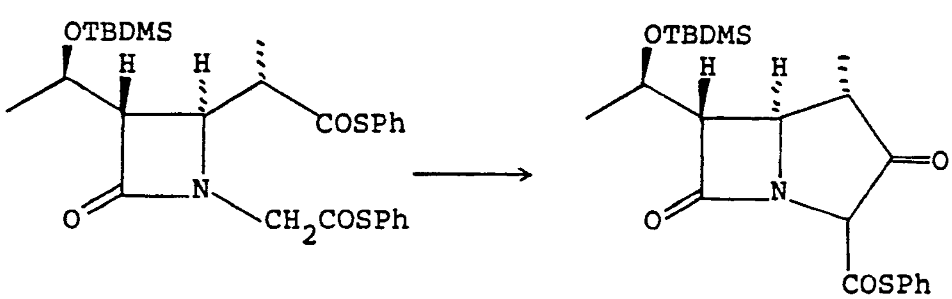

To a solution of (3S,4S)-3-[(1R)-1-t-butyldimethylsilyloxyethyl]-4-[(1S)-1-phenylthiocarbonylethyl]-1-phenylthiocarbonylmethyl-2-azetidinone (20 mg) in dry hexamethylphosphoramide (HMPA) and tetrahydrofuran (1:100) (0.2 ml), there was added a 0.5 M solution (0.3 ml) of lithium bis(trimethylsilyl)amide in tetrahydrofuran under a nitrogen stream at −30°C, followed by stirring at 0 to 5°C for 25 minutes. The reaction mixture was diluted with diethyl ether and a phosphate buffer solution (pH, 6.86). The aqueous layer was separated from the organic layer and extracted with diethyl ether two times. The extracts were combined with the organic layer, washed with brine three times, dried over sodium sulfate and evaporated. The residue was purified by silica gel chromatography to give (4S,5R,6S,8R)-4-methyl-6-(1-t-butyldimethylsilyloxyethyl)-1-azabicyclo[3.2.0]hept-3,7-dione-2-carboxylic acid phenylthio ester.

IR$\nu_{max}^{neat}$ (cm$^{-1}$): 1760, 1700, 1435, 1367, 1247, 827, 765, 740, 680.

Example 16-1

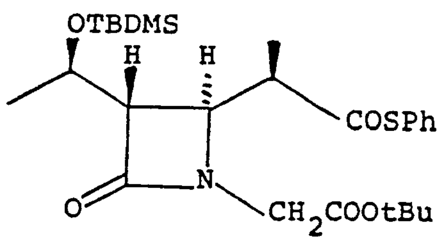

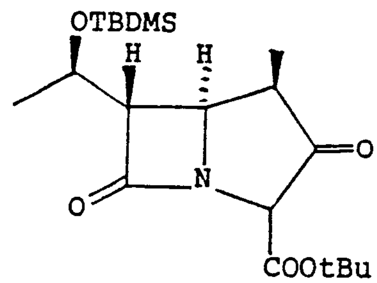

To a solution of (3S,4S)-3-[(1R)-1-t-butyldimethylsilyloxyethyl]-4-[(1R)-1-phenylthiocarbonylethyl]-1-(t-butyloxycarbonylmethyl)-2-azetidinone (65 mg) in dry tetrahydrofuran (1.0 ml), there was added a 0.1 M solution (3.84 ml) of lithium bis(trimethylsilyl)amide in tetrahydrofuran at −70°C under a nitrogen stream. After elevating the temperature to 0 to 5°C, the reaction mixture was quenched with a phosphate buffer solution (pH, 8.0) and extracted with diethyl ether two times. The organic layer was washed with a phosphate buffer solution (pH, 8.0) two times, dried over sodium sulfate and distilled off to remove the solvent to give (4R,5R,6S,8R)-t-butyl-4-methyl-6-(1-t-butyldimethylsilyloxyethyl)-1-azabicyclo[3.2.0]hept-3,7-dione-2-carboxylate.

NMR δ ($CDCl_3$): 0.10 (6H, s), 0.89 (9H, s), 1.18 (3H, d, J=7.9 Hz), 1.27 (3H, d, J=6.6 Hz), 1.46 (9H, s), 2.76 (1H, m), 3.18 (1H, dd, J=2.5 and 5.8 Hz), 4.22 (1H, dd, J=2.5 and 8.1 Hz), 4.58 (1H, s).

Example 16-2

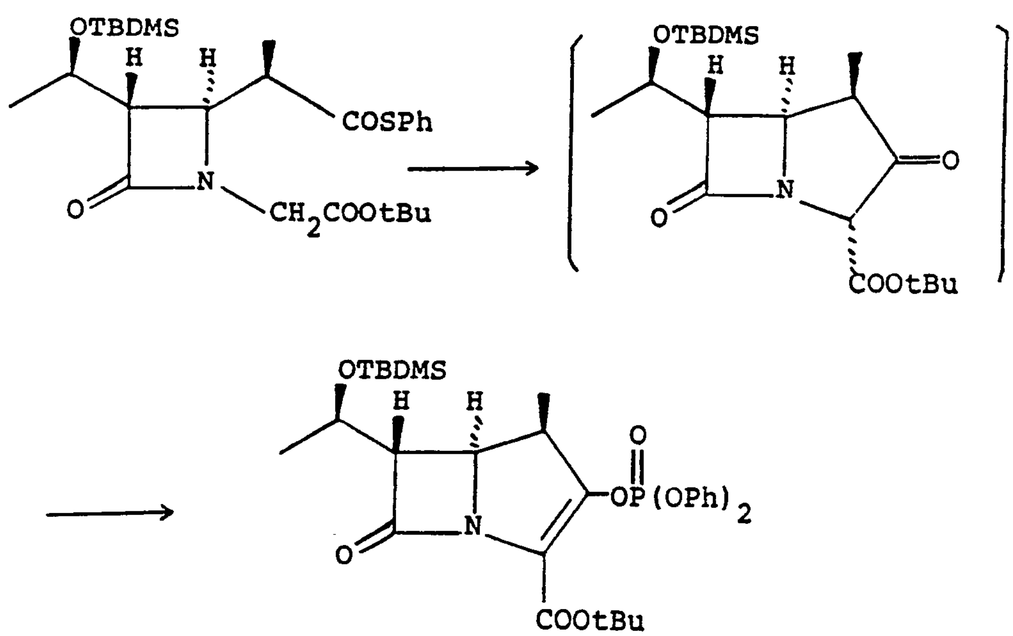

To a solution of (3S,4S)-4-[(1R)-1-phenylthiocarboxyethyl]-3-[(1R)-1-t-butyldimethylsilyloxyethyl]-1-(t-butyloxycarbonylmethyl)-2-azetidinone (101 mg) in dry tetrahydrofuran (2 ml), there was added a 0.1 M solution (5 ml) of lithium diisopropylamide in tetrahydrofuran under a nitrogen stream at −50°C, followed by warming gradually to 0°C for 2 hours. A solution of diphenyl chlorophosphate (120 ml) in dry acetonitrile (7 ml) was added thereto under ice-cooling, followed by stirring for 2 hours. The reaction mixture was

poured into cold diethyl ether (20 ml) and a phosphate buffer solution (pH, 6.86; 20 ml). The organic layer was washed with brine, dried over magnesium sulfate and evaporated in vacuo to remove the solvent. The residue was purified by thin layer chromatography on silica gel to give (4R,5R,6S,8R)-3-(diphenylphosphoryloxy) - 4 - methyl - 6 - (1 - t - butyldimethylsilyloxyethyl) - 1 - azabicyclo[3.2.0]hept - 2 - en - 7 - one - 2 -carboxylic acid t-butyl ester (83.5 mg).

IR $\nu_{max}^{neat}$ ($cm^{-1}$): 1780, 1718, 1635, 1585, 1482, 1360, 1285, 1185, 1158, 960, 940, 830, 765.

NMR δ ($CDCl_3$): 1.17 (3H, d, J=7.6 Hz), 1.21 (3H, d, J=6.3 Hz), 3.22 (1H, dd, J=3.0 and 6.0 Hz), 3.42 (1H, m), 4.12 (1H, dd, J=3.0 and 10.0 Hz), 4.21 (1H, m).

Example 16-3

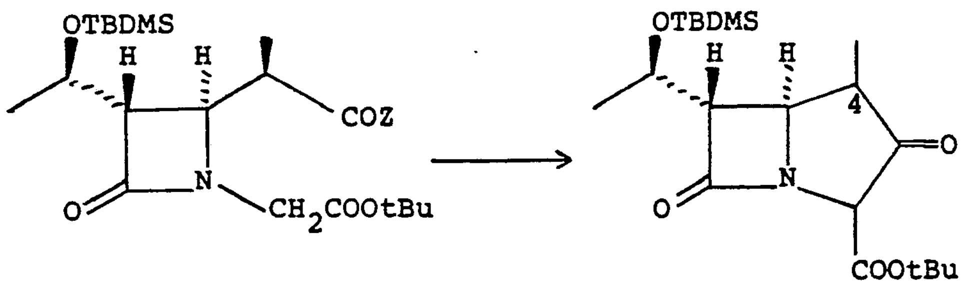

Compound (A)

In the same manner as in Example 16-1 but using the starting material as shown in the table below, there was produced a mixture of (4R,5R,6S,8R)-t-butyl-4-methyl-6-(1-t-butyldimethylsilyloxyethyl)-1-azabicyclo[3.2.0]hept-3,7-dione-2-carboxylate and (4S,5R,6S,8R)-t-butyl-4-methyl-6-(1-t-butyldimethylsilyloxyethyl)-1-azabicyclo[3.2.0]hept-3,7-dione-2-carboxylate (Compound (A)).

| No. | Starting material<br>-Z | Reaction condition |
|---|---|---|
| 1 | -Cl | $LiN[Si(CH_3)_3]_2$/THF;<br>-70°C ⟶ 0 to 5°C |
| 2 | 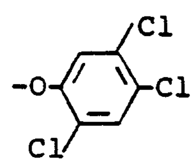 | (same as above) |
| 3 | -OBt | (same as above) |
| 4 | 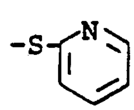 | (same as above) |

The IR and NMR spectra data of the product were identical to those of the compound as in Example 16-1 and Example 17-2.

Example 17-1(3)

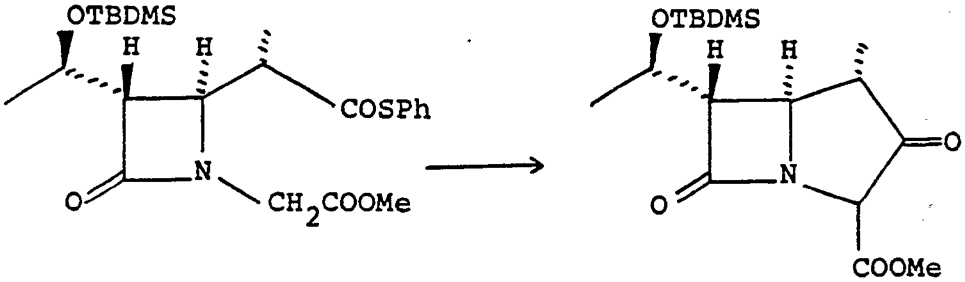

To a solution of (3S,4S)-3-[(1R)-1-t-butyldimethylsilyloxyethyl]-4-[(1S)-1-phenylthiocarbonylethyl]-1-(methoxycarbonylmethyl)azetidin-2-one (50 mg) in dry hexamethylphosphoramide and tetrahydrofuran (1:100) (0.6 ml) was added a 1 M solution (0.44 ml) of lithium bis(trimethylsilyl)amide in tetrahydrofuran at −30°C under a nitrogen stream, followed by stirring at room temperature. After disappearance of the starting material, the reaction mixture was diluted with a phosphate buffer solution (pH, 6.86) and diethyl ether under ice-cooling. The aqueous layer was separated from the organic layer and extracted with diethyl ether two times. The extracts were combined with the organic layer, washed with brine five times, dried over sodium sulfate and evaporated. The residue was purified by silica gel chromatography to give (4S,5R,6S,8R) - methyl - 4 - methyl - 6 - (1 - t - butyldimethylsilyloxyethyl) - 1 - azabicyclo[3.2.0]hept - 3,7 - dione - 2 - carboxylate.

IR $\nu_{max}^{CHCl_3}$ ($cm^{-1}$): 1770, 1760, 1740, 1435, 1245, 1120, 825.

NMR δ ($CDCl_3$): 0.10 (6H, s), 0.90 (9H, s), 1.27 (3H, d, J=7.0 Hz), 1.30 (3H, d, J=6.2 Hz), 2.29 (1H, m), 3.14 (1H, dd, J=1.5 and 5.7 Hz), 3.77 (3H, s), 4.31 (1H, m), 4.71 (1H, s).

Example 17-1(2)

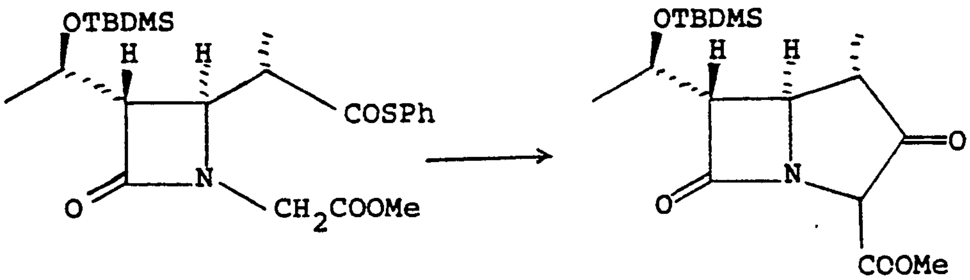

To a solution of (3S,4S)-3-[(1R)-1-t-butyldimethylsilyloxyethyl]-4-[(1S)-1-phenylthiocarbonylethyl]-1-(methoxycarbonylmethyl)azetidin-2-one (15 mg) in dry hexamethylphosphoramide and tetrahydrofuran (1:100) (0.2 ml), there was added a 2.6 M sodium methylsulfinylmethide solution (0.05 ml), prepared from sodium hydride and dimethylsulfixide, at −20 to −25°C under a nitrogen stream. After stirring at 0 to 5°C for 30 minutes and at room temperature for 20 minutes, the reaction mixture was diluted with a phosphate buffer solution (pH, 6.86) and diethyl ether at 0 to 5°C. The aqueous layer was separated and extracted with diethyl ether. The extract was combined with the organic layer, washed with water three times, dried over sodium sulfate and evaporated. The residue was purified by silica gel chromatography to give (4S,5S,6S,8R) - methyl - 4 - methyl - 6 - (1 - t - butyldimethylsilyloxyethyl) - 1 - azabicyclo[3.2.0]hept - 3,7 - dione - 2 - carboxylate.

The IR and NMR spectra data of this compound were identical to those of the compound as obtained in Example 17-1(1).

Example 17-1(3)

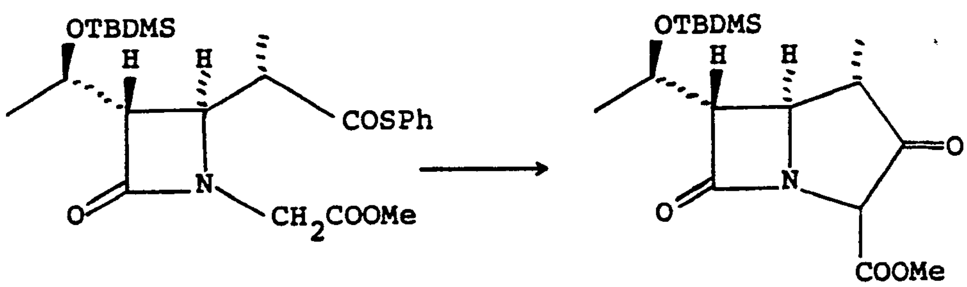

To a solution of (3S,4S)-3-[(1R)-1-t-butyldimethylsilyloxyethyl]-4-[(1S)-1-phenylthiocarbonylethyl]-1-(methoxycarbonylmethyl)azetidin-2-one (15 mg) in dry hexamethylphosphoramide and tetrahydrofuran (1:100) (0.2 ml), there was added potassium t-butoxide (15 mg) at 0 to 5°C, followed by stirring at room temperature for 30 minutes. The reaction mixture was diluted with phosphate buffer solution (pH, 6.86) and diethyl ether at 0 to 5°C. The aqueous layer was separated from the organic layer and extracted with diethyl ether. The extract was combined with the organic layer, washed successively with water two times and brine, dried over sodium sulfate and evaporated. The residue was purified by silica gel chromatography to give (4S,5R,6S,8R)-methyl-4-methyl-6-(1-t-butyldimethylsilyloxyethyl)-1-azabicyclo[3.2.0]hept-3,7-dione-2-carboxylate.

The IR and NMR spectra data of this compound were identical to those of the compound as in Example 17-1(1).

Example 17-1(1)

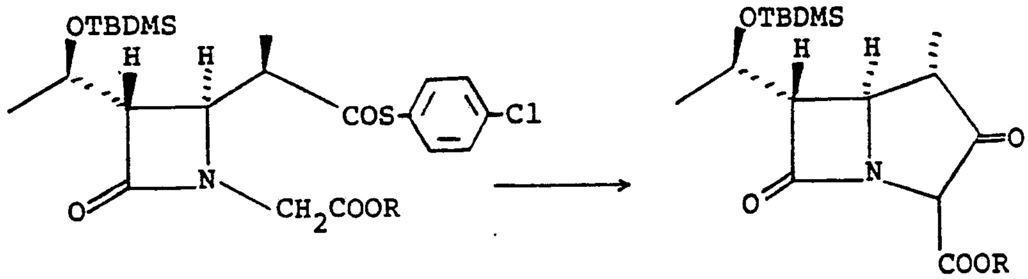

Compound (B)

In the same manner as in Example 17-1(1) but using the starting material as shown in the table below, there was obtained (4S,5R,6S,8R)-4-methyl-6-(1-t-butyldimethylsilyloxyethyl)-1-azabicyclo[3.2.0]hept-3,7-dione-2-carboxylic acid ester (Compounds (B)).

| No. | Starting material | Reaction condition |
|---|---|---|
| | -R | |
| 1 | -PNB | $LiN[Si(CH_3)_3]_2$/ HMPA-THF (1 : 100); -30°C ⟶ room temperature |
| 2 | -tBu | (same as above) |

HMPA: Hexamethylphosphoric triamide

(4S,5R,6S,8R) - p - Nitrobenzyl - 4 - methyl - 6 - (1 - t - butyldimethylsilyloxyethyl) - 1 - azabicyclo[3.2.0]hept - 3,7 - dione - 2 - carboxylate (Compound (B): R = —PNB):—

IR $\nu_{max}^{CHCl_3}$ ($cm^{-1}$): 1780, 1760, 1720, 1520, 1345, 1245, 1178, 835.

NRM δ ($CDCl_3$): 0.08 (3H, s), 0.10 (3H, s), 0.88 (9H, s), 1.26 (3H, d, J=6.8 Hz), 1.30 (3H, d, J=6.2 Hz), 2.28 (1H, m), 3.17 (1H, dd, J=2 and 7 Hz), 3.67 (1H, dd, J=2 and 8 Hz), 4.30 (1H. m), 4.80 (1H, s), 5.29 (2H, s), 7.53 (2H, d, J=9 Hz), 8.24 (2H, d, J=9 Hz).

(4S,5R,6S,8R) - t - Butyl - 4 - methyl - 6 - [1 - t - butyldimethylsilyloxyethyl] - 1 - azabicyclo[3.2.0]hept - 3,7 - dione - 2 - carboxylate (Compound (B): R = —tBu):—

IR $\nu_{max}^{CHCl_3}$ ($cm^{-1}$): 1760, 1730, 1360, 1142, 825.

NRM δ ($CDCl_3$): 0.10 (6H, s), 0.90 (9H, s), 1.27 (3H, d, J=6.9 Hz), 1.30 (3H, d, J=5.9 Hz), 1.46 (9H, s), 2.24 (1H, m), 3.12 (1H, dd, J=2.0 and 6.3 Hz), 3.66 (1H, dd, J=1.9 and 8.1 Hz), 4.29 (1H, m), 4.57 (1H, s).

Example 17-3

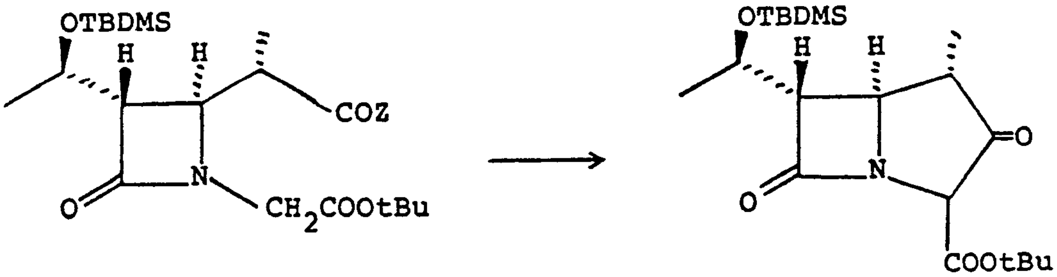

Compound (C)

In the same manner as in Example 14-1 but using the starting material as shown in the table below, there was obtained (4S,5R,6S,8R)-t-butyl-4-methyl-6-[1-t-butyldimethylsilyloxyethyl]-1-azabicyclo[3.2.0]-hept-3,7-dione-2-carboxylate (Compound (C)).

| No. | Starting material -Z | Reaction condition |
|---|---|---|
| 1 | -SPh | NaH/DMF; room temperature |
| 2 | 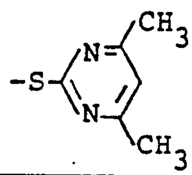 | (same as above) |
| 3 | 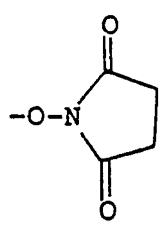 | (same as above) |
| 4 | -Im | (same as above) |

The IR and NMR spectra data of the product were identical to those of the compound as in Example 17-2.

**Claims for the Contracting States: BE CH DE FR GT IT LI LU NL SE**

1. A beta-lactam compound of the general formula:

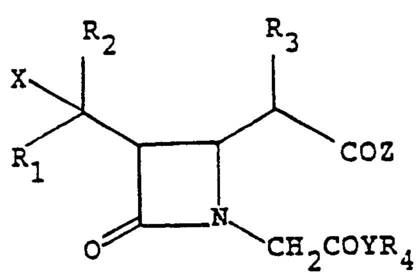

(I)

wherein $R_1$ and $R_2$ are, the same or different, each a hydrogen atom or a $C_{1-4}$-alkyl group, $R_3$ is a $C_{1-4}$-alkyl

group, $R_4$ is a hydrogen atom, a conventional carboxyl-protecting group or a conventional thiocarboxyl-protecting group, X is a hydrogen group, a hydroxyl group or a conventionally protected hydroxyl group, Y is an oxygen atom or a sulfur atom and COZ is a carboxyl group, an activated or conventionally protected carboxyl group, a thiolcarboxyl group or an activated or conventionally protected thiolcarboxyl group.

2. The beta-lactam compound according to claim 1, which is represented by the general formula:

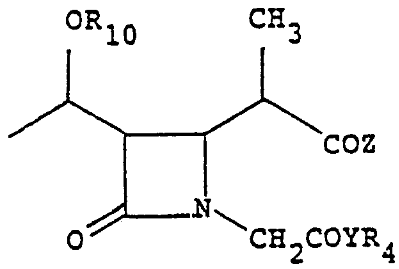

wherein $R_4$, Y and Z are each as defined in claim 1 and $R_{10}$ is a hydrogen atom or a conventional hydroxyl-protecting group.

3. A process for preparing beta-lactam compounds of the general formula:

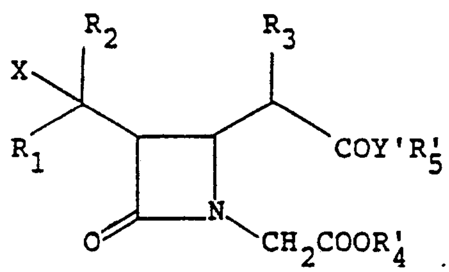

(I-1)

wherein $R_1$ and $R_2$ are, the same or different, each a hydrogen atom or a $C_{1-4}$-alkyl group, $R_3$ is a $C_{1-4}$-alkyl group, $R_4'$ is a conventional carboxyl-protecting group, $R_5'$ is a conventional carboxyl-protecting group or a conventional thiolcarboxyl-protecting group, X is a hydrogen atom, a hydroxyl group or a conventionally protected hydroxyl group and y' is an oxygen atom or a sulfur atom, which comprises reacting a compound of the general formula:

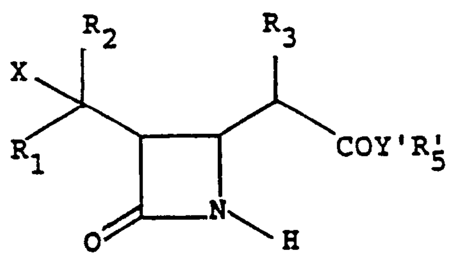

(II)

wherein $R_1$, $R_2$, $R_3$, $R_5'$, X and Y' are each as defined above with a compound of the general formula:

$$M{-}CH_2COOR_4' \qquad (III)$$

wherein $R_4'$ is as defined above and M is an active ester of a hydroxyl group or a halogen atom in an inert solvent in the presence of a base.

4. A process for preparing beta-lactam compounds of the general formula:

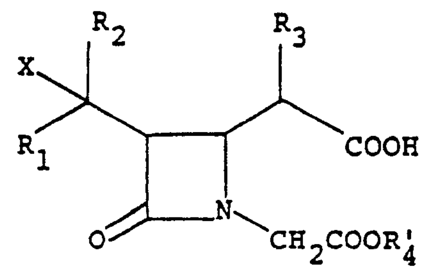

(I-2)

wherein $R_1$ and $R_2$ are, the same or different, each a hydrogen atom or a $C_{1-4}$-alkyl group, $R_3$ is a $C_{1-4}$-alkyl group, $R_4'$ is a conventional carboxyl-protecting group and X is a hydrogen atom, a hydroxyl group or a conventionally protected hydroxyl group, which comprises subjecting a compound of the general formula:

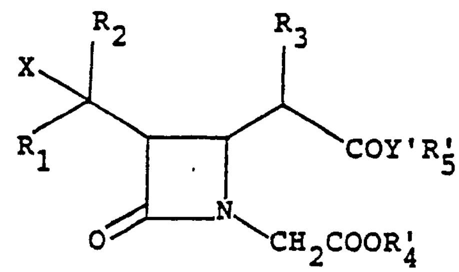

(I-1)

wherein $R_1$, $R_2$, $R_3$, $R_4'$ and X are each as defined above and $R_5'$ is a conventional carboxyl-protecting group or a conventional thiolcarboxyl-protecting group and Y' is an oxygen atom or a sulfur atom to selective elimination of the carboxy-protecting group or selective elimination of the thiolcarboxyl-protecting group.

5. A process for preparing beta-lactam compounds of the general formula:

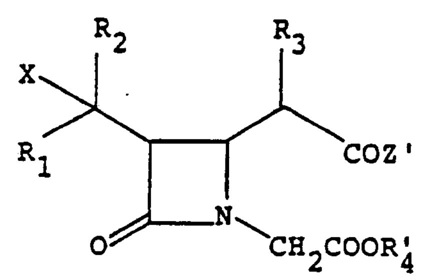

(I-4)

wherein $R_1$ and $R_2$ are, the same or different, each a hydrogen atom or a $C_{1-4}$-alkyl group, $R_3$ is a $C_{1-4}$-alkyl group, $R_4'$ is a conventional carboxyl-protecting group, X is a hydrogen atom, a hydroxyl group or a conventionally protected hydroxyl group and COZ' is an activated or conventionally protected carboxyl group or an activated or conventionally protected thiolcarboxyl group, which comprises subjecting a compound of the general formula:

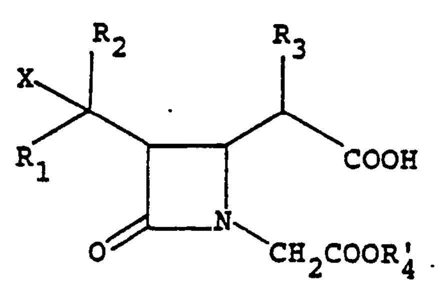

(I-2)

wherein $R_1$, $R_2$, $R_3$, $R_4'$ and X are each as defined above to treatment with a conventional carboxyl-activating

agent, optionally followed by treatment with a hydroxyl or thiol compound, or treatment with a hydroxyl or thiol compound in the presence of a condensing agent.

6. A process for preparing beta-lactam compounds of the general formula:

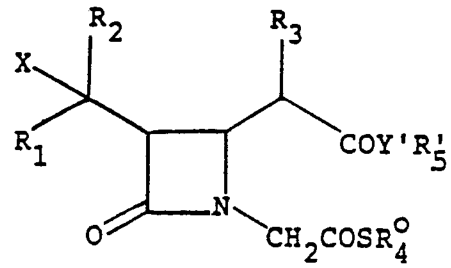

(I-6)

wherein $R_1$ and $R_2$ are, the same or different, each a hydrogen atom or a $C_{1-4}$-alkyl group, $R_3$ is a $C_{1-4}$-alkyl group, $R_4^o$ is a conventional thiolcarboxyl-protecting group, $R_5'$ is a conventional carboxyl-protecting group or a conventional thiolcarboxyl-protecting group, X is a hydrogen atom, a hydroxyl group or a conventionally protected hydroxyl group and Y' is an oxygen atom or a sulfur atom, which comprises subjecting a compound of the general formula:

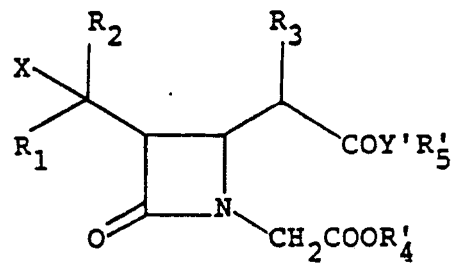

(I-1)

wherein $R_1$, $R_2$, $R_3$, $R_5'$, X and Y' are each as defined above and $R_4'$ is a conventional carboxyl-protecting group to selective elimination of the carboxyl-protecting group represented by the symbol $R_4'$ and reacting the resultant compound of the general formula:

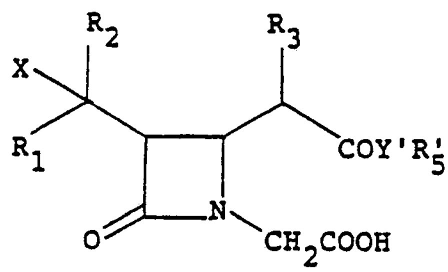

(I-5)

wherein $R_1$, $R_2$, $R_3$, $R_5'$, X and Y' are each as defined above with a compound of the general formula:

$$HSR_4^o$$

wherein $R_4^o$ is as defined above in their reactive forms.

7. A process for preparing beta-lactam compounds of the general formula:

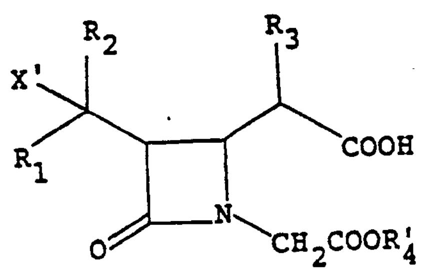

($I-2^o$)

wherein $R_1$ and $R_2$ are, the same or different, each a hydrogen atom or a $C_{1-4}$-alkyl group, $R_3$ is a $C_{1-4}$-alkyl

group, $R_4'$ is a conventional carboxyl-protecting group and X' is a hydrogen atom or a conventionally protected hydroxyl group, which comprises reacting a compound of the general formula:

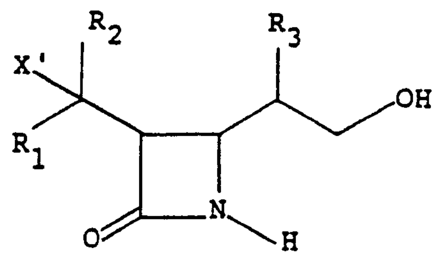

(IV)

wherein $R_1$, $R_2$, $R_3$ and X' are each as defined above with a compound of the general formula:

$$M—CH_2COOR_4' \qquad (III)$$

wherein $R_4'$ is a conventional carboxyl-protecting group and M is an active ester of a hydroxyl group or a halogen atom in an inert solvent in the presence of a base and reacting the resulting compound of the general formula:

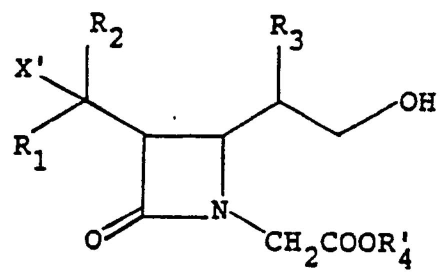

(V)

wherein $R_1$, $R_2$, $R_3$, $R_4'$ and X' are each as defined above with an oxidizing agent.

8. A process for preparing carbapenem compounds of the general formula:

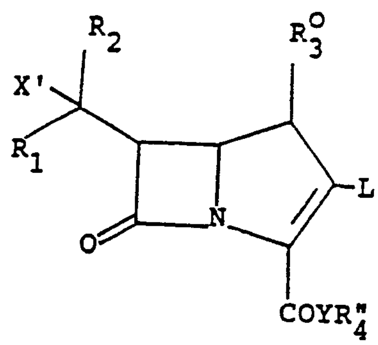

(IX)

wherein $R_1$ and $R_2$ are, the same or different, each a hydrogen atom or a $C_{1-4}$-alkyl group, $R_3^o$ is a hydrogen atom or a $C_{1-4}$-alkyl group, $R_4''$ is a conventional carboxyl-protecting group or a conventional thiolcarboxyl-protecting group, X' is a hydrogen atom or a conventionally protected hydroxyl group, Y is an oxygen atom or a sulfur atom and L is an active ester of a hydroxyl group, which comprises subjecting a compound of the general formula:

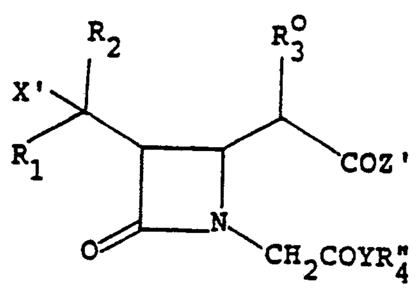

($I-8^o$)

wherein $R_1$, $R_2$, $R_3^o$, $R_4''$, X' and Y are each as defined above and COZ' is an activated or conventionally

protected group or an activated or conventionally protected thiolcarboxyl group to (a) treatment with a base, (b) treatment with an alkylating or acylating agent for catching the group represented by the symbol Z' and (c) treatment with an active esterifying agent for a hydroxyl group in order, said treatments (a), (b) and (c) being carried out in the same reaction vessel.

9. The process according to claim 8, wherein $R_3^o$ is a $C_{1-4}$-alkyl group.

10. The process according to claim 9, wherein $R_3^o$ is a methyl group.

11. A process for preparing carbapenem compounds of the general formula:

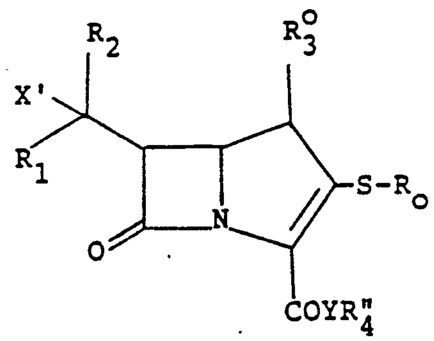

(VII)

wherein $R_o$ is an organic group, $R_1$ and $R_2$ are, the same or different, each a hydrogen atom or a $C_{1-4}$-alkyl group, $R_3^o$ is a hydrogen atom or a $C_{1-4}$-alkyl group, $R_4''$ is a conventional carboxyl-protecting group or a conventional thiolcarboxyl-protecting group, X' is a hydrogen atom or a conventionally protected hydroxyl group and Y is an oxygen atom or a sulfur atom, which comprises subjecting a compound of the general formula:

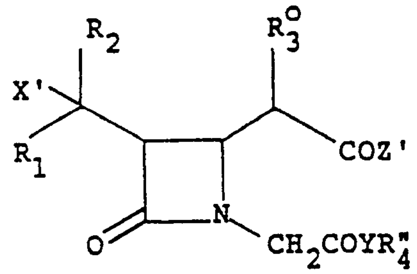

(I-8°)

wherein $R_1$, $R_2$, $R_3^o$, $R_4''$, X' and Y are each as defined above and COZ' is an activated or conventionally protected carboxyl group or an activated or conventionally protected thiolcarboxyl group to (a) treatment with a base, (b) treatment with an alkylating or acylating agent for catching the group represented by the symbol Z' and (c) treatment with an active esterifying agent for a hydroxyl group in order, followed by (d) reacting the resulting product of the general formula:

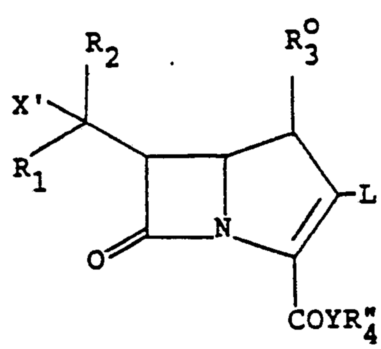

(IX)

wherein $R_1$, $R_2$, $R_3^o$, $R_4''$, X' and Y are each as defined above and L is an active ester of a hydroxyl group with a compound of the general formula:

$$HSR_o \qquad (X)$$

wherein $R_o$ is as defined above in the presence of a base or with a salt of the compound (X) with the base,

said treatments (a), (b) and (c) or (a), (b), (c) and (d) being carried out in the same reaction vessel.

12. The process according to claim 11, wherein $R_3^o$ is a $C_{1-4}$-alkyl group.

13. The process according to claim 12, wherein $R_3^o$ is a methyl group.

**Claims for the Contracting State: AT**

1. A process for preparing beta-lactam compounds of the general formula

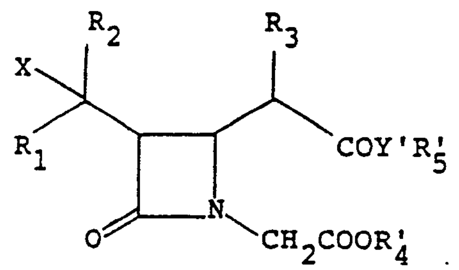

(I-1)

wherein $R_1$ and $R_2$ are, the same or different, each a hydrogen atom or a $C_{1-4}$-alkyl group, $R_3$ is a $C_{1-4}$-alkyl group, $R_4'$ is a conventional carboxyl-protecting group, $R_5'$ is a conventional carboxyl-protecting group or a conventional thiolcarboxyl-protecting group, X is a hydrogen atom, a hydroxyl group or a conventionally protected hydroxyl group and Y' is an oxygen atom or a sulfur atom, which comprises reacting a compound of the general formula:

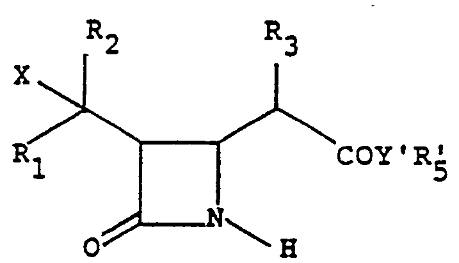

(II)

wherein $R_1$, $R_2$, $R_3$, $R_5'$, X and Y' are each as defined above with a compound of the general formula:

$$M—CH_2COOR_4' \qquad (III)$$

wherein $R_4'$ is as defined above and M is an active ester of a hydroxyl group or a halogen atom in an inert solvent in the presence of a base.

2. A process for preparing beta-lactam compounds of the general formula:

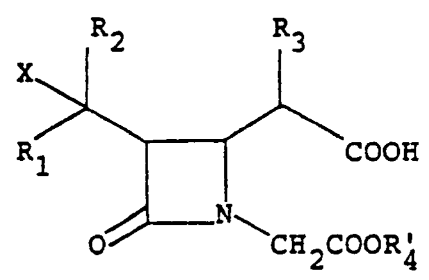

(I-2)

wherein $R_1$ and $R_2$ are, the same or different, each a hydrogen atom or a $C_{1-4}$-alkyl group, $R_3$ is a $C_{1-4}$-alkyl group, $R_4'$ is a conventional carboxyl-protecting group and X is a hydrogen atom, a hydroxyl group or a conventionally protected hydroxyl group, which comprises subjecting a compound of the general formula:

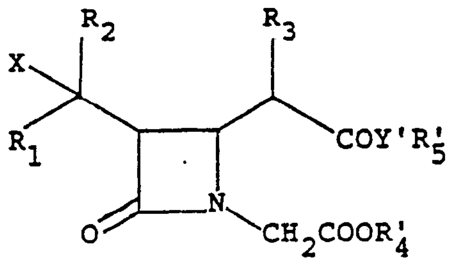

(I-1)

wherein $R_1$, $R_2$, $R_3$, $R_4'$ and X are each as defined above and $R_5'$ is a conventional carboxyl-protecting group or a conventional thiolcarboxyl-protecting group and Y' is an oxygen atom or a sulfur atom to selective elimination of the carboxyl-protecting group or selective elimination of the thiolcarboxyl-protecting group.

3. A process for preparing beta-lactam compounds of the general formula:

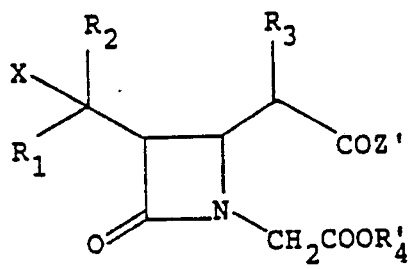

(I-4)

wherein $R_1$ and $R_2$ are, the same or different, each a hydrogen atom or a $C_{1-4}$-alkyl group, $R_3$ is a $C_{1-4}$-alkyl group, $R_4'$ is a conventional carboxyl-protecting group, X is a hydrogen atom, a hydroxyl group or a conventionally protected hydroxyl group and COZ' is an activated or conventionally protected carboxyl group or an activated or conventionally protected thiolcarboxyl group, which comprises subjecting a compound of the general formula:

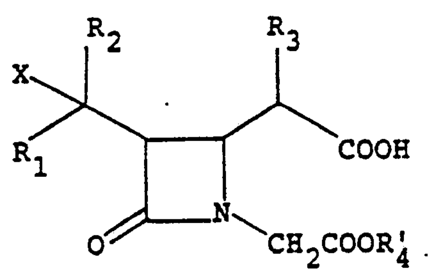

(I-2)

wherein $R_1$, $R_2$, $R_3$, $R_4'$ and X are each as defined above to treatment with a conventional carboxyl-activating agent, optionally followed by treatment with a hydroxyl or thiol compound, or treatment with a hydroxyl or thiol compound in the presence of a condensing agent.

4. A process for preparing beta-lactam compounds of the general formula:

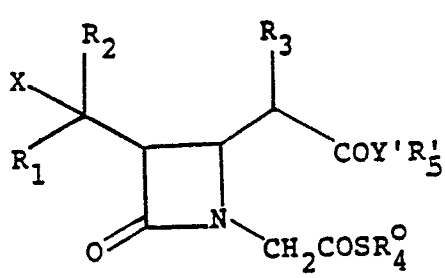

(I-6)

wherein $R_1$ and $R_2$ are, the same or different, each a hydrogen atom or a $C_{1-4}$-alkyl group, $R_3$ is a $C_{1-4}$-alkyl group, $R_4^o$ is a conventional thiolcarboxyl-protecting group, $R_5'$ is a conventional carboxyl-protecting group or a conventional thiolcarboxyl-protecting group, X is a hydrogen atom, a hydroxyl group or a conventionally protected hydroxyl group and Y' is an oxygen atom or a sulfur atom, which comprises subjecting a compound of the general formula:

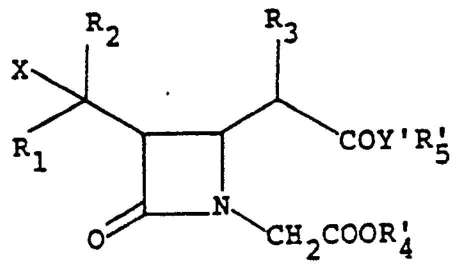

(I-1)

wherein $R_1$, $R_2$, $R_3$, $R'_5$, X and Y' are each as defined above and $R'_4$ is a conventional carboxyl-protecting group to selective elimination of the carboxyl-protecting group represented by the symbol $R'_4$ and reacting the resultant compound of the general formula:

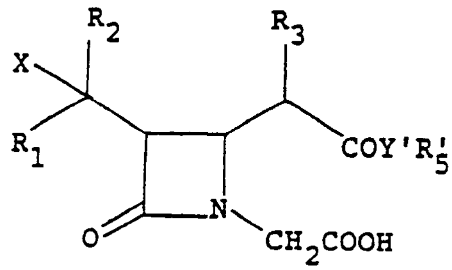

(I-5)

wherein $R_1$, $R_2$, $R_3$, $R'_5$, X and Y' are each as defined above with a compound of the general formula:

$$HSR_4^o$$

wherein $R_4^o$ is as defined above in their reactive forms.

5. A process for preparing beta-lactam compounds of the general formula:

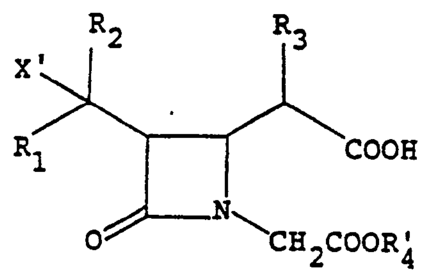

($I-2^o$)

wherein $R_1$ and $R_2$ are, the same or different, each a hydrogen atom or a $C_{1-4}$-alkyl group, $R_3$ is a $C_{1-4}$-alkyl group, $R'_4$ is a conventional carboxyl-protecting group and X' is a hydrogen atom or a conventionally protected hydroxyl group, which comprises reacting a compound of the general formula:

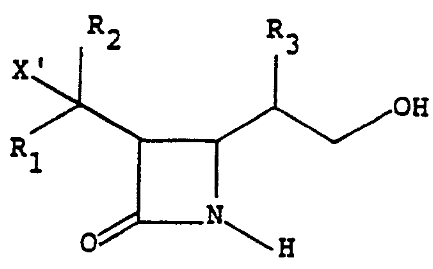

(IV)

wherein $R_1$, $R_2$, $R_3$ and X' are each as defined above with a compound of the general formula:

$$M—CH_2COOR'_4 \quad (III)$$

wherein $R'_4$ is a conventional carboxyl-protecting group and M is an active ester of a hydroxyl group or a

halogen atom in an inert solvent in the presence of a base and reacting the resulting compound of the general formula:

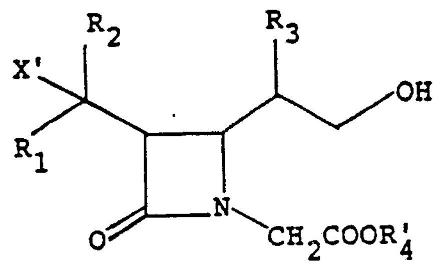

(V)

wherein $R_1$, $R_2$, $R_3$, $R_4'$ and X' are each as defined above with an oxidizing agent.

6. A process for preparing carbapenem compounds of the general formula:

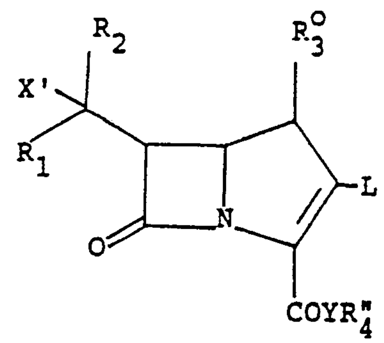

(IX)

wherein $R_1$ and $R_2$ are, the same or different, each a hydrogen atom or a $C_{1-4}$-alkyl group, $R_3^o$ is a hydrogen atom or a $C_{1-4}$-alkyl group, $R_4''$ is a conventional carboxyl-protecting group or a conventional thiolcarboxyl-protecting group, X' is a hydrogen atom or a conventionally protected hydroxyl group, Y is an oxygen atom or a sulfur atom and L is an active ester of a hydroxyl group, which comprises subjecting a compound of the general formula:

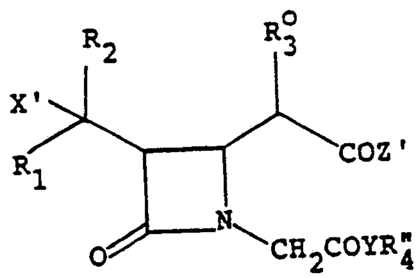

(I-8°)

wherein $R_1$, $R_2$, $R_3^o$, $R_4''$, X' and Y are each as defined above and COZ' is an activated or conventionally protected carboxyl group or an activated or conventionally protected thiolcarboxyl group to (a) treatment with a base, (b) treatment with an alkylating or acylating agent for catching the group represented by the symbol Z' and (c) treatment with an active esterifying agent for a hydroxyl group in order, said treatments (a), (b) and (c) being carried out in an identical reaction vessel.

7. The process according to claim 6, wherein $R_3^o$ is a $C_{1-4}$-alkyl group.

8. The process according to claim 7, wherein $R_3^o$ is a methyl group.

9. A process for preparing carbapenem compounds of the general formula:

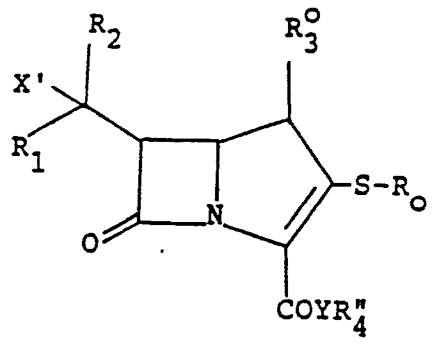

(VII)

wherein $R_o$ is an organic group, $R_1$ and $R_2$ are, the same or different, each a hydrogen atom or a $C_{1-4}$-alkyl group, $R_3^o$ is a hydrogen atom or a $C_{1-4}$-alkyl group, $R_4''$ is a conventional carboxyl-protecting group or a conventional thiolcarboxyl-protecting group, X' is a hydrogen atom or a conventionally protected hydroxyl group and Y is an oxygen atom or a sulfur atom, which comprises subjecting a compound of the general formula:

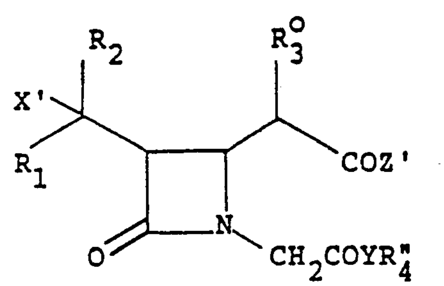

(I-8°)

wherein $R_1$, $R_2$, $R_3^o$, $R_4''$, X' and Y are each as defined above and COZ' is an activated or conventionally protected carboxyl group or an activated or conventionally protected thiolcarboxyl group to (a) treatment with a base, (b) treatment with an alkylating or acylating agent for catching the group represented by the symbol Z' and (c) treatment with an active esterifying agent for a hydroxyl group in order, followed by (d) reacting the resulting product of the general formula:

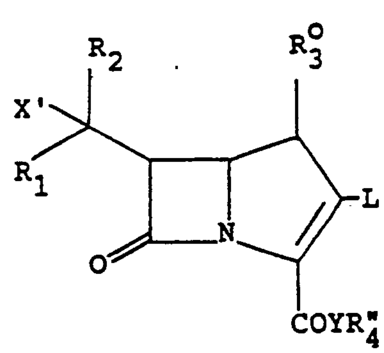

(IX)

wherein $R_1$, $R_2$, $R_3^o$, $R_4''$, X' and Y are each as defined above and L is an active ester of a hydroxyl group with a compound of the general formula:

$HSR_o$ (X)

wherein $R_o$ is as defined above in the presence of a base or with a salt of the compound (X) with the base, said treatments (a), (b) and (c) or (a), (b), (c) and (d) being carried out in an identical reaction vessel.

10. The process according to claim 9, wherein $R_3^o$ is a $C_{1-4}$-alkyl group.

11. The process according to claim 10, wherein $R_3^o$ is a methyl group.

**Patentansprüche für die benannten Vertragsstaaten: BE CH DE FR GB IT LI LU NL SE**

1. beta-Lactamverbindung der allgemeinen Formel:

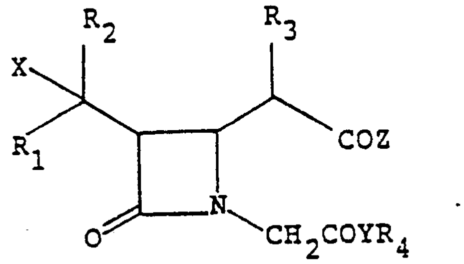

(I)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R_4$ ein Wasserstoffatom, eine übliche Carboxyl-Schutzgruppe oder eine übliche Thiolcarboxyl-Schutzgruppe ist, X ein Wasserstoffatom, eine Hydroxylgruppe oder eine in üblicher Weise geschützte Hydroxylgruppe ist, Y ein Sauerstoffatom oder ein Schwefelatom ist und COZ eine Carboxylgruppe, eine aktivierte oder in üblicher Weise geschützte Carboxylgruppe, eine Thiolcarboxylgruppe oder eine aktivierte oder in üblicher Weise geschützte Thiolcarboxylgruppe ist.

2. beta-Lactamverbindung nach Anspruch 1, wiedergegeben durch die allgemeine Formel:

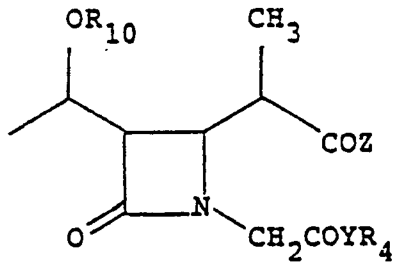

in der $R_4$, Y und Z jeweils wie in Anspruch 1 definiert sind und $R_{10}$ ein Wasserstoffatom oder eine übliche Hydroxyl-Schutzgruppe ist.

3. Verfahren zur Herstellung von beta-Lactamverbindungen der allgemeinen Formel:

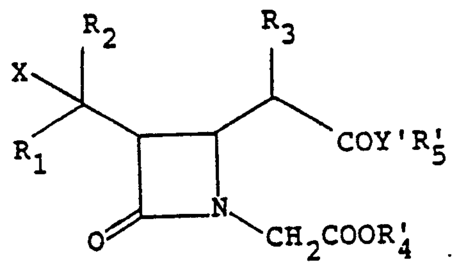

(I-1)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R'_4$ eine übliche Carboxyl-Schutzgruppe ist, $R'_5$ eine übliche Carboxyl-Schutzgruppe oder eine übliche Thiolcarboxyl-Schutzgruppe ist, X ein Wasserstdchutzgruppe ist, X ein Wasserstoffatom, eine Hydroxylgruppe oder eine in üblicher Weise geschützte Hydroxylgruppe ist und Y' ein Sauerstoffatom oder ein Schwefelatom ist, welches die Umsetzung einer Verbindung der allgemeinen Formel:

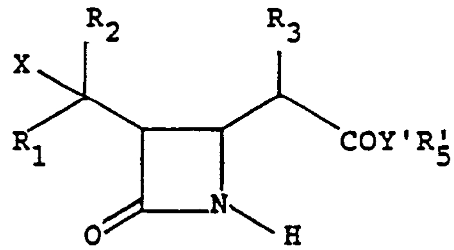

(II)

in der $R_1$, $R_2$, $R_3$, $R'_5$, X und Y' jeweils wie vorstehend definiert sind, mit einer Verbindung der allgemeinen Formel:

$$M—CH_2COOR'_4 \qquad (III)$$

in der $R'_4$ wie vorstehend definiert ist und M ein aktiver Ester einer Hydroxylgruppe oder ein Halogenatom ist, in einem inerten Lösungsmittel in Gegenwart einer Base umfaßt.

4. Verfahren zur Herstellung von beta-Lactamverbindungen der allgemeinen Formel:

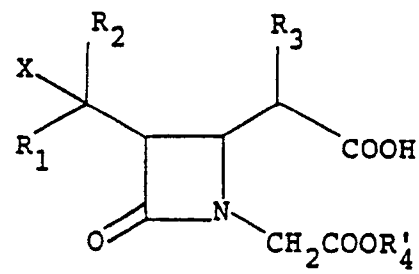

(I-2)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R'_4$ eine übliche Carboxyl-Schutzgruppe ist und X ein Wasserstoffatom, eine Hydroxylgruppe oder eine in üblicher Weise geschützte Hydroxylgruppe ist, welches die Durchführung einer selektiven Eliminierung der Carboxyl-Schutzgruppe oder einer selektiven Eliminierung der Thiolcarboxyl-Schutzgruppe bei einer Verbindung der allgemeinen Formel:

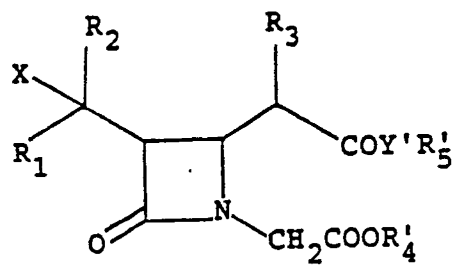

(I-1)

umfaßt, in der $R_1$, $R_2$, $R_3$, $R'_4$ und X jeweils wie vorstehend definiert sind, und $R'_5$ eine übliche Carboxyl-Schutzgruppe oder eine übliche Thiolcarboxyl-Schutzgruppe ist und Y' ein Sauerstoffatom oder Schwefelatom ist.

5. Verfahren zur Herstellung von beta-Lactamverbindungen der allgemeinen Formel:

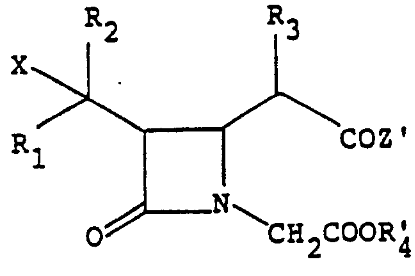

(I-4)

in der $R_1$ und $R_2$, die gleich oder verschieden sind, jeweils ein Wasserstoffatom oder ein $C_{1-4}$-Alkylrest sind,

$R_3$ ein $C_{1-4}$-Alkylrest ist, $R'_4$ eine übliche Carboxyl-Schutzgruppe ist, X ein Wasserstoffatom, eine Hydroxylgruppe oder eine in üblicher Weise geschützte Hydroxylgruppe ist, und COZ' eine aktivierte oder in üblicher Weise geschützte Carboxylgruppe oder eine aktivierte oder in üblicher Weise geschützte Thiolcarboxylgruppe ist, welches die Behandlung einer Verbindung der allgemeinen Formel:

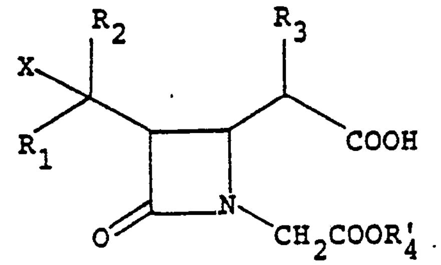

(I-2)

in der $R_1$, $R_2$, $R_3$, $R'_4$ und X jeweils wie vorstehend definiert sind, mit einem üblichen Carboxyl-aktivierenden Agens, gegebenenfalls gefolgt von einer Behandlung mit einer Hydroxy- oder Thiolverbindung oder Behandlung mit einer Hydroxyl- oder Thiolverbindung in Gegenwart eines Kondensierungsmittels, umfaßt.

6. Verfahren für die Herstellung von beta-Lactamverbindungen der allgemeinen Formel:

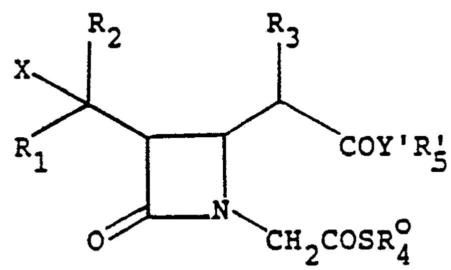

(I-6)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R^\circ_4$ eine übliche Thiolcarboxyl-Schutzgruppe ist, $R'_5$ eine übliche Carboxyl-Schutzgruppe oder eine übliche Thiolcarboxyl-Schutzgruppe ist, X ein Wasserstoffatom, eine Hydroxylgruppe oder eine in üblicher Weise geschützte Hydroxylgruppe ist und Y' ein Sauerstoffatom oder ein Schwefelatom ist, welches die Durchführung einer selektiven Eliminierung der Carboxyl-Schutzgruppe, dargestellt durch das Symbol $R'_4$, bei einer Verbindung der allgemeinen Formel:

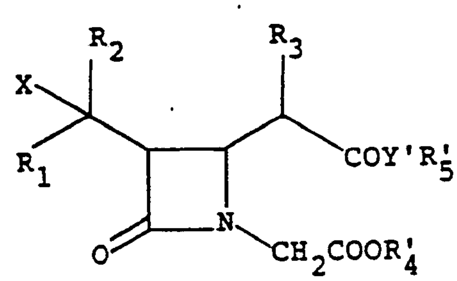

(I-1)

in der $R_1$, $R_2$, $R_3$, $R'_5$, X und Y' jeweils wie vorstehend definiert sind und $R'_4$ eine übliche Carboxyl-Schutzgruppe ist, und die Umsetzung der entstandenen Verbindung der allgemeinen Formel:

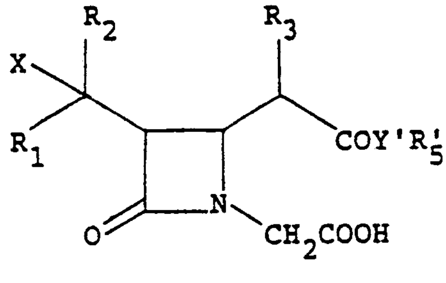

(I-5)

in der $R_1$, $R_2$, $R_3$, $R'_5$, X und Y' jeweils wie vorstehend definiert sind, mit einer Verbindung der allgemeinen Formel:

$$HSR_4^o$$

in der $R^o_4$ wie vorstehend definiert ist, in ihren reaktiven Formen, umfaßt.

7. Verfahren zur Herstellung von beta-Lactamverbindungen der allgemeinen Formel:

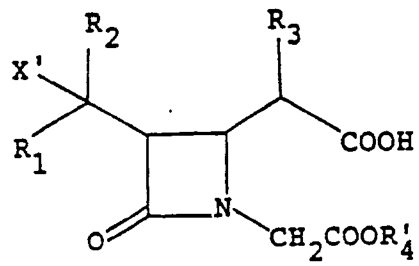

(I-2°)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R'_4$ eine übliche Carboxyl-Schutzgruppe ist und X' ein Wasserstoffatom oder eine in üblicher Weise geschützte Hydroxylgruppe ist, welches die Umsetzung einer Verbindung der allgemeinen Formel:

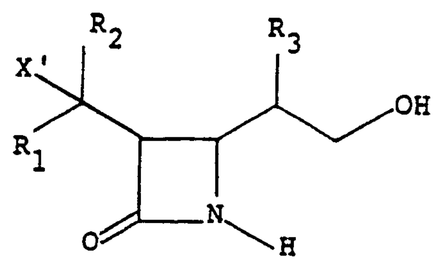

(IV)

in der $R_1$, $R_2$, $R_3$ und X' jeweils wie vorstehend definiert sind, mit einer Verbindung der allgemeinen Formel:

$$M—CH_2COOR'_4 \quad (III)$$

in der $R'_4$ eine übliche Carboxyl-Schutzgruppe ist und M ein aktiver Ester einer Hydroxylgruppe oder ein Halogenatom ist, in einem inerten Lösungsmittel in Gegenwart einer Base und die Umsetzung der entstandenen Verbindung der allgemeinen Formel:

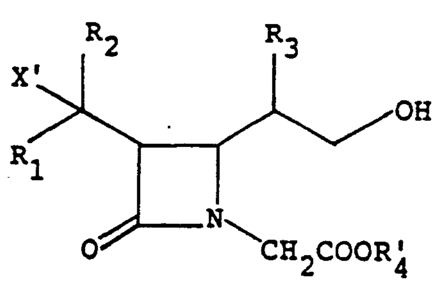

(V)

in der $R_1$, $R_2$, $R_3$, $R'_4$ und X' jeweils wie vorstehend definiert sind, mit einem Oxidationsmittel umfaßt.

8. Verfahren zur Herstellung von Carbapenem-Verbindungen der allgemeinen Formel:

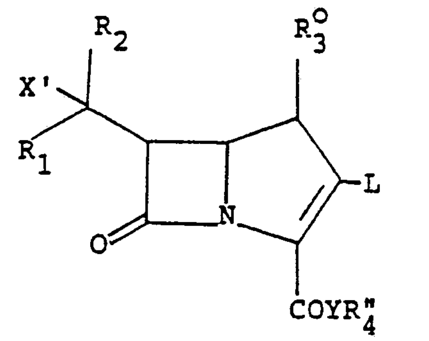

(IX)

in der $R_1$ und $R_2$, die gleich oder verschieden sind, jeweils ein Wasserstoffatom oder ein $C_{1-4}$-Alkylrest sind, $R^\circ_3$ ein Wasserstoffatom oder ein $C_{1-4}$-Alkylrest ist, $R''_4$ eine übliche Carboxyl-Schutzgruppe oder eine übliche Thiolcarboxyl-Schutzgruppe ist, X' ein Wasserstoffatom oder eine in üblicher Weise geschützte Hydroxylgruppe ist, Y ein Sauerstoffatom oder ein Schwefelatom ist und L ein aktiver Ester einer Hydroxylgruppe ist, welches bei einer Verbindung der allgemeinen Formel:

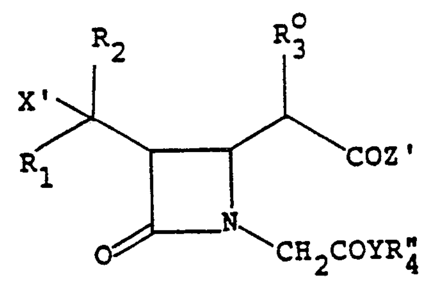

(I-8°)

in der $R_1$, $R_2$, $R^\circ_3$, $R''_4$, X' und Y jeweils wie vorstehend definiert sind und COZ' eine aktivierte oder in üblicher Weise geschützte Carboxylgruppe oder eine aktivierte oder in üblicher Weise geschützte Thiolcarboxylgruppe ist, (a) Behandlung mit einer Base, (b) Behandlung mit einem Alkylierungs- oder Acylierungsmittel zum Abfangen der Gruppe, die durch das Symbol Z' dargestellt ist, und (c) Behandlung mit einem aktiven Veresterungsagens für eine Hydroxylgruppe in dieser Reihenfolge umfaßt, wobei die genannten Behandlungen (a), (b) und (c) im selben Reaktionsgefäß durchgeführt werden.

9. Verfahren nach Anspruch 8, in dem $R^\circ_3$ ein $C_{1-4}$-Alkylrest ist.

10. Verfahren nach Anspruch 9, in dem $R^\circ_3$ eine Methylgruppe ist.

11. Verfahren zur Herstellung von Carbapenem-Verbindungen der allgemeinen Formel:

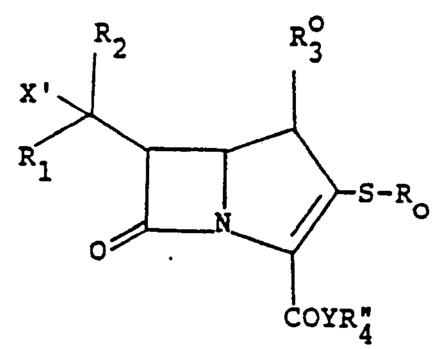

(VII)

in der $R_o$ ein organischer Rest ist, $R_1$ und $R_2$, die gleich oder verschieden sind, jeweils ein Wasserstoffatom oder ein $C_{1-4}$-Alkylrest sind, $R^\circ_3$ ein Wasserstoffatom oder ein $C_{1-4}$-Alkylrest ist, $R''_4$ eine übliche Carboxyl-Schutzgruppe oder eine übliche Thiolcarboxyl-Schutzgruppe ist, X' ein Wasserstoffatom oder eine in üblicher Weise geschützte Hydroxylgruppe ist und Y ein Sauerstoffatom oder ein Schwefelatom ist, welches bei einer Verbindung der allgemeinen Formel:

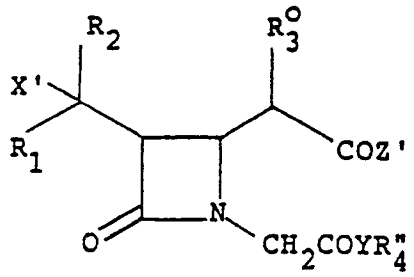

(I-8°)

in der $R_1$, $R_2$, $R^\circ_3$, $R''_4$, X' und Y wie vorstehend definiert sind und COZ' eine aktivierte oder in üblicher Weise geschützte Carboxylgruppe oder eine aktivierte oder in üblicher Weise geschützte Thiolcarboxylgruppe ist, (a) Behandlung mit einer Base, (b) Behandlung mit einem Alkylierungs- oder Acylierungsmittel zum Abfangen der Gruppe, dargestellt durch das Symbol Z', und (c) Behandlung mit, einem aktiven Veresterungsagens für eine Hydroxylgruppe in dieser Reihenfolge, gefolgt von (d) einer Umsetzung des entstandenen Produkts der allgemeinen Formel:

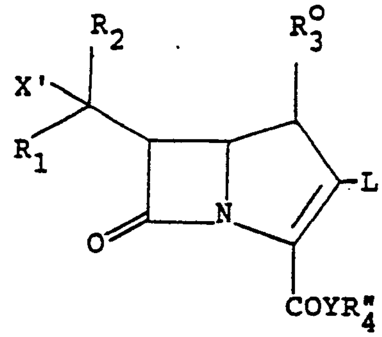

(IX)

in der $R_1$, $R_2$, $R^\circ_3$, $R''_4$, X' und Y wie vorstehend definiert sind und L ein aktiver Ester einer Hydroxylgruppe ist, mit einer Verbindung der allgemeinen Formel:

$$HSR_o \quad (X)$$

in der $R_o$ wie vorstehend definiert ist, in Gegenwart einer Base oder mit einem Salz der Verbindung (X) mit der Base umfaßt, wobei besagte Behandlungen (a), (b) und (c) oder (a), (b), (c) und (d) im selben Reaktionsgefäß durchgeführt werden.

12. Verfahren nach Anspruch 11, in dem $R^\circ_3$ ein $C_{1-4}$-Alkylrest ist.

13. Verfahren nach Anspruch 12, in dem $R^\circ_3$ eine Methylgruppe ist.

**Patentansprüche für den benannten Vertragsstaat: AT**

1. Verfahren zur Herstellung von beta-Lactamverbindungen der allgemeinen Formel:

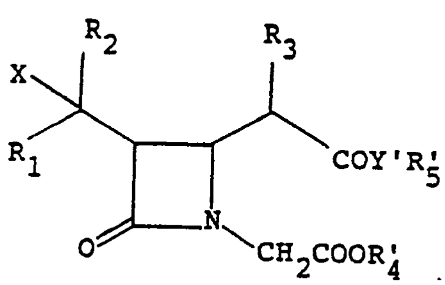

(I-1)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R'_4$ eine übliche Carboxyl-Schutzgruppe ist, $R'_5$ eine übliche Carboxyl-Schutzgruppe oder eine übliche Thiolcarboxyl-Schutzgruppe ist, X ein Wasserstoffatom, eine Hydroxylgruppe oder eine in üblicher Weise geschützte Hydroxylgruppe ist und Y' ein Sauerstoffatom oder ein Schwefelatom ist, welches die Umsetzung einer Verbindung der allgemeinen Formel:

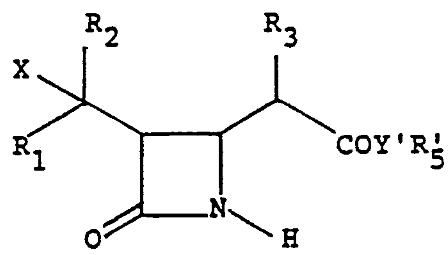

(II)

in der $R_1$, $R_2$, $R_3$, $R'_5$, X und Y' jeweils wie vorstehend definiert sind, mit einer Verbindung der allgemeinen Formel:

$$M—CH_2COOR'_4 \qquad (III)$$

in der $R'_4$ wie vorstehend definiert ist und M ein aktiver Ester einer Hydroxylgruppe oder ein Halogenatom ist, in einem inerten Lösungsmittel in Gegenwart einer Base umfaßt.

2. Verfahren zur Herstellung von beta-Lactamverbindungen der allgemeinen Formel:

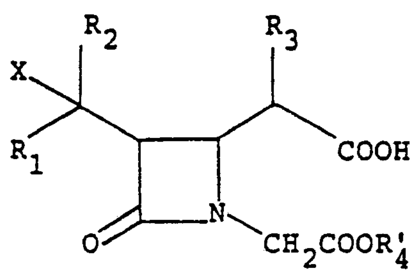

(I-2)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R'_4$ eine übliche Carboxyl-Schutzgruppe ist und X ein Wasserstoffatom, eine Hydroxylgruppe oder eine in üblicher Weise geschützte Hydroxylgruppe ist, welches die Durchführung einer selektiven Eliminierung der Carboxyl-Schutzgruppe oder einer selektiven Eliminierung der Thiolcarboxyl-Schutzgruppe bei einer Verbindung der allgemeinen Formel:

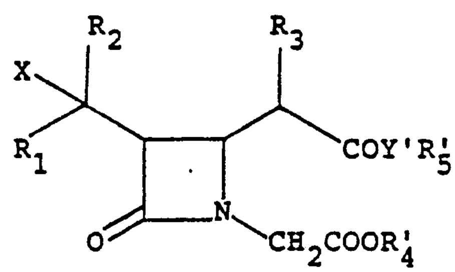

(I-1)

umfaßt, in der $R_1$, $R_2$, $R_3$, $R'_4$ und X jeweils wie vorstehend definiert sind, und $R'_5$ eine übliche Carboxyl-Schutzgruppe oder eine übliche Thiolcarboxyl-Schutzgruppe ist und Y' ein Sauerstoffatom oder Schwefelatom ist.

3. Verfahren zur Herstellung von beta-Lactamverbindungen der allgemeinen Formel:

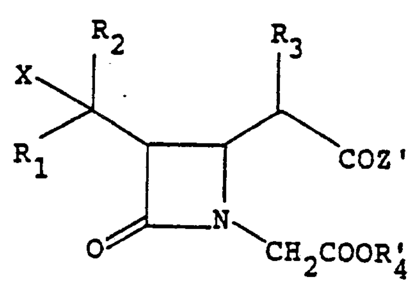

(I-4)

in der $R_1$ und $R_2$, die gleich oder verschieden sind, jeweils ein Wasserstoffatom oder ein $C_{1-4}$-Alkylrest sind,

$R_3$ ein $C_{1-4}$-Alkylrest ist, $R'_4$ eine übliche Carboxyl-Schutzgruppe ist, X ein Wasserstoffatom, eine Hydroxylgruppe oder eine in üblicher Weise geschützte Hydroxylgruppe ist, und COZ' eine aktivierte oder in üblicher Weise geschützte Carboxylgruppe oder eine aktivierte oder in üblicher Weise geschützte Thiolcarboxylgruppe ist, welches die Behandlung einer Verbindung der allgemeinen Formel:

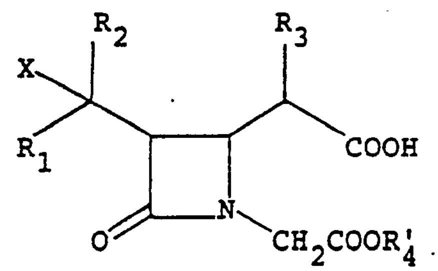

(I-2)

in der $R_1$, $R_2$, $R_3$, $R'_4$ und X jeweils wie vorstehend definiert sind, mit einem üblichen Carboxyl-aktivierenden Agens, gegebenenfalls gefolgt von einer Behandlung mit einer Hydroxy- oder Thiolverbindung oder Behandlung mit einer Hydroxyl- oder Thiolverbindung in Gegenwart eines Kondensierungsmittels, umfaßt.

4. Verfahren für die Herstellung von beta-Lactamverbindungen der allgemeinen Formel:

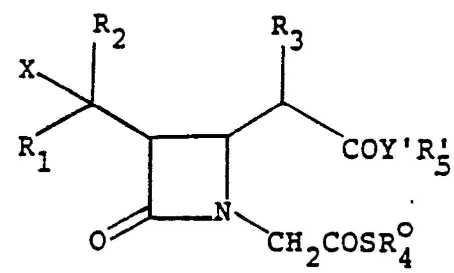

(I-6)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R^{\circ}_4$ eine übliche Thiolcarboxyl-Schutzgruppe ist, $R'_5$ eine übliche Carboxyl-Schutzgruppe oder eine übliche Thiolcarboxyl-Schutzgruppe ist, X ein Wasserstoffatom, eine Hydroxylgruppe oder eine in üblicher Weise geschützte Hydroxylgruppe ist und Y' ein Sauerstoffatom oder ein Schwefelatom ist, welches die Durchführung einer selektiven Eliminierung der Carboxyl-Schutzgruppe, dargestellt durch das Symbol $R'_4$, bei einer Verbindung der allgemeinen Formel:

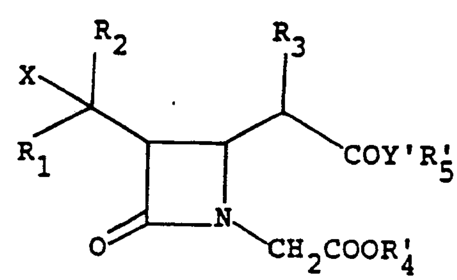

(I-1)

in der $R_1$, $R_2$, $R_3$, $R'_5$, X und Y' jeweils wie vorstehend definiert sind und $R'_4$ eine übliche Carboxyl-Schutzgruppe ist, und die Umsetzung der entstandenen Verbindung der allgemeinen Formel:

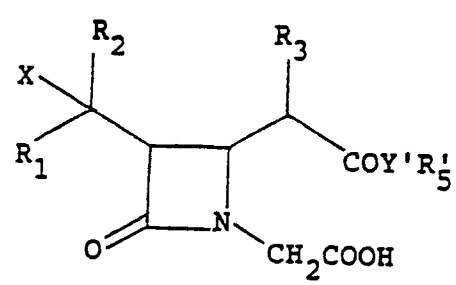

(I-5)

in der $R_1$, $R_2$, $R_3$, $R'_5$, X und Y' jeweils wie vorstehend definiert sind, mit einer Verbindung der allgemeinen Formel:

$$HSR^{\circ}_4$$

in der $R^{\circ}_4$ wie vorstehend definiert ist, in ihren reaktiven Formen, umfaßt.

5. Verfahren zur Herstellung von beta-Lactamverbindungen der allgemeinen Formel:

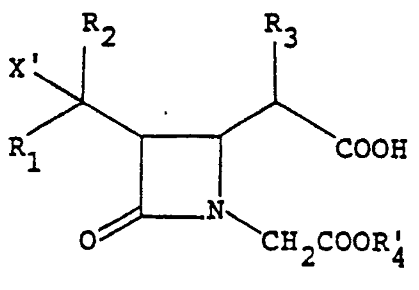

(I-2°)

in der $R_1$ und $R_2$, die gleich oder verschieden sind, jeweils ein Wasserstoffatom oder ein $C_{1-4}$-Alkylrest sind, $R_3$ ein $C_{1-4}$-Alkylrest ist, $R'_4$ eine übliche Carboxyl-Schutzgruppe ist und X' ein Wasserstoffatom oder eine in üblicher Weise geschützte Hydroxylgruppe ist, welches die Umsetzung einer Verbindung der allgemeinen Formel:

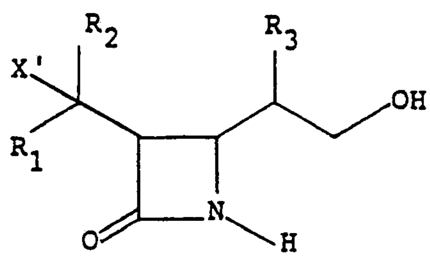

(IV)

in der $R_1$, $R_2$, $R_3$ und X' jeweils wie vorstehend definiert sind, mit einer Verbindung der allgemeinen Formel:

$$M{-}CH_2COOR'_4 \qquad (III)$$

in der $R'_4$ eine übliche Carboxyl-Schutzgruppe ist und M ein aktiver Ester einer Hydroxylgruppe oder ein Halogenatom ist, in einem inerten Lösungsmittel in Gegenwart einer Base und die Umsetzung der entstandenen Verbindung der allgemeinen Formel:

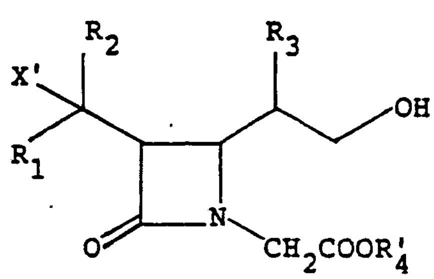

(V)

in der $R_1$, $R_2$, $R_3$, $R'_4$ und X' jeweils wie vorstehend definiert sind, mit einem Oxidationsmittel umfaßt.

6. Verfahren zur Herstellung von Carbapenem-Verbindungen der allgemeinen Formel:

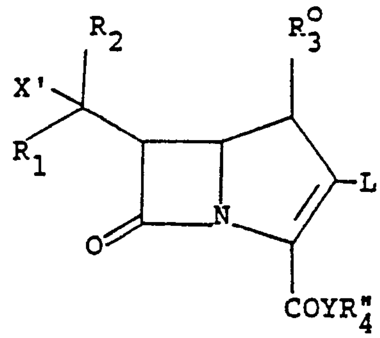

(IX)

in der $R_1$ und $R_2$, die gleich oder verschieden sind, jeweils ein Wasserstoffatom oder ein $C_{1-4}$-Alkylrest sind, $R^\circ_3$ ein Wasserstoffatom oder ein $C_{1-4}$-Alkylrest ist, $R''_4$ eine übliche Carboxyl-Schutzgruppe oder eine übliche Thiolcarboxyl-Schutzgruppe ist, X' ein Wasserstoffatom oder eine in üblicher Weise geschützte Hydroxylgruppe ist, Y ein Sauerstoffatom oder ein Schwefelatom ist und L ein aktiver Ester einer Hydroxylgruppe ist, welches bei einer Verbindung der allgemeinen Formel:

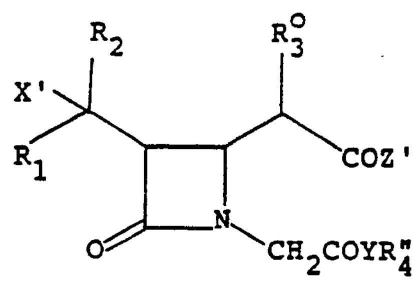

(I-8°)

in der $R_1$, $R_2$, $R^\circ_3$, $R''_4$, X' und Y jeweils wie vorstehend definiert sind und COZ' eine aktivierte oder in üblicher Weise geschützte Carboxylgruppe oder eine aktivierte oder in üblicher Weise geschützte Thiolcarboxylgruppe ist, (a) Behandlung mit einer Base, (b) Behandlung mit einem Alkylierungs- oder Acylierungsmittel zum Abfangen der Gruppe, die durch das Symbol Z' dargestellt ist, und (c) Behandlung mit einem aktiven Veresterungsagens für eine Hydroxylgruppe in dieser Reihenfolge umfaßt, wobei die genannten Behandlungen (a), (b) und (c) im selben Reaktionsgefäß durchgeführt werden.

7. Verfahren nach Anspruch 6, in dem $R^\circ_3$ ein $C_{1-4}$-Alkylrest ist.

8. Verfahren nach Anspruch 7, in dem $R^\circ_3$ eine Methylgruppe ist.

9. Verfahren zur Herstellung von Carbapenem-Verbindungen der allgemeinen Formel:

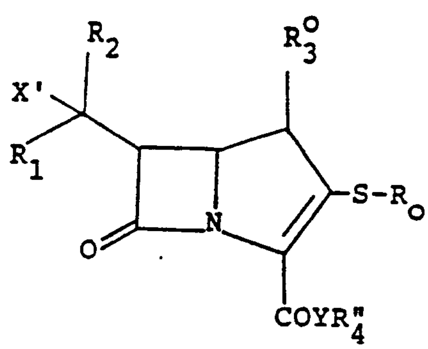

(VII)

in der $R_o$ ein organischer Rest ist, $R_1$ und $R_2$, die gleich oder verschieden sind, jeweils ein Wasserstoffatom oder ein $C_{1-4}$-Alkylrest sind, $R^\circ_3$ ein Wasserstoffatom oder ein $C_{1-4}$-Alkylrest ist, $R''_4$ eine übliche Carboxyl-Schutzgruppe oder eine übliche Thiolcarboxyl-Schutzgruppe ist, X' ein Wasserstoffatom oder eine in üblicher Weise geschützte Hydroxylgruppe ist und Y ein Sauerstoffatom oder ein Schwefelatom ist, welches bei einer Verbindung der allgemeinen Formel:

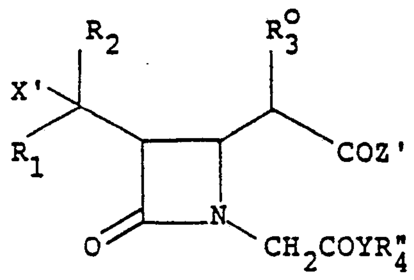

$(I\text{-}8^{o})$

in der $R_1$, $R_2$, $R^o_3$, $R''_4$, X' und Y wie vorstehend definiert sind und COZ' eine aktivierte oder in üblicher Weise geschützte Carboxylgruppe oder eine aktivierte oder in üblicher Weise geschützte Thiolcarboxylgruppe ist, (a) Behandlung mit einer Base, (b) Behandlung mit einem Alkylierungs- oder Acylierungsmittel zum Abfangen der Gruppe, dargestellt durch das Symbol Z', und (c) Behandlung mit einem aktiven Veresterungsagens für eine Hydroxylgruppe in dieser Reihenfolge, gefolgt von (d) einer Umsetzung des entstandenen Produkts der allgemeinen Formel:

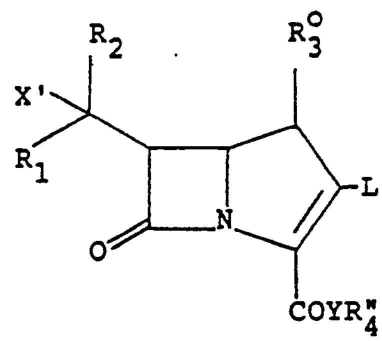

(IX)

in der $R_1$, $R_2$, $R^o_3$, $R''_4$, X' und Y wie vorstehend definiert sind und L ein aktiver Ester einer Hydroxylgruppe ist, mit einer Verbindung der allgemeinen Formel:

$$HSR_o \qquad (X)$$

in der $R_o$ wie vorstehend definiert ist, in Gegenwart einer Base oder mit einem Salz der Verbindung (X) mit der Base umfaßt, wobei besagte Behandlungen (a), (b) und (c) oder (a), (b), (c) und (d) im selben Reaktionsgefäß durchgeführt werden.

10. Verfahren nach Anspruch 9, in dem $R^o_3$ ein $C_{1-4}$-Alkylrest ist.

11. Verfahren nach Anspruch 10, in dem $R^o_3$ eine Methylgruppe ist.

**Revendications pour les Etats contractants: BE CH DE FR GB IT LI LU NL SE**

1. Un β-lactame de formule générale:

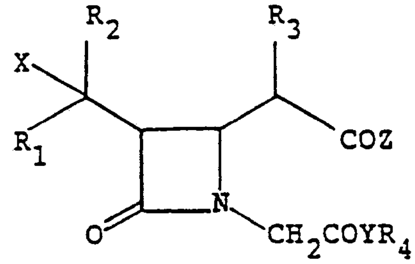

(I)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R_4$ est un atome d'hydrogène, un groupe protecteur classique du groupe carboxyle ou un groupe protecteur classique du groupe thiolcarboxyle, X est un atome d'hydrogène, un groupe hydroxyle ou un groupe hydroxyle protégé de manière classique, Y est un atome d'hydrogène ou un atome de soufre et COZ est un groupe carboxyle, un groupe carboxyle

activé ou protégé de manière classique, un groupe thiolcarboxyle ou un groupe thiolcarboxyle activé ou protégé de manière classique.

2. Le β-lactame selon la revendication 1, qui est représenté par la formule générale:

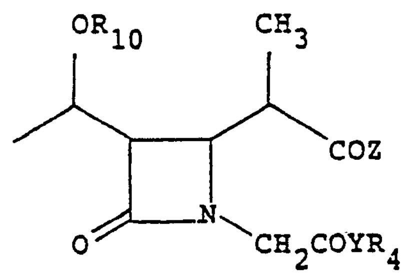

dans laquelle $R_4$, Y et Z sont définis chacun à la revendication 1 et $R_{10}$ est un atome d'hydrogène ou un groupe protecteur du groupe hydroxyle.

3. Un procédé pour fabriquer les β-lactames de formule générale:

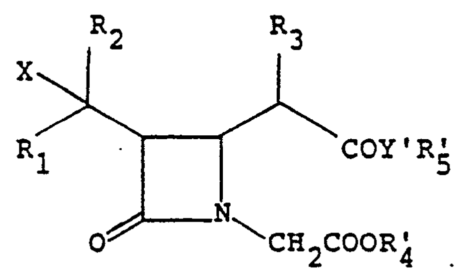

(I-1)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle, $R'_5$ est un groupe protecteur classique du groupe carboxyle ou du groupe thiolcarboxyle, X est un atome d'hydrogène, un groupe hydroxyle ou un groupe hydroxyle protégé de manière classique et Y' est un atome d'oxygène ou de soufre, qui consiste à faire réagir un composé de formule générale:

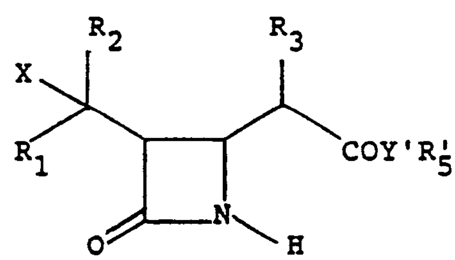

(II)

dans laquelle $R_1$, $R_2$, $R_3$, $R'_5$, X et Y' sont définis chacun comme ci-dessus avec un composé de formule générale:

$$M—CH_2COOR'_4 \qquad (III)$$

dans laquelle $R'_4$ est défini comme ci-dessus et M est un ester réactif d'un groupe hydroxyle ou un atome d'halogène, dans un solvant inerte en présence d'une base.

4. Un procédé pour fabriquer les β-lactames de formule générale:

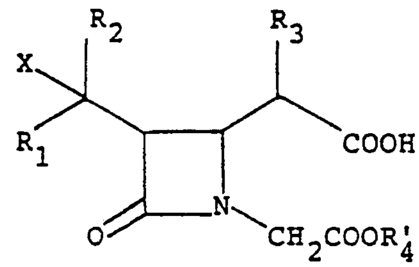

(I-2)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle et X est un atome d'hydrogène, un groupe hydroxyle ou un groupe hydroxyle protégé de manière classique, qui consiste à soumettre un composé de formule générale:

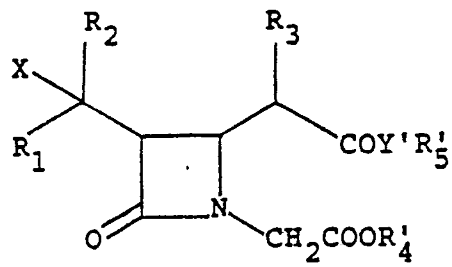

(I-1)

dans laquelle $R_1$, $R_2$, $R_3$, $R'_4$ et X sont définis chacun comme ci-dessus et $R'_5$ est un groupe protecteur classique du groupe carboxyle ou un groupe protecteur classique du groupe thiolcarboxyle et Y' est un atome d'oxygène ou de soufre à l'élimination sélective du groupe protecteur du groupe carboxyle ou à l'élimination sélective du groupe protecteur du groupe thiolcarboxyle.

5. Un procédé pour fabriquer les β-lactames de formule générale:

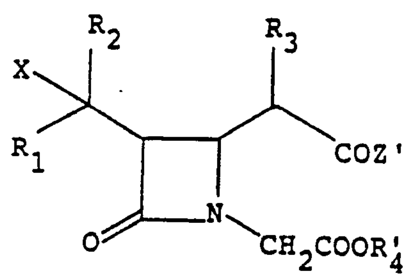

(I-4)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle, X est un atome d'hydrogène, un groupe hydroxyle ou un groupe hydroxyle protégé de manière classique et COZ' est un groupe carboxyle activé ou protégé de manière classique ou un groupe thiolcarboxyle activé ou protégé de manière classique, qui consiste à soumettre un composé de formule générale:

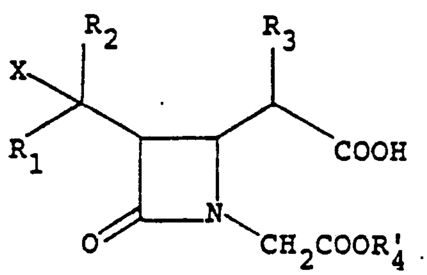

(I-2)

dans laquelle $R_1$, $R_2$, $R_3$, $R'_4$ et X sont définis chacun comme ci-dessus au traitement par un agent activant

classique du groupe carboxyle, puis facultativement au traitement par un composé hydroxylé ou un thiol, ou au traitement par un composé hydroxylé ou un thiol en présence d'un agent de condensation.

6. Un procédé pour fabriquer les β-lactames de formule générale:

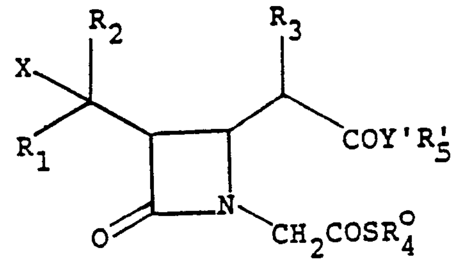

(I-6)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R_4^o$ est un groupe protecteur classique du groupe thiolcarboxyle, $R'_5$ est un groupe protecteur classique du groupe carboxyle ou un groupe protecteur classique du groupe thiolcarboxyle, X est un atome d'hydrogène, un groupe hydroxyle ou un groupe hydroxyle protégé de manière classique et Y' est un atome d'oxygène ou de soufre, qui consiste à soumettre un composé de formule générale:

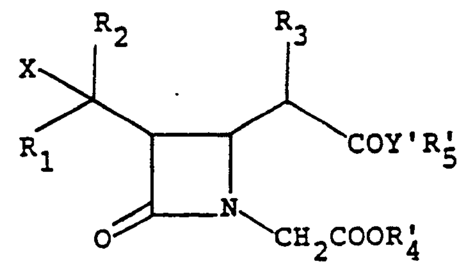

(I-1)

dans laquelle $R_1$, $R_2$, $R_3$, $R'_5$, X et Y' sont définis chacun comme ci-dessus et $R'_4$ est un groupe protecteur classique du groupe carboxyle à l'élimination sélective du groupe protecteur du groupe carboxyle représenté par $R'_4$ et à faire réagir le composé résultant de formule générale:

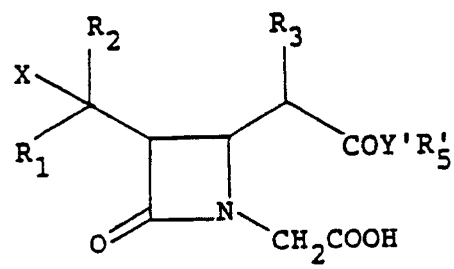

(I-5)

dans laquelle $R_1$, $R_2$, $R_3$, $R'_5$, X et Y' sont définis chacun comme ci-dessus avec un composé de formule générale:

$$HSR_4^o$$

dans laquelle $R_4^o$ est défini comme ci-dessus sous leurs formes réactives.

7. Un procédé pour fabriquer les β-lactames de formule générale:

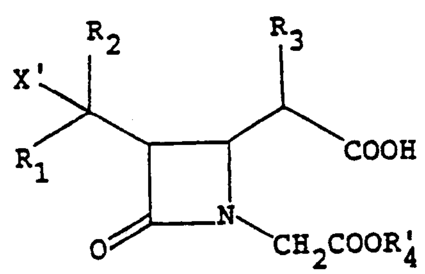

(I-2°)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle et X' est un atome d'hydrogène ou un groupe hydroxyle protégé de manière classique, qui consiste à faire réagir un composé de formule générale:

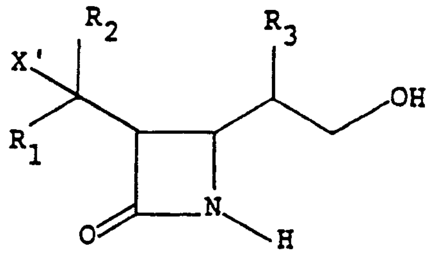

(IV)

dans laquelle $R_1$, $R_2$, $R_3$ et X' sont définis chacun comme ci-dessus avec un composé de formule générale:

$$M—CH_2COOR'_4 \qquad (III)$$

dans laquelle $R'_4$ est un groupe protecteur classique du groupe carboxyle et M est un ester réactif d'un groupe hydroxyle ou un atome d'halogène dans un solvant inerte en présence d'une base et à faire réagir le composé résultant de formule générale:

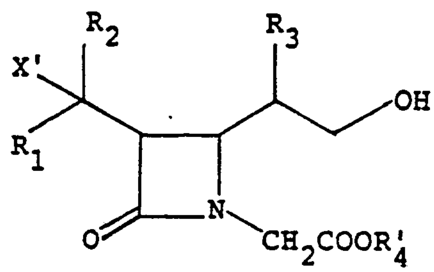

(V)

dans laquelle $R_1$, $R_2$, $R_3$, $R'_4$ et X' sont définis chacun comme ci-dessus avec un agent oxydant.

8. Un procédé pour fabriquer des composés carbapenem de formule générale:

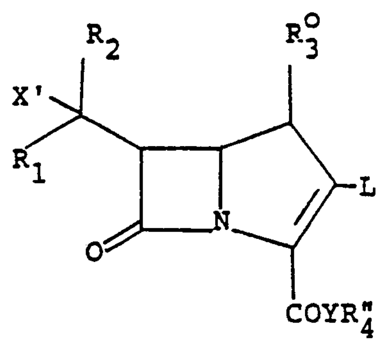

(IX)

dans laquelle $R_1$ et $R_2$ sont identiques ou différents et représentent chacun un atome d'hydrogène ou un groupe alkyle en $C_1$—$C_4$, $R_3^o$ est un atome d'hydrogène ou un groupe alkyle en $C_1$—$C_4$, $R''_4$ est un groupe protecteur classique du groupe carboxyle ou un groupe protecteur classique du groupe thiolcarboxyle, X' est un atome d'hydrogène ou un groupe hydroxyle protégé de manière classique, Y est un atome d'oxygène ou de soufre et L est un ester réactif d'un groupe hydroxyle, qui consiste à soumettre un composé de formule générale:

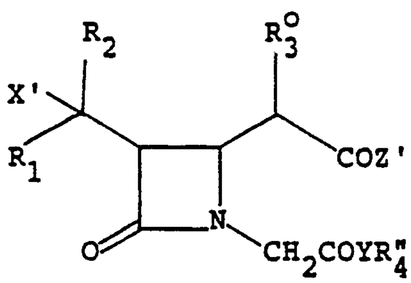

(I-8°)

dans laquelle $R_1$, $R_2$, $R_3^o$, $R''_4$, X' et Y sont définis chacun comme ci-dessus et COZ' est un groupe carboxyle activé ou protégé de manière classique ou un groupe thiolcarboxyle activé ou protégé de manière classique (a) au traitement par une base, (b) au traitement par un agent alkylant ou acylant pour fixer le groupe représenté par Z' et (c) au traitement par un agent estérifiant réactif pour un groupe hydroxyle, dans cet ordre, lesdits traitements (a), (b) et (c) étant mis en oeuvre dans le même récipient de réaction.

9. Le procédé selon la revendication 8 dans lequel $R_3^o$ est un groupe alkyle en $C_1$—$C_4$.

10. Le procédé selon la revendication 9 dans lequel $R_3^o$ est un groupe méthyle.

11. Un procédé pour fabriquer des composés carbapenem de formule générale:

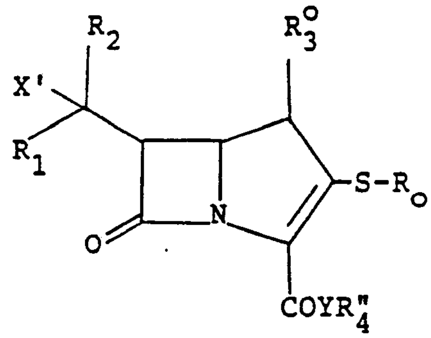

(VII)

dans laquelle $R_o$ est un groupe organique, $R_1$ et $R_2$ sont identiques ou différents et représentent chacun un atome d'hydrogène ou un groupe alkyle en $C_1$—$C_4$, $R_3^o$ est un atome d'hydrogène ou un groupe alkyle en $C_1$—$C_4$, $R''_4$ est un groupe protecteur classique de groupe carboxyle ou un groupe protecteur classique de groupe thiolcarboxyle, X' est un atome d'hydrogène ou un groupe hydroxyle protégé de manière classique et Y est un atome d'oxygène ou de soufre, qui consiste à soumettre un composé de formule générale:

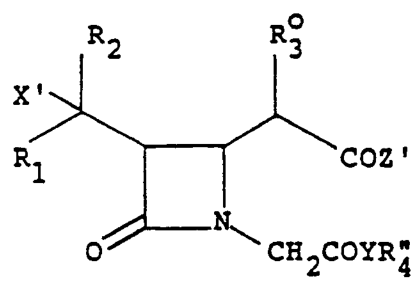

(I-8°)

dans laquelle $R_1$, $R_2$, $R_3^o$, $R''_4$, X' et Y sont définis chacun comme ci-dessus et COZ' est un groupe carboxyle activé ou protégé de manière classique ou un groupe thiolcarboxyle activé ou protégé de manière classique (a) au traitement par une base, (b) au traitement par un agent alkylant ou acylant pour fixer le groupe représenté par Z' et (c) au traitement par un agent estérifiant réactif pour un groupe hydroxyle, dans cet ordre, puis (d) à faire réagir le produit résultant de formule générale:

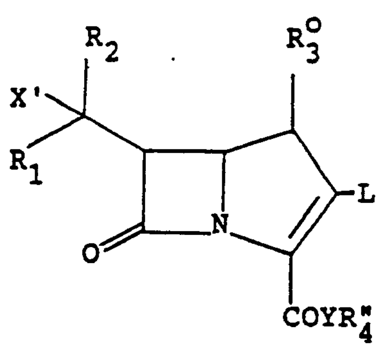

(IX)

dans laquelle $R_1$, $R_2$, $R_3^o$, $R''_4$, X' et Y sont définis chacun comme ci-dessus et L est un ester réactif d'un groupe hydroxyle avec un composé de formule générale:

$HSR_o$ (X)

dans laquelle $R_o$ est défini comme ci-dessus en présence d'une base ou avec un sel du composé (X) avec la

base, lesdits traitements (a), (b) et (c) ou (a), (b), (c) et (d) étant mis en oeuvre dans le même récipient de réaction.

12. Le procédé selon la revendication 11 dans lequel $R^{o}_{3}$ est un groupe alkyle en $C_1$—$C_4$.

13. Le procédé selon la revendication 12 dans lequel $R^{o}_{3}$ est un groupe méthyle.

**Revendications pour l'Etat contractant: AT**

1. Un procédé pour fabriquer les β-lactames de formule générale:

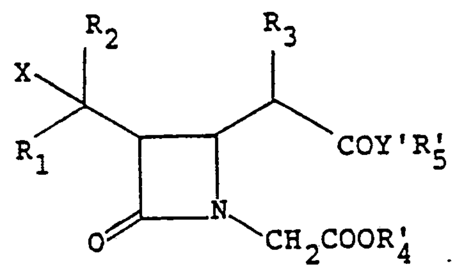

(I-1)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle, $R'_5$ est un groupe protecteur classique du groupe carboxyle ou du groupe thiolcarboxyle, X est un atome d'hydrogène, un groupe hydroxyle ou un groupe hydroxyle protégé de manière classique et Y' est un atome d'oxygène ou de soufre, qui consiste à faire réagir un composé de formule générale:

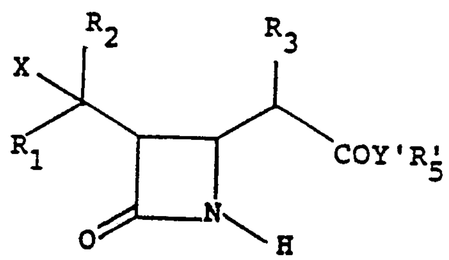

(II)

dans laquelle $R_1$, $R_2$, $R_3$, $R'_5$, X et Y' sont définis chacun comme ci-dessus avec un composé de formule générale:

$$M—CH_2COOR'_4 \qquad (III)$$

dans laquelle $R'_4$ est défini comme ci-dessus et M est un ester réactif d'un groupe hydroxyle ou un atome d'halogène, dans un solvant inerte en présence d'une base.

2. Un procédé pour fabriquer les β-lactames de formule générale:

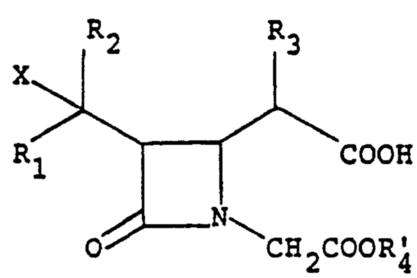

(I-2)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle et X est un atome d'hydrogène, un groupe hydroxyle ou un groupe hydroxyle protégé de manière classique, qui consiste à soumettre un composé de formule générale:

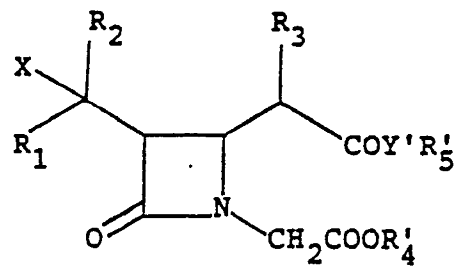

(I-1)

dans laquelle $R_1$, $R_2$, $R_3$, $R'_4$ et X sont définis chacun comme ci-dessus et $R'_5$ est un groupe protecteur classique du groupe carboxyle ou un groupe protecteur classique du groupe thiolcarboxyle et Y' est un atome d'oxygène ou de soufre à l'élimination sélective du groupe protecteur du groupe carboxyle ou à l'élimination sélective du groupe protecteur du groupe thiolcarboxyle.

3. Un procédé pour fabriquer les β-lactames de formule générale:

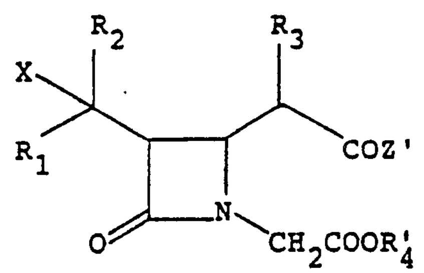

(I-4)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle, X est un atome d'hydrogène, un groupe hydroxyle ou un groupe hydroxyle protégé de manière classique et COZ' est un groupe carboxyle activé ou protégé de manière classique ou un groupe thiolcarboxyle activé ou protégé de manière classique, qui consiste à soumettre un composé de formule générale:

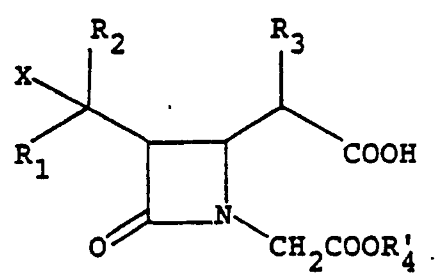

(I-2)

dans laquelle $R_1$, $R_2$, $R_3$, $R'_4$ et X sont définis chacun comme ci-dessus au traitement par un agent activant classique du groupe carboxyle, puis facultativement au traitement par un composé hydroxylé ou un thiol, ou au traitement par un composé hydroxylé ou un thiol en présence d'un agent de condensation.

4. Un procédé pour fabriquer les β-lactames de formule générale:

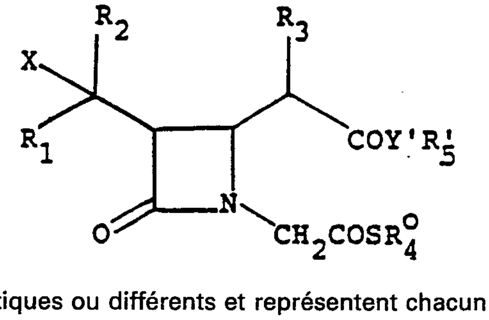

(I-6)

dans laquelle $R_1$ et $R_2$ sont identiques ou différents et représentent chacun un atome d'hydrogène ou un

groupe alkyle en $C_1$—$C_4$, $R_3$ est un groupe alkyle en $C_1$—$C_4$, $R_4^\circ$ est un groupe protecteur classique du groupe thiolcarboxyle, $R'_5$ est un groupe protecteur classique du groupe carboxyle ou un groupe protecteur classique du groupe thiolcarboxyle, X est un atome d'hydrogène, un groupe hydroxyle ou un groupe hydroxyle protégé de manière classique et Y' est un atome d'oxygène ou de soufre, qui consiste à soumettre un composé de formule générale:

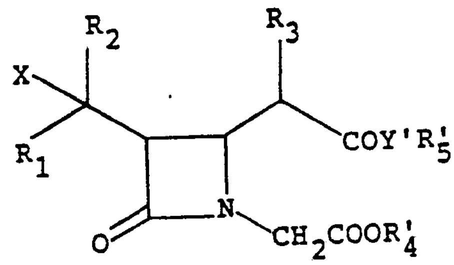

(I-1)

dans laquelle $R_1$, $R_2$, $R_3$, $R'_5$, X et Y' sont définis chacun comme ci-dessus et $R'_4$ est un groupe protecteur classique du groupe carboxyle à l'élimination sélective du groupe protecteur du groupe carboxyle représenté par $R'_4$ et à faire réagir le composé résultant de formule générale:

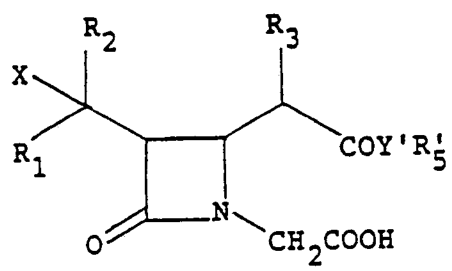

(I-5)

dans laquelle $R_1$, $R_2$, $R_3$, $R'_5$, X et Y' sont définis chacun comme ci-dessus avec un composé de formule générale:

$HSR_4^\circ$

dans laquelle $R_4^\circ$ est défini comme ci-dessus sous leurs formes réactives.

5. Un procédé pour fabriquer les β-lactames de formule générale:

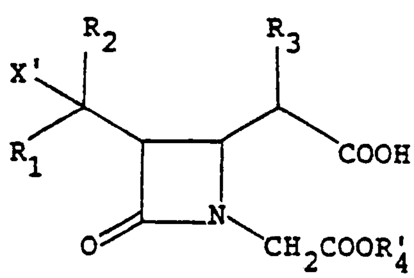

(I-2°)

dans laquelle $R_1$ et $R_2$ sont identiques ou différents et représentent chacun un atome d'hydrogène ou un groupe alkyle en $C_1$—$C_4$, $R_3$ est un groupe alkyle en $C_1$—$C_4$, $R'_4$ est un groupe protecteur classique du groupe carboxyle et X' est un atome d'hydrogène ou un groupe hydroxyle protégé de manière classique, qui consiste à faire réagir un composé de formule générale:

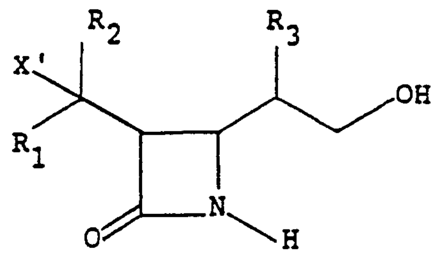

(IV)

dans laquelle $R_1$, $R_2$, $R_3$ et X′ sont définis chacun comme ci-dessus avec un composé de formule générale:

$$M—CH_2COOR'_4 \quad (III)$$

dans laquelle $R'_4$ est un groupe protecteur classique du groupe carboxyle et M est un ester réactif d'un groupe hydroxyle ou un atome d'halogène dans un solvant inerte en présence d'une base et à faire réagir le composé résultant de formule générale:

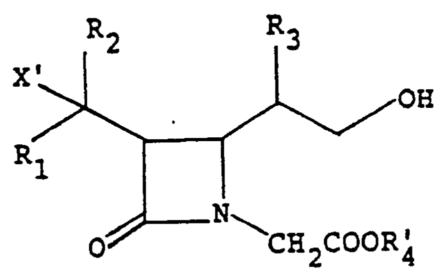

(V)

dans laquelle $R_1$, $R_2$, $R_3$, $R'_4$ et X′ sont définis chacun comme ci-dessus avec un agent oxydant.

6. Un procédé pour fabriquer des composés carbapenem de formule générale:

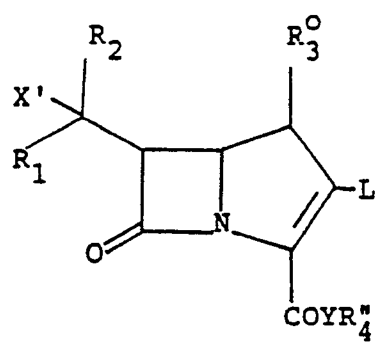

(IX)

dans laquelle $R_1$ et $R_2$ sont identiques ou différents et représentent chacun un atome d'hydrogène ou un groupe alkyle en $C_1—C_4$, $R_3^o$ est un atome d'hydrogène ou un groupe alkyle en $C_1—C_4$, $R''_4$ est un groupe protecteur classique du groupe carboxyle ou un groupe protecteur classique du groupe thiolcarboxyle, X′ est un atome d'hydrogène ou un groupe hydroxyle protégé de manière classique, Y est un atome d'oxygène ou de soufre et L est un ester réactif d'un groupe hydroxyle, qui consiste à soumettre un composé de formule générale:

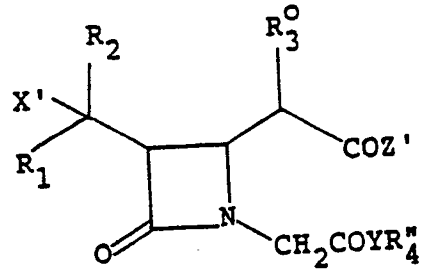

(I-8°)

dans laquelle $R_1$, $R_2$, $R_3^o$, $R''_4$, X′ et Y sont définis chacun comme ci-dessus et COZ′ est un groupe carboxyle

activé ou protégé de manière classique ou un groupe thiolcarboxyle activé ou protégé de manière classique (a) au traitement par une base, (b) au traitement par un agent alkylant ou acylant pour fixer le groupe représenté par Z' et (c) au traitement par un agent estérifiant réactif pour un groupe hydroxyle, dans cet ordre, lesdits traitements (a), (b) et (c) étant mis en oeuvre dans le même récipient de réaction.

7. Le procédé selon la revendication 6 dans lequel $R_3^o$ est un groupe alkyle en $C_1$—$C_4$.

8. Le procédé selon la revendication 7 dans lequel $R_3^o$ est un groupe méthyle.

9. Un procédé pour fabriquer des composés carbapenem de formule générale:

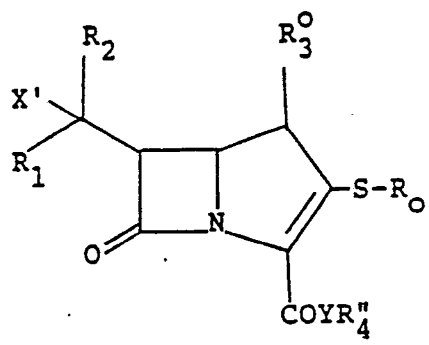

(VII)

dans laquelle $R_o$ est un groupe organique, $R_1$ et $R_2$ sont identiques ou différents et représentent chacun un atome d'hydrogène ou un groupe alkyle en $C_1$—$C_4$, $R_3^o$ est un atome d'hydrogène ou un groupe alkyle en $C_1$—$C_4$, $R''_4$ est un groupe protecteur classique de groupe carboxyle ou un groupe protecteur classique de groupe thiolcarboxyle, X' est un atome d'hydrogène ou un groupe hydroxyle protégé de manière classique et Y est un atome d'oxygène ou de soufre, qui consiste à soumettre un composé de formule générale:

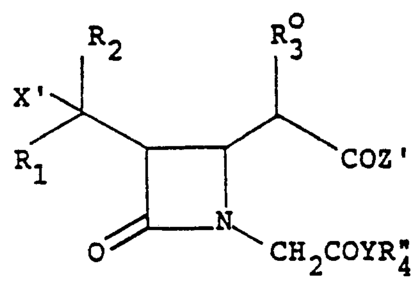

(I-8°)

dans laquelle $R_1$, $R_2$, $R_3^o$, $R''_4$, X' et Y sont définis chacun comme ci-dessus et COZ' est un groupe carboxyle activé ou protégé de manière classique ou un groupe thiolcarboxyle activé ou protégé de manière classique (a) au traitement par une base, (b) au traitement par un agent alkylant ou acylant pour fixer le groupe représenté par Z' et (c) au traitement par un agent estérifiant réactif pour un groupe hydroxyle, dans cet ordre, puis (d) à faire réagir le produit résultant de formule générale:

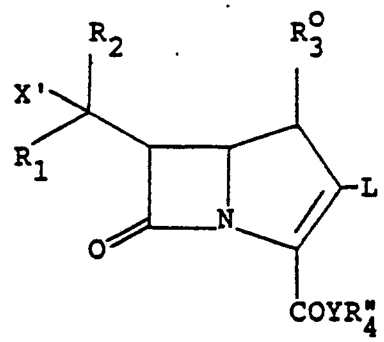

(IX)

dans laquelle $R_1$, $R_2$, $R_3^o$, $R''_4$, X' et Y sont définis chacun comme ci-dessus et L est un ester réactif d'un groupe hydroxyle avec un composé de formule générale:

$$HSR_o \qquad (X)$$

dans laquelle $R_o$ est défini comme ci-dessus en présence d'une base ou avec un sel du composé (X) avec la base, lesdits traitements (a), (b) et (c) ou (a), (b), (c) et (d) étant mis en oeuvre dans le même récipient de réaction.

10. Le procédé selon la revendication 9 dans lequel $R_3^o$ est un groupe alkyle en $C_1$—$C_4$.

11. Le procédé selon la revendication 10 dans lequel $R_3^o$ est un groupe méthyle.